# EUROPEAN PATENT SPECIFICATION

(11) **EP 3 353 160 B1**
(45) Date of publication and mention of the grant of the patent: **04.03.2020**
(21) Application number: 16767288.0
(22) Date of filing: 20.09.2016
(51) Int. Cl.: C07D 401/04, C07D 413/04, A01N 43/707, A01N 43/80, A01N 43/824, A01N 43/56

(54) **PESTICIDALLY ACTIVE HETEROCYCLIC DERIVATIVES WITH SULPHUR CONTAINING SUBSTITUENTS**
PESTIZIDWIRKSAME HETEROCYCLISCHE DERIVATE MIT SCHWEFELHALTIGEN SUBSTITUENTEN
DÉRIVÉS HÉTÉROCYCLIQUES ACTIFS SUR LE PLAN PESTICIDE AVEC DES SUBSTITUANTS CONTENANT DU SOUFRE

(30) Priority: 25.09.2015 EP 15186944
(43) Date of publication of application: 01.08.2018
(73) Proprietor: Syngenta Participations AG, 4058 Basel (CH)
(72) Inventor: MUEHLEBACH, Michel, 4332 Stein (CH); TITULAER, Ruud, 6546 BB Nijmegen (NL); EDMUNDS, Andrew, 4332 Stein (CH); JUNG, Pierre, Joseph, Marcel, 4332 Stein (CH); EMERY, Daniel, 4332 Stein (CH); BUCHHOLZ, Anke, 4332 Stein (CH)
(74) Representative: HGF B.V.
(86) International application number: PCT/EP2016/072285
(87) International publication number: WO 2017/050751

(56) References cited:
- EP-A1- 1 386 915
- WO-A1-00/24735
- WO-A1-01/07413
- WO-A1-2014/066120
- WO-A1-2014/102245
- WO-A1-2015/067646
- DE-A1-102007 003 036
- US-A- 4 788 210
- STEPHAN HOHLOCH ET AL: "Introducing Potential Hemilability into "Click" Triazoles and Triazolylidenes: Synthesis and Characterization of d 6 -Metal Complexes and Oxidation Catalysis", EUROPEAN JOURNAL OF INORGANIC CHEMISTRY., vol. 2014, no. 20, 28 July 2014 (2014-07-28), pages 3164-3171, XP055224248, DE ISSN: 1434-1948, DOI: 10.1002/ejic.201402178
- JIABIN XU ET AL: "Copper-Catalyzed Trifluoromethylthiolation of Aryl Halides with Diverse Directing Groups", ORGANIC LETTERS, vol. 16, no. 15, 1 August 2014 (2014-08-01), pages 3942-3945, XP055224275, US ISSN: 1523-7060, DOI: 10.1021/ol501742a
- DAVID SCHWEINFURTH ET AL: "Expanding the scope of Click derived 1,2,3-triazole ligands: New palladium and platinum complexes", INORGANICA CHIMICA ACTA, ELSEVIER BV, NL, vol. 374, no. 1, 27 February 2011 (2011-02-27), pages 253-260, XP028240186, ISSN: 0020-1693, DOI: 10.1016/J.ICA.2011.02.085 [retrieved on 2011-03-03]
- M. S. R. MURTY ET AL: "Recyclable CuO nanoparticles-catalyzed synthesis of novel-2,5-disubstituted 1,3,4-oxadiazoles as antiproliferative, antibacterial, and antifungal agents", MEDICINAL CHEMISTRY RESEARCH., vol. 23, no. 10, 15 May 2014 (2014-05-15), pages 4579-4594, XP055224169, US ISSN: 1054-2523, DOI: 10.1007/s00044-014-1025-x
- DATABASE REGISTRY [Online] CHEMICAL ABSTRACTS SERVICE, COLUMBUS, OHIO, US; 30 May 2001 (2001-05-30), XP002749265, retrieved from CHEMICAL ABSTRACTS accession no. 339014-40-3 Database accession no. 339014-40-3
- DATABASE REGISTRY [Online] CHEMICAL ABSTRACTS SERVICE, COLUMBUS, OHIO, US; 30 May 2001 (2001-05-30), XP002749266, retrieved from CHEMICAL ABSTRACTS accession no. 339014-38-9 Database accession no. 339014-38-9
- DATABASE REGISTRY [Online] CHEMICAL ABSTRACTS SERVICE, COLUMBUS, OHIO, US; 30 May 2001 (2001-05-30), XP002749269, retrieved from CHEMICAL ABSTRACTS accession no. 339014-34-5 Database accession no. 339014-34-5
- DATABASE REGISTRY [Online] CHEMICAL ABSTRACTS SERVICE, COLUMBUS, OHIO, US; 30 May 2001 (2001-05-30), XP002749270, retrieved from CHEMICAL ABSTRACTS accession no. 339014-14-1 Database accession no. 339014-14-1
- DATABASE REGISTRY [Online] CHEMICAL ABSTRACTS SERVICE, COLUMBUS, OHIO, US; 30 May 2001 (2001-05-30), XP002749274, retrieved from CHEMICAL ABSTRACTS accession no. 339014-10-7 Database accession no. 339014-10-7
- DATABASE REGISTRY [Online] CHEMICAL ABSTRACTS SERVICE, COLUMBUS, OHIO, US; 30 May 2001 (2001-05-30), XP002749276, retrieved from CHEMICAL ABSTRACTS accession no. 339014-15-2 Database accession no. 339014-15-2
- DATABASE REGISTRY [Online] CHEMICAL ABSTRACTS SERVICE, COLUMBUS, OHIO, US; 30 May 2001 (2001-05-30), XP002749277, retrieved from CHEMICAL ABSTRACTS accession no. 339014-32-3 Database accession no. 339014-32-3
- DATABASE REGISTRY [Online] CHEMICAL ABSTRACTS SERVICE, COLUMBUS, OHIO, US; 30 May 2001 (2001-05-30), XP002749278, retrieved from CHEMICAL ABSTRACTS accession no. 339014-28-7 Database accession no. 339014-28-7
- DATABASE REGISTRY [Online] CHEMICAL ABSTRACTS SERVICE, COLUMBUS, OHIO, US; 12 January 2014 (2014-01-12), XP002749282, retrieved from CHEMICAL ABSTRACTS accession no. 1517792-01-6 Database accession no. 1517792-01-6

## Description

The present invention relates to pesticidally active, in particular insecticidally active heterocyclic derivatives containing sulphur substituents, to processes for their preparation, to compositions comprising those compounds, and to their use for controlling animal pests, including arthropods and in particular insects or representatives of the order *Acarina.*

Heterocyclic compounds with pesticidal action are known and described, for example, in WO 2010/125985, WO 2015/163478, WO 2016/024587 and WO 2013/018928. There have now been found novel pesticidally active heterocyclic derivatives with sulphur containing phenyl and pyridyl substituents.

This document discloses compounds of formula I, 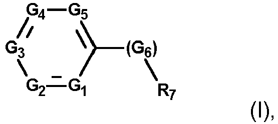 wherein
G₁ is nitrogen or CR₂;
G₂ is nitrogen or CR₃;
G₃ is nitrogen or CR₄;
G₄ is nitrogen or CR₅;
G₅ is nitrogen or CR₆, with the proviso that not more than 2 nitrogen as G may follow consecutively; R₂, R₃, R₄, R₅ and R₆ are, independently from each other, hydrogen, halogen, C₁-C₄haloalkyl,
C₁-C₄haloalkyl substituted by one or two hydroxy, C₁-C₄haloalkyl substituted by one or two methoxy, C₁-C₄haloalkyl substituted by one or two cyano; or
R₂, R₃, R₄, R₅ and R₆ are, independently from each other, C₁-C₄haloalkylsulfanyl, C₁-C₄haloalkylsulfinyl, C₁-C₄haloalkylsulfonyl, C₁-C₄haloalkoxy, SF₅, phenylcarbonylthio, cyano, mercapto, C₁-C₄alkoxycarbonyl, C₁-C₄alkylcarbonyl or -C(O)C₁-C₄haloalkyl; or
R₂, R₃, R₄, R₅ and R₆ are, independently from each other, C₃-C₆cycloalkyl which can be mono- or polysubstituted by substituents selected from the group consisting of halogen, cyano and C₁-C₄alkyl; or two adjacent Rᵢ, wherein Rᵢ is selected from R₂, R₃, R₄, R₅ and R₆, taken together may form a fragment -OCH₂O- or -OCF₂O-;
G₆ is a radical selected from the group consisting of U-0 to U-10 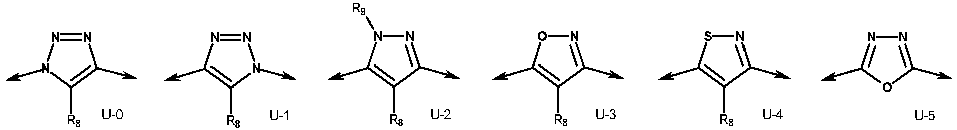 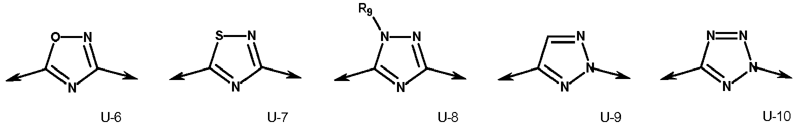
wherein the arrow to the left of each group U denotes the point of attachment to the 6-membered ring containing G₁ and wherein the arrow to the right of each group U denotes the point of attachment to the radical R₇; and wherein
R₈ is hydrogen, C₁-C₄alkyl or C₁-C₄haloalkyl;
R₉ is hydrogen, C₁-C₄alkyl or C₁-C₄haloalkyl;
R₇ is a radical selected from the group consisting of Q₁ and Q₂ 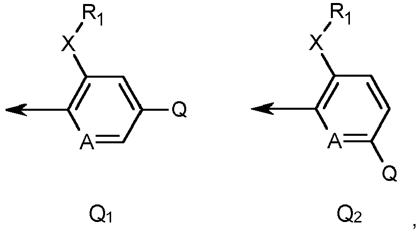
wherein the arrow denotes the point of attachment to the radical U;
and wherein A represents CH or N;
Q is hydrogen, halogen or C₁-C₆haloalkyl; or
Q is phenyl which can be mono- or polysubstituted by substituents selected from the group consisting of halogen, cyano, C₁-C₄alkyl, C₁-C₄haloalkyl, C₁-C₄haloalkoxy, C₁-C₄alkoxy, C₁-C₄haloalkylsulfanyl, C₁-C₄haloalkylsulfinyl, C₁-C₄haloalkylsulfonyl and -C(O)C₁-C₄haloalkyl; or
Q is a five- to ten-membered monocyclic or fused bicyclic ring system linked via a carbon atom to the ring which contains the group A, said ring system can be aromatic, partially saturated or fully saturated and contains 1 to 4 hetero atoms selected from the group consisting of nitrogen, oxygen and sulfur, it not being possible for each ring system to contain more than 2 oxygen atoms and more than 2 sulfur atoms, said five- to ten-membered ring system can be mono- to polysubstituted by substituents independently selected from the group consisting of halogen, cyano, C₁-C₄alkyl, C₁-C₄haloalkyl, C₁-C₄haloalkoxy, C₁-C₄alkoxy, C₁-C₄alkylsulfanyl, C₁-C₄alkylsulfinyl, C₁-C₄alkylsulfonyl, -C(O)C₁-C₄alkyl, C₁-C₄haloalkylsulfanyl, C₁-C₄haloalkylsulfinyl, C₁-C₄haloalkylsulfonyl and -C(O)C₁-C₄haloalkyl; or
Q is a five- to six-membered, aromatic, partially saturated or fully saturated ring system linked via a nitrogen atom to the ring which contains the group A, said ring system can be mono- or polysubstituted by substituents selected from the group consisting of halogen, cyano, C₁-C₄alkyl, C₁-C₄haloalkyl, C₁-C₄haloalkoxy, C₁-C₄alkoxy, C₁-C₄alkylsulfanyl, C₁-C₄alkylsulfinyl, C₁-C₄alkylsulfonyl, -C(O)C₁-C₄alkyl, C₁-C₄haloalkylsulfanyl, C₁-C₄haloalkylsulfinyl, C₁-C₄haloalkylsulfonyl and -C(O)C₁-C₄haloalkyl; and said ring system contains 1, 2 or 3 heteroatoms selected from the group consisting of nitrogen, oxygen and sulfur, wherein said ring system may not contain more than one oxygen atom and not more than one sulfur atom; or
Q is C₃-C₆cycloalkyl, or C₃-C₆cycloalkyl mono- or polysubstituted by substituents selected from the group consisting of halogen, cyano, C₁-C₄alkyl, C₁-C₄haloalkyl, C₃-C₆cycloalkyl and phenyl, wherein said phenyl can be mono- or polysubstituted by substituents selected from the group consisting of halogen, cyano, C₁-C₄alkyl, C₁-C₄haloalkyl, C₁-C₄haloalkoxy, C₁-C₄alkoxy, C₁-C₄haloalkylsulfanyl, C₁-C₄haloalkylsulfinyl, C₁-C₄haloalkylsulfonyl and -C(O)C₁-C₄haloalkyl; or
Q is C₂-C₆alkenyl, or C₂-C₆alkenyl mono- or polysubstituted by substituents selected from the group consisting of halogen, cyano, C₁-C₄alkyl, C₁-C₄haloalkyl, C₁-C₄alkoxy, C₃-C₆cycloalkyl and phenyl, wherein said phenyl can be mono- or polysubstituted by substituents selected from the group consisting of halogen, cyano, C₁-C₄alkyl, C₁-C₄haloalkyl, C₁-C₄haloalkoxy, C₁-C₄alkoxy, C₁-C₄haloalkylsulfanyl, C₁-C₄halo-alkylsulfinyl, C₁-C₄haloalkylsulfonyl and -C(O)C₁-C₄haloalkyl; or
Q is C₂-C₆alkynyl, or C₂-C₆alkynyl mono- or polysubstituted by substituents selected from the group consisting of halogen, cyano, C₁-C₄alkyl, C₁-C₄haloalkyl, C₃-C₆cycloalkyl, tri(C₁-C₄alkyl)silyl and phenyl, wherein said phenyl can be mono- or polysubstituted by substituents selected from the group consisting of halogen, cyano, C₁-C₄alkyl, C₁-C₄haloalkyl, C₁-C₄haloalkoxy, C₁-C₄alkoxy, C₁-C₄haloalkylsulfanyl, C₁-C₄haloalkylsulfinyl, C₁-C₄haloalkylsulfonyl and -C(O)C₁-C₄haloalkyl; or
Q is C₁-C₆haloalkylsulfanyl, C₁-C₆haloalkylsulfinyl, C₁-C₆haloalkylsulfonyl, C₁-C₆haloalkoxy, -C(O)C₁-C₄haloalkyl, C₁-C₆alkylsulfanyl, C₁-C₆alkylsulfinyl, or C₁-C₆alkylsulfonyl;
X is S, SO or SO₂; and
R₁ is C₁-C₄alkyl, C₁-C₄haloalkyl, C₃-C₆cycloalkyl, C₃-C₆cycloalkyl-C₁-C₄alkyl; or
R₁ is C₃-C₆cycloalkyl mono- or polysubstituted by substituents selected from the group consisting of halogen, cyano and C₁-C₄alkyl; or
R₁ is C₃-C₆cycloalkyl-C₁-C₄alkyl mono- or polysubstituted by substituents selected from the group consisting of halogen, cyano and C₁-C₄alkyl; or
R₁ is C₂-C₆alkenyl, C₂-C₆haloalkenyl or C₂-C₆alkynyl; with the exception of the compounds 3-(o-methylthiophenyl)-1-methyl-5-(o-trifluoromethyl-phenyl)-1H-1,2,4-triazole, 1-(2-methylsulfanylphenyl)-4-phenyl-triazole, 4-phenyl-1-[2-(trifluoromethylsulfanyl)phenyl]triazole, 2-[1-(2-methylsulfanylphenyl)triazol-4-yl]pyridine,
2-(2-allylsulfanylphenyl)-5-(3-pyridyl)-1,3,4-oxadiazole, 4-[5-(2-allylsulfanylphenyl)-1,3,4-oxadiazol-2-yl]-2-ethoxy-phenol, 2-(2-allylsulfanylphenyl)-5-(2,6-difluorophenyl)-1,3,4-oxadiazole, 2-(2-allylsulfanylphenyl)-5-(2,3,4,5,6-pentafluorophenyl)-1,3,4-oxadiazole, 2-(4-bromophenyl)-5-(2-methylsulfanylphenyl)-1,3,4-oxadiazole, 2-(4-chlorophenyl)-5-(2-methylsulfonylphenyl)-1,3,4-oxadiazole, 2-(3,4-dichlorophenyl)-5-(2-methylsulfonylphenyl)-1,3,4-oxadiazole, 2-(4-fluorophenyl)-5-(2-methylsulfanylphenyl)-1,3,4-oxadiazole, 2-(3,4-dichlorophenyl)-5-(2-methylsulfanylphenyl)-1,3,4-oxadiazole, 2-(2-methylsulfanylphenyl)-5-phenyl-1,3,4-oxadiazole, 2-(2,4-dichlorophenyl)-5-(2-methylsulfanylphenyl)-1,3,4-oxadiazole, 2-(4-chlorophenyl)-5-(2-methylsulfanylphenyl)-1,3,4-oxadiazole and 3-(2-methylsulfanylphenyl)-5-phenyl-4H-1,2,4-triazole; and agrochemically acceptable salts, stereoismers, enantiomers, tautomers and N-oxides of the compounds of formula I.

Compounds of formula I which have at least one basic centre can form, for example, acid addition salts, for example with strong inorganic acids such as mineral acids, for example perchloric acid, sulfuric acid, nitric acid, nitrose acid, a phosphorus acid or a hydrohalic acid, with strong organic carboxylic acids, such as C₁-C₄alkanecarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄alkane- or arylsulfonic acids which are unsubstituted or substituted, for example by halogen, for example methane- or p-toluenesulfonic acid. Compounds of formula I which have at least one acidic group can form, for example, salts with bases, for example mineral salts such as alkali metal or alkaline earth metal salts, for example sodium, potassium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine.

The alkyl groups occurring in the definitions of the substituents can be straight-chain or branched and are, for example, methyl, ethyl, n-propyl, isopropyl, n-butyl, sec-butyl, iso-butyl, tert-butyl, pentyl, hexyl, nonyl, decyl and their branched isomers. Alkylsulfanyl, alkylsulfinyl, alkylsulfonyl, alkoxy, alkenyl and alkynyl radicals are derived from the alkyl radicals mentioned. The alkenyl and alkynyl groups can be mono- or polyunsaturated.

Halogen is generally fluorine, chlorine, bromine or iodine. This also applies, correspondingly, to halogen in combination with other meanings, such as haloalkyl or halophenyl.

Haloalkyl groups preferably have a chain length of from 1 to 6 carbon atoms. Haloalkyl is, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl; preferably trichloromethyl, difluorochloromethyl, difluoromethyl, trifluoromethyl and dichlorofluoromethyl.

Alkoxy groups preferably have a preferred chain length of from 1 to 6 carbon atoms. Alkoxy is, for example, methoxy, ethoxy, propoxy, i-propoxy, n-butoxy, isobutoxy, sec-butoxy and tert-butoxy and also the isomeric pentyloxy and hexyloxy radicals; preferably methoxy and ethoxy.

Alkoxyalkyl groups preferably have a chain length of 1 to 6 carbon atoms.
Alkoxyalkyl is, for example, methoxymethyl, methoxyethyl, ethoxymethyl, ethoxyethyl, n-propoxymethyl, n-propoxyethyl, isopropoxymethyl or isopropoxyethyl.

Alkylsulfanyl is for example methylsulfanyl, ethylsulfanyl, propylsulfanyl, isopropylsulfanyl, butylsulfanyl, pentylsulfanyl, and hexylsulfanyl.

Alkylsulfinyl is for example methylsulfinyl, ethylsulfinyl, propylsulfinyl, isopropylsulfinyl, a butylsulfinyl, pentylsulfinyl, and hexylsulfinyl.

Alkylsulfonyl is for example methylsulfonyl, ethylsulfonyl, propylsulfonyl, isopropylsulfonyl, butylsulfonyl, pentylsulfonyl, and hexylsulfonyl.

The cycloalkyl groups preferably have from 3 to 6 ring carbon atoms, for example cyclopropyl, cyclobutyl, cyclopentyl and cyclohexyl.

Haloalkoxy groups preferably have a chain length of from 1 to 4 carbon atoms. Haloalkoxy is, for example, difluoromethoxy, trifluoromethoxy or 2,2,2-trifluoroethoxy.

Haloalkylsulfanyl groups preferably have a chain length of from 1 to 4 carbon atoms. Haloalkylsulfanyl is, for example, difluoromethylsulfanyl, trifluoromethylsulfanyl or 2,2,2-trifluoroethylsulfanyl. Similar considerations apply to the radicals C₁-C₄haloalkylsulfinyl and C₁-C₄haloalkylsulfonyl, which may be, for example, trifluoromethylsulfinyl, , trifluoromethylsulfonyl or 2,2,2-trifluoroethylsulfonyl.

According to the present document, when two adjacent Rᵢ, wherein Rᵢ is selected from R₂, R₃, R₄, R₅ and R₆, taken together form a fragment -OCH₂O- or -OCF₂O- then an additional five-membered dioxolane or difluoro-dioxolane ring is formed. For example, compounds of the formula I, wherein G₂ is CR₃, G₃ is CR₄, and in which R₃ and R₄ taken together form the fragment -OCF₂O-, will have the following structure:

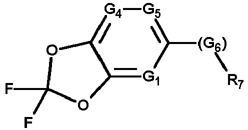

In the context of this document, examples of Q being a five- to six-membered, aromatic, partially saturated or fully saturated ring system that are linked via a nitrogen atom to the ring which contains the group A, are selected from pyrazole, pyrazoline, pyrazolidine, pyrrole, pyrroline, pyrrolidine, pyrrolidine-2-one, piperidine, piperazine, morpholine, imidazole, imidazoline, imidazolidine, triazole and pyridine-2-one.

In the context of this document "mono- to polysubstituted" in the definition of the substituents, means typically, depending on the chemical structure of the substituents, monosubstituted to seven-times substituted, preferably monosubstituted to five-times substituted, more preferably mono-, double- or triple-substituted.

Free radicals represents methyl groups.

The compounds of formula I according to the documentalso include hydrates which may be formed during the salt formation.

According to the present document, Q as a five- to ten-membered monocyclic or fused bicyclic ring system that is linked via a carbon atom to the ring which contains the group A, said ring system can be aromatic, partially saturated or fully saturated and contains 1 to 4 hetero atoms selected from the group consisting of nitrogen, oxygen and sulfur, it not being possible for each ring system to contain more than 2 oxygen atoms and more than 2 sulfur atoms,
or Q as a three- to ten-membered, monocyclic or fused bicyclic ring system which may be aromatic, partially saturated or fully saturated,
is, depending of the number of ring members, preferably selected from the group consisting of the following heterocyclic groups:
pyrrolyl; pyrazolyl; isoxazolyl; furanyl; thienyl; imidazolyl; oxazolyl; thiazolyl; isothiazolyl; triazolyl; oxadiazolyl; thiadiazolyl; tetrazolyl; furyl; pyridyl; pyrimidyl; pyrazinyl; pyridazinyl; triazinyl, pyranyl; quinazolinyl; isoquinolinyl; indolizinyl; isobenzofuranylnaphthyridinyl; quinoxalinyl; cinnolinyl; phthalazinyl; benzothiazolyl; benzoxazolyl; benzotriazolyl; indazolyl; indolyl; (1H-pyrrol-1-yl)-; (1H-pyrrol-2-yl)-; (1H-pyrrol-3-yl)-; (1H-pyrazol-1-yl)-; (1H-pyrazol-3-yl)-; (3H-pyrazol-3-yl)-; (1H-pyrazol-4-yl)-; (3-isoxazolyl)-; (5-isoxazolyl)-; (2-furanyl)-; (3-furanyl)-; (2-thienyl)-; (3-thienyl)-; (1H-imidazol-2-yl)-; (1H-imidazol-4-yl)-; (1H-imidazol-5-yl)-; (2-oxazol-2-yl)-; (oxazol-4-yl)-; (oxazol-5-yl)-; (thiazol-2-yl)-; (thiazol-4-yl)-; (thiazol-5-yl)-; (isothiazol-3-yl)-; (isothiazol-5-yl)-; (1H-1,2,3-triazol-1-yl)-; (1H-1,2,4-triazol-3-yl)-; (4H-1,2,4-triazol-4-yl)-; (1H-1,2,4-triazol-1-yl)-; (1,2,3-oxadiazol-2-yl)-; (1,2,4-oxadiazol-3-yl)-; (1,2,4-oxadiazol-4-yl)-; (1,2,4-oxadiazol-5-yl)-; (1,2,3-thiadiazol-2-yl)-; (1,2,4-thiadiazol-3-yl)-; (1,2,4-thiadiazol-4-yl)-; (1,3,4-thiadiazol-5-yl)-; (1H-tetrazol-1-yl)-; (1H-tetrazol-5-yl)-; (2H-tetrazol-5-yl)-; (2-pyridyl)-; (3-pyridyl)-; (4-pyridyl)-; (2-pyrimidinyl)-; (4-pyrimidinyl)-; (5-pyrimidinyl)-;(2-pyrazinyl)-; (3-pyridazinyl)-; (4-pyridazinyl)-; (1,3,5-triazin-2-yl)-; (1,2,4-triazin-5-yl)-; (1,2,4-triazin-6-yl)-; (1,2,4-triazin-3-yl)-;(furazan-3-yl)-; (2-quinolinyl)-; (3-quinolinyl)-; (4-quinolinyl)-; (5-quinolinyl)-; (6-quinolinyl)-; (3-isoquinolnyl)-; (4-isoquinolnyl)-; (2-quinozolinyl)-; (2-quinoxalinyl)-; (5-quinoxalinyl)-; (pyrido[2,3-b]pyrazin-7-yl)-; (benzoxazol-5-yl)-; (benzothiazol-5-yl)-; (benzo[b]thien-2-yl)- and (benzo[1,2,5]oxadiazol-5-yl)-; indolinyl and tetrahydroquinolynyl.

Preferably G₆ is a radical selected from the group consisting of U-0, U-1, U-2, U-3, U-4, U-7, U-8, U-9 and U-10.

In preferred compounds of formula I, Q is selected from the group consisting of J-0 to J-48 (where the arrow represents the point of attachment of the group J to the ring which contains the group A): 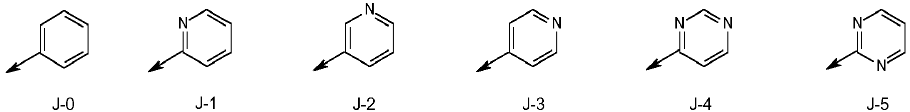 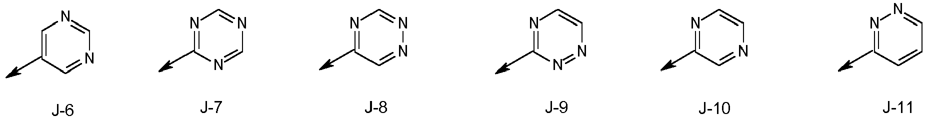 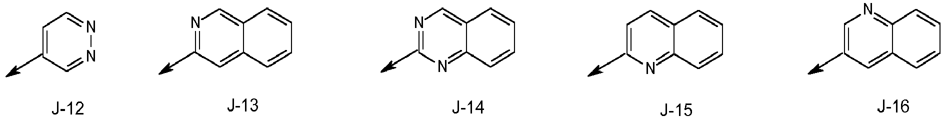 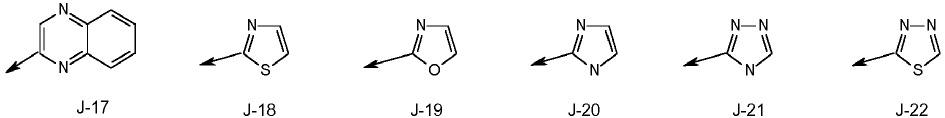 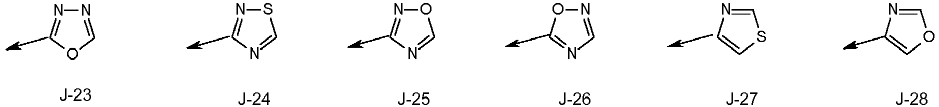 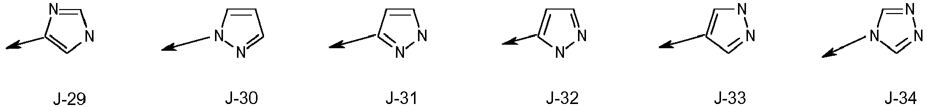 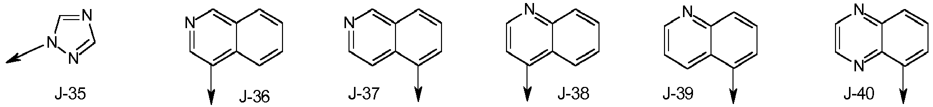 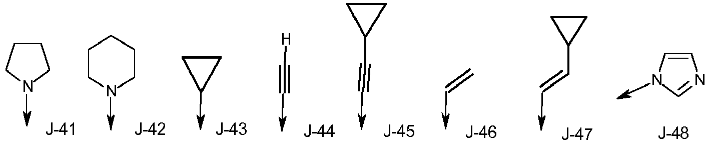 wherein each group J-0 to J-48 is mono-, di- or trisubstituted with Rx, wherein each Rx is, independently selected from hydrogen, halogen, cyano, C₁-C₄alkyl, C₁-C₄haloalkyl, C₁-C₄haloalkoxy, C₁-C₄alkoxy, C₁-C₄alkylsulfanyl, C₁-C₄alkylsulfinyl, C₁-C₄alkylsulfonyl, -C(O)C₁-C₄alkyl, C₁-C₄haloalkylsulfanyl, C₁-C₄haloalkylsulfinyl, C₁-C₄haloalkylsulfonyl and -C(O)C₁-C₄haloalkyl.

Also preferred are compounds of formula I wherein Q is selected from the group consisting of hydrogen, halogen, C₁-C₆haloalkoxy, C₁-C₆haloalkyl, C₁-C₆haloalkylsulfanyl, C₁-C₆haloalkylsulfinyl, C₁-C₆haloalkylsulfonyl and -C(O)C₁-C₄haloalkyl.

In further preferred compounds of formula I, G₆ is selected from the group consisting of formula U-0, U-1, U-2, U-3 and U-5: 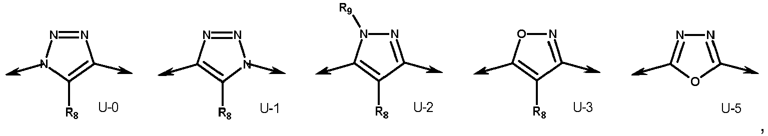
wherein the arrow to the left of each group U denotes the point of attachment to the 6-membered ring containing G₁ and wherein the arrow to the right of each group U denotes the point of attachment to the radical R₇; and
wherein each R₈ is independently selected from hydrogen and C₁-C₄alkyl; and wherein R₉ is selected from hydrogen and C₁-C₄alkyl.

In other preferred compounds of formula I, the radical 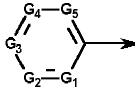 is phenyl, pyridyl or pyrimidinyl mono- or polysubstituted by substituents independently selected from C₁-C₄haloalkyl, C₁-C₄haloalkylsulfanyl, C₁-C₄haloalkylsulfinyl, C₁-C₄haloalkylsulfonyl, C₁-C₄haloalkoxy, and -C(O)C₁-C₄haloalkyl.More preferably, the radical 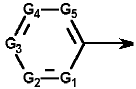 is selected from the group consisting of formula G7, G8 and G9: 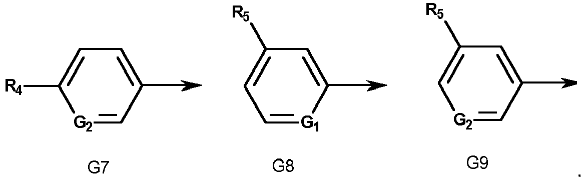 in particular selected from the group consisting of formula G7 and G8,
wherein R₄ and R₅ are independently selected from C₁-C₄haloalkyl, C₁-C₄haloalkylsulfanyl, C₁-C₄haloalkylsulfinyl, C₁-C₄haloalkylsulfonyl, C₁-C₄haloalkoxy, and -C(O)C₁-C₄haloalkyl; and wherein G₁ and G₂ are independently selected from N and CH.
A preferred group of compounds of formula I is represented by the compounds of formula I-1 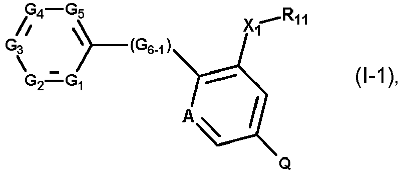
wherein A, Q, G₁, G₂, G₃, G₄ and G₅ are as defined under formula I above; and wherein X₁ is S, SO or SO₂; and R₁₁ is methyl, ethyl, n-propyl, i-propyl or cyclopropylmethyl; and G₆₋₁ is selected from the group consisting of formula U-0, U-1, U-2, U-3 and U-5: 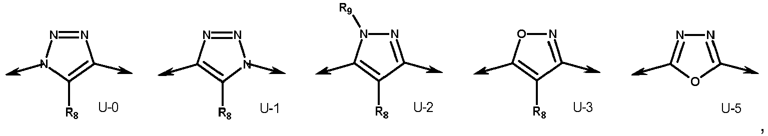
wherein the arrow to the left of each group U denotes the point of attachment to the 6-membered ring containing G₁ and wherein the arrow to the right of each group U denotes the point of attachment to the radical R₇; and
wherein each R₈ is independently selected from hydrogen and C₁-C₄alkyl, preferably hydrogen and methyl; and wherein R₉ is selected from hydrogen and C₁-C₄alkyl, preferably hydrogen and methyl; and agrochemically acceptable salts, stereoismers, enantiomers, tautomers and N-oxides of those compounds.

Also preferred are compounds of formula I-1 wherein the radical 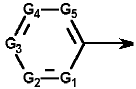 is phenyl, pyridyl or pyrimidinyl mono- or polysubstituted by substituents independently selected from C₁-C₄haloalkyl, C₁-C₄haloalkylsulfanyl, C₁-C₄haloalkylsulfinyl, C₁-C₄haloalkylsulfonyl, C₁-C₄haloalkoxy and -C(O)C₁-C₄haloalkyl.

More preferred are compounds of formula I-1 wherein the radical 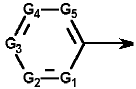 is selected from the group consisting of formula G7, G8 and G9: 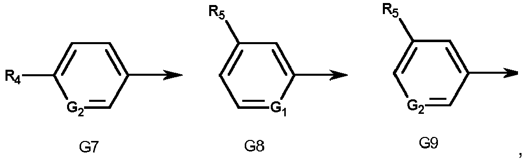 in particular selected from the group consisting of formula G7 and G8,
wherein R₄ and R₅ are independently selected from C₁-C₄haloalkyl, preferably trifluoromethyl; and
wherein G₁ and G₂ are independently selected from N and CH.

In an especially preferred group of compounds of formula I-1, G₆₋₁ is selected from the group consisting of formula U-0, U-2, U-3 and U-5: 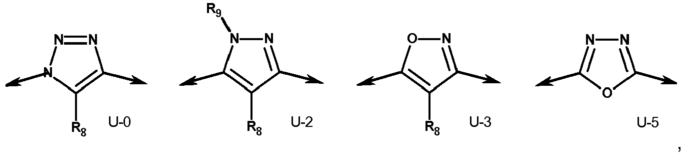 in particular selected from the group consisting of of formula U-0, U-3 and U-5,
wherein the arrow to the left of each group U denotes the point of attachment to the 6-membered ring containing G₁ and wherein the arrow to the right of each group U denotes the point of attachment to the radical R₇;
wherein each R₈ is independently selected from hydrogen and C₁-C₄alkyl; and wherein R₉ is C₁-C₄alkyl, preferably methyl.

In said especially preferred group of compounds of formula I-1, R₈ is preferably hydrogen or methyl, and R₉ is preferably methyl. A further preferred embodiment of said especially preferred group of compounds of formula I-1 comprises compounds wherein A is preferably N or CH, X₁ is preferably S or SO₂ and R₁₁ is preferably ethyl.

In compounds of formula I-1 and all of the preferred embodiments of compounds of formula I-1 mentioned above, Q is preferably selected from the group consisting of hydrogen, halogen, C₁-C₆haloalkoxy, C₁-C₆haloalkyl, C₁-C₆haloalkylsulfanyl, C₁-C₆haloalkylsulfinyl, C₁-C₆haloalkylsulfonyl, -C(O)C₁-C₄haloalkyl and from the group consisting of J-0 to J-48: 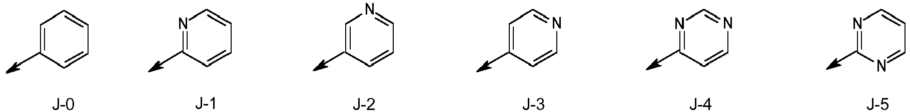 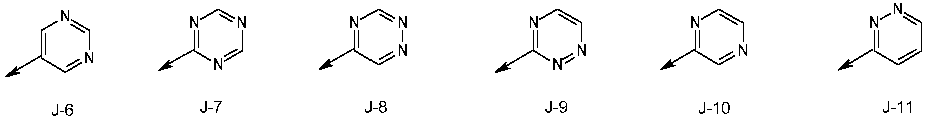 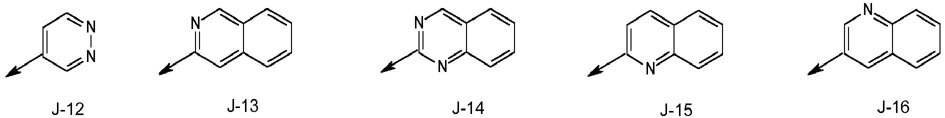 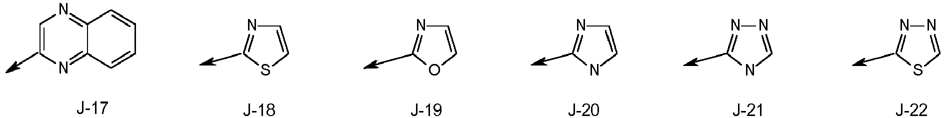 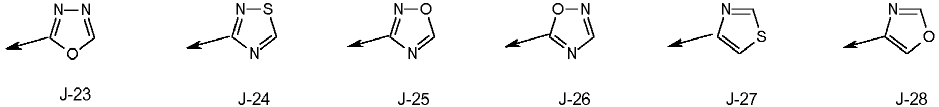 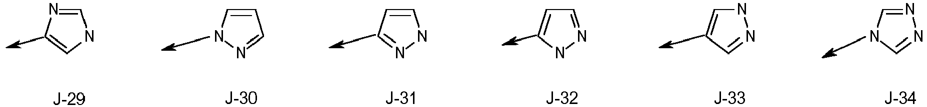 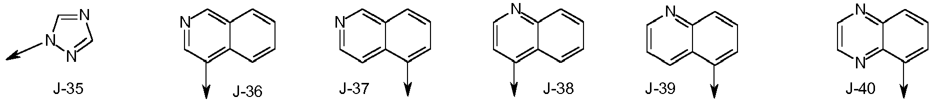 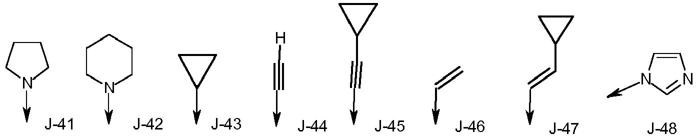 wherein each group J-0 to J-48 is mono-, di- or trisubstituted with Rx, wherein
each Rx is, independently selected from hydrogen, halogen, cyano, C₁-C₄alkyl, C₁-C₄haloalkyl, C₁-C₄haloalkoxy, C₁-C₄alkoxy, C₁-C₄alkylsulfanyl, C₁-C₄alkylsulfinyl, C₁-C₄alkylsulfonyl, -C(O)C₁-C₄alkyl, C₁-C₄haloalkylsulfanyl, C₁-C₄haloalkylsulfinyl, C₁-C₄haloalkylsulfonyl and -C(O)C₁-C₄haloalkyl.

In compounds of formula I-1 and all of the preferred embodiments of compounds of formula I-1 mentioned above, Q is even more preferably hydrogen, halogen, C₁-C₆haloalkyl and the group J-0; in particular Q is more preferably C₁-C₆haloalkyl.

Another preferred group of compounds of formula I is represented by the compounds of formula I-2 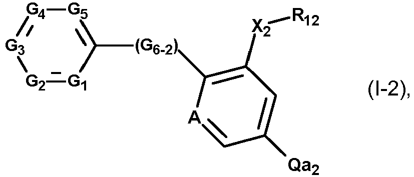 wherein A, G₁, G₂, G₃, G₄ and G₅ are as defined under formula I above; and wherein X₂ is S, SO or SO₂; and R₁₂ is methyl, ethyl, n-propyl, i-propyl or cyclopropylmethyl; and G₆₋₂ is selected from the group consisting of formula U-0, U-2, U-3 and U-5: 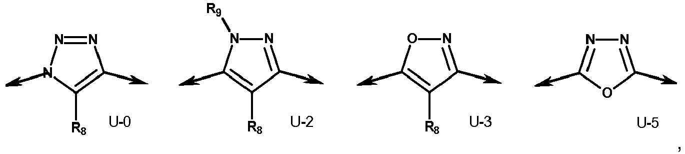 in particular selected from the group consisting of of formula U-0, U-3 and U-5,
wherein the arrow to the left of each group U denotes the point of attachment to the 6-membered ring containing G₁ and wherein the arrow to the right of each group U denotes the point of attachment to the radical R₇; and
wherein each R₈ is independently selected from hydrogen and C₁-C₄alkyl, preferably hydrogen and methyl; and wherein R₉ is C₁-C₄alkyl, preferably methyl; and wherein Qa₂ is selected from hydrogen, halogen, C₁-C₆haloalkoxy, C₁-C₆haloalkyl, C₁-C₆haloalkylsulfanyl, C₁-C₆haloalkylsulfinyl, C₁-C₆haloalkylsulfonyl, -C(O)C₁-C₄haloalkyl and from the group consisting of J-0 to J-48: 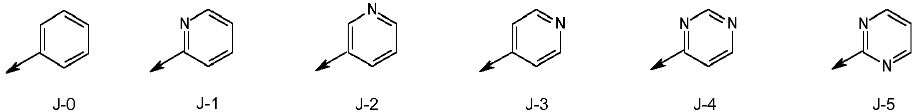 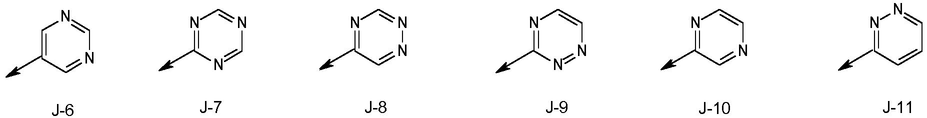 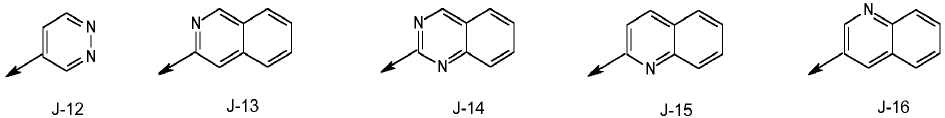 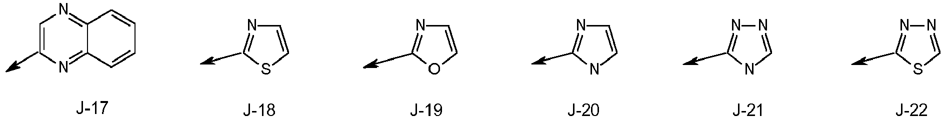 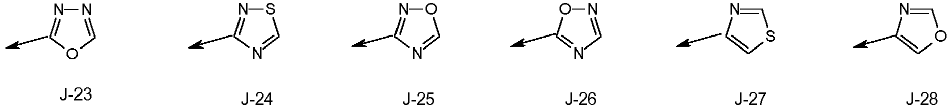 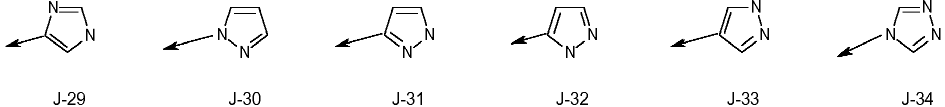 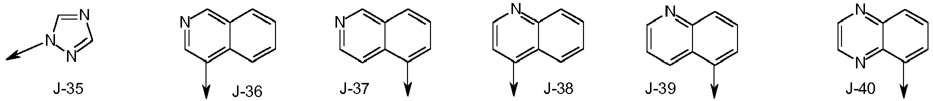 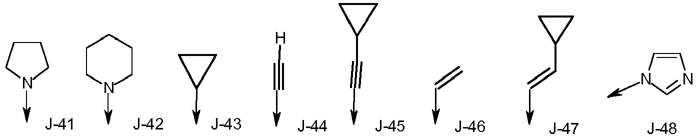 wherein each group J-0 to J-48 is mono-, di- or trisubstituted with Rx, wherein
   each Rx is, independently selected from hydrogen, halogen, cyano, C₁-C₄alkyl, C₁-C₄haloalkyl, C₁-C₄haloalkoxy, C₁-C₄alkoxy, C₁-C₄alkylsulfanyl, C₁-C₄alkylsulfinyl, C₁-C₄alkylsulfonyl, -C(O)C₁-C₄alkyl, C₁-C₄haloalkylsulfanyl, C₁-C₄haloalkylsulfinyl, C₁-C₄haloalkylsulfonyl and -C(O)C₁-C₄haloalkyl; and agrochemically acceptable salts, stereoismers, enantiomers, tautomers and N-oxides of those compounds.

More preferred are compounds of formula I-2 wherein Qa₂ is selected from hydrogen, halogen, C₁-C₆haloalkyl and the group J-0; in particular Qa₂ is C₁-C₆haloalkyl.

Even more preferred compounds of formula I-2 are those, in which Qa₂ is trifluoromethyl. In said especially preferred group of compounds of formula I-2, R₈ is preferably hydrogen or methyl, and R₉ is preferably methyl. A further preferred embodiment of said especially preferred group of compounds of formula I-2 comprises compounds wherein A is preferably N or CH, X₂ is preferably S or SO₂ and R₁₂ is preferably ethyl.

Other more preferred compounds of formula I-2 are those, in which Qa₂ is hydrogen. In said especially preferred group of compounds of formula I-2, R₈ is preferably hydrogen or methyl, and R₉ is preferably methyl. A further preferred embodiment of said especially preferred group of compounds of formula I-2 comprises compounds wherein A is preferably N or CH, X₂ is preferably S or SO₂ and R₁₂ is preferably ethyl.

Other more preferred compounds of formula I-2 are those, in which Qa₂ is halogen, preferably bromine. In said especially preferred group of compounds of formula I-2, R₈ is preferably hydrogen or methyl, and R₉ is preferably methyl. A further preferred embodiment of said especially preferred group of compounds of formula I-2 comprises compounds wherein A is preferably N or CH, X₂ is preferably S or SO₂ and R₁₂ is preferably ethyl.

Other more preferred compounds of formula I-2 are those, in which Qa₂ is the group J-0 mono-substituted with Rx, wherein Rx is halogen, preferably chlorine. In said especially preferred group of compounds of formula I-2, R₈ is preferably hydrogen or methyl, and R₉ is preferably methyl. A further preferred embodiment of said especially preferred group of compounds of formula I-2 comprises compounds wherein A is preferably N or CH, X₂ is preferably S or SO₂ and R₁₂ is preferably ethyl.

In compounds of formula I-2 and all of the preferred embodiments of compounds of formula I-2 mentioned above, the radical 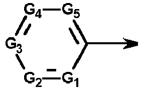 is preferably phenyl, pyridyl or pyrimidinyl mono- or polysubstituted by substituents independently selected from C₁-C₄haloalkyl, C₁-C₄haloalkylsulfanyl, C₁-C₄haloalkylsulfinyl, C₁-C₄haloalkylsulfonyl, C₁-C₄haloalkoxy and -C(O)C₁-C₄haloalkyl.

In compounds of formula I-2 and all of the preferred embodiments of compounds of formula I-2 mentioned above, the radical 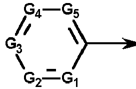 is even more preferably selected from the group consisting of formula G7, G8 and G9: 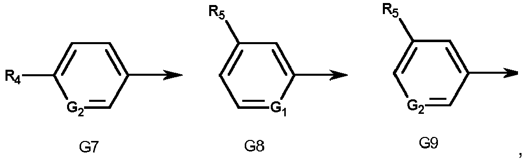 in particular selected from the group consisting of formula G7 and G8,
wherein R₄ and R₅ are independently selected from C₁-C₄haloalkyl, preferably trifluoromethyl; and
wherein G₁ and G₂ are independently selected from N and CH.

A further preferred embodiment of the document comprises compounds of formula I represented by the compounds of formula I-3 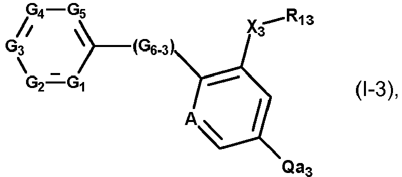 wherein
A is N or CH;
the radical 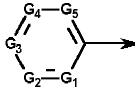 is phenyl or pyridyl both mono-substituted by substituents independently selected from the group consisting of C₁-C₄haloalkyl, in particular trifluoromethyl;
G₆₋₃ is selected from the group consisting of formula U-0, U-2, U-3 and U-5: 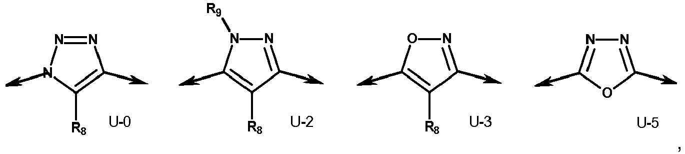
in particular selected from the group consisting of of formula U-0, U-3 and U-5,
wherein the arrow to the left of each group U denotes the point of attachment to the 6-membered ring containing G₁ and wherein the arrow to the right of each group U denotes the point of attachment to the radical R₇; and
wherein each R₈ is independently selected from hydrogen and C₁-C₄alkyl, in particular hydrogen and methyl;
R₉ is C₁-C₄alkyl, preferably methyl;
X₃ is S, SO or SO₂, in particular S or SO₂, preferably SO₂;
Qa₃ is selected from the group consisting of hydrogen, halogen, preferably bromine, C₁-C₆haloalkyl, in particular trifluoromethyl ; and the group J-0 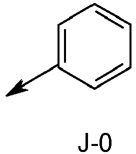
wherein J-0 can be mono-substituted with Rx, wherein Rx is halogen, preferably chlorine; in particular Qa₃ is C₁-C₆haloalkyl, in particular trifluoromethyl;
R₁₃ is C₁-C₄alkyl, in particular methyl or ethyl; and
agrochemically acceptable salts, stereoisomers, enantiomers, tautomers and N-oxides of the compounds of formula I-3.

In said preferred embodiment comprising compounds of formula I-3, the radical 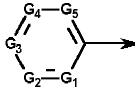 is preferably selected from the group consisting of formula G7, G8 and G9: 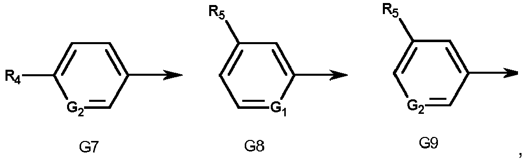 in particular selected from the group consisting of formula G7 and G8,
wherein R₄ and R₅ are independently selected from C₁-C₄haloalkyl, preferably trifluoromethyl; and
wherein G₁ and G₂ are independently selected from N and CH.

The present invention relates to a compound of formula (1-4) 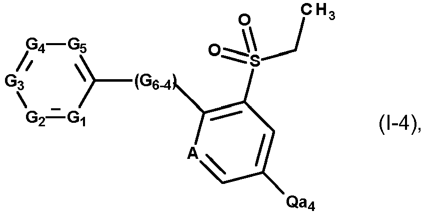
wherein A is N or CH;
the radical 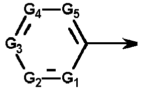 is selected from the group consisting of formula G7, G8 and G9: 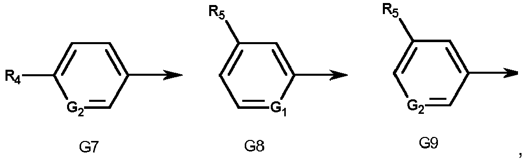 wherein R₄ and R₅ are independently selected from C₁-C₄haloalkyl; and wherein G₁ and G₂ are independently selected from N and CH; G₆₋₄ is of formula U-0 : 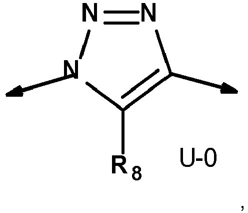 wherein the arrow to the left of each group U denotes the point of attachment to the 6-membered ring containing G₁ and wherein the arrow to the right of group U-0 denotes the point of attachment to the radical containing A; and wherein R₈ is selected from hydrogen and C₁-C₄alkyl; Qa₄ is selected from the group consisting of hydrogen, halogen, C₁-C₆haloalkyl and the group J-0 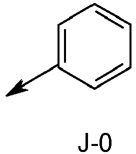 wherein J-0 can be mono-substituted with Rx, wherein Rx is halogen; and agrochemically acceptable salts, stereoismers, enantiomers, tautomers and N-oxides of the compounds of formula (I-4).

Preferably R₄ and R₅ are trifluoromethyl.

Another preferred group of compounds of formula I is represented by the compounds of formula I-1p 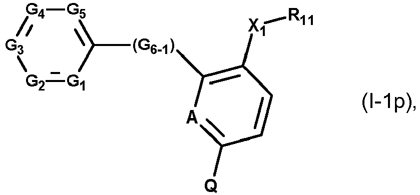 wherein A, Q, G₁, G₂, G₃, G₄ and G₅ are as defined under formula I above; and wherein X₁ is S, SO or SO₂; and R₁₁ is methyl, ethyl, n-propyl, i-propyl or cyclopropylmethyl; and G₆₋₁ is selected from the group consisting of formula U-0, U-1, U-2, U-3 and U-5: 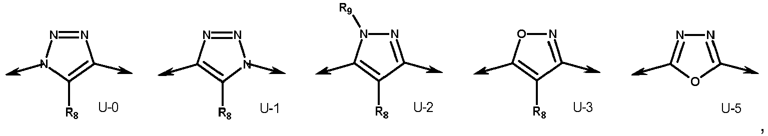
wherein the arrow to the left of each group U denotes the point of attachment to the 6-membered ring containing G₁ and wherein the arrow to the right of each group U denotes the point of attachment to the radical R₇; and
wherein each R₈ is independently selected from hydrogen and C₁-C₄alkyl, preferably hydrogen and methyl; and wherein R₉ is selected from hydrogen and C₁-C₄alkyl, preferably hydrogen and methyl; and agrochemically acceptable salts, stereoismers, enantiomers, tautomers and N-oxides of those compounds.

Also preferred are compounds of formula I-1p wherein the radical 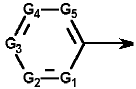 is phenyl, pyridyl or pyrimidinyl mono- or polysubstituted by substituents independently selected from C₁-C₄haloalkyl, C₁-C₄haloalkylsulfanyl, C₁-C₄haloalkylsulfinyl, C₁-C₄haloalkylsulfonyl, C₁-C₄haloalkoxy and -C(O)C₁-C₄haloalkyl.

More preferred are compounds of formula I-1p wherein the radical 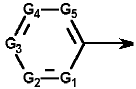 is selected from the group consisting of formula G7, G8 and G9: 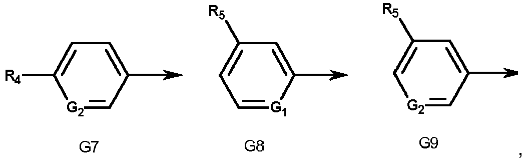 wherein R₄ and R₅ are independently selected from C₁-C₄haloalkyl, preferably trifluoromethyl; and wherein G₁ and G₂ are independently selected from N and CH, preferably N.

In an especially preferred group of compounds of formula I-1p, G₆₋₁ is selected from the group consisting of formula U-0, U-2, U-3 and U-5: 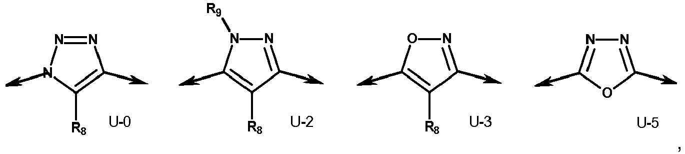 preferably G₆₋₁ is U-2,
wherein the arrow to the left of each group U denotes the point of attachment to the 6-membered ring containing G₁ and wherein the arrow to the right of each group U denotes the point of attachment to the radical R₇;
wherein each R₈ is independently selected from hydrogen and C₁-C₄alkyl; and wherein R₉ is C₁-C₄alkyl, preferably methyl.

In said especially preferred group of compounds of formula I-1p, R₈ is preferably hydrogen or methyl, and R₉ is preferably methyl. A further preferred embodiment of said especially preferred group of compounds of formula I-1p comprises compounds wherein A is preferably N or CH, X₁ is preferably S or SO₂ and R₁₁ is preferably ethyl.

In compounds of formula I-1p and all of the preferred embodiments of compounds of formula I-1p mentioned above, Q is preferably selected from the group consisting of hydrogen, halogen, C₁-C₆haloalkoxy, C₁-C₆haloalkyl, C₁-C₆haloalkylsulfanyl, C₁-C₆haloalkylsulfinyl, C₁-C₆haloalkylsulfonyl, -C(O)C₁-C₄haloalkyl and from the group consisting of J-0 to J-48: 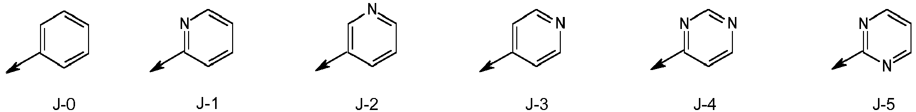 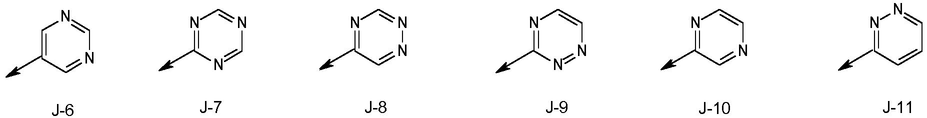 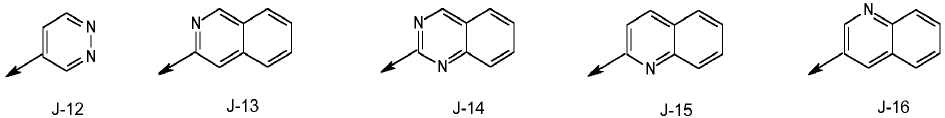 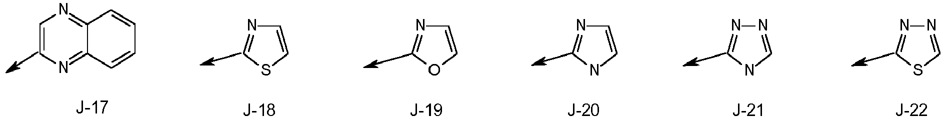 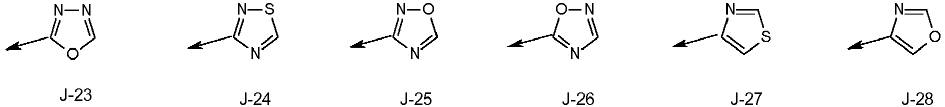 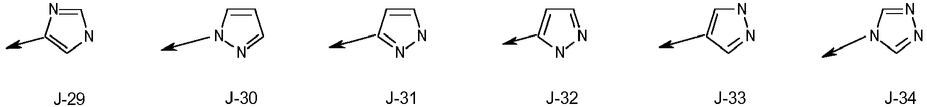 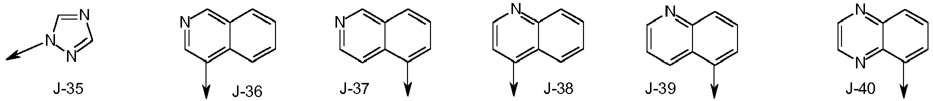 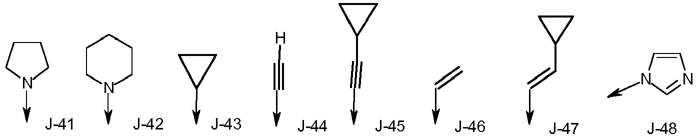 wherein each group J-0 to J-48 is mono-, di- or trisubstituted with Rx, wherein
each Rx is, independently selected from hydrogen, halogen, cyano, C₁-C₄alkyl, C₁-C₄haloalkyl, C₁-C₄haloalkoxy, C₁-C₄alkoxy, C₁-C₄alkylsulfanyl, C₁-C₄alkylsulfinyl, C₁-C₄alkylsulfonyl, -C(O)C₁-C₄alkyl, C₁-C₄haloalkylsulfanyl, C₁-C₄haloalkylsulfinyl, C₁-C₄haloalkylsulfonyl and -C(O)C₁-C₄haloalkyl.

In compounds of formula I-1p and all of the preferred embodiments of compounds of formula I-1p mentioned above, Q is even more preferably hydrogen, halogen, and the group J-35.

Another preferred group of compounds of formula I is represented by the compounds of formula I-2p 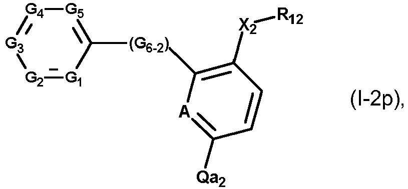 wherein A, G₁, G₂, G₃, G₄ and G₅ are as defined under formula I above; and wherein X₂ is S, SO or SO₂; and R₁₂ is methyl, ethyl, n-propyl, i-propyl or cyclopropylmethyl; and G₆₋₂ is selected from the group consisting of formula U-0, U-2, U-3 and U-5: 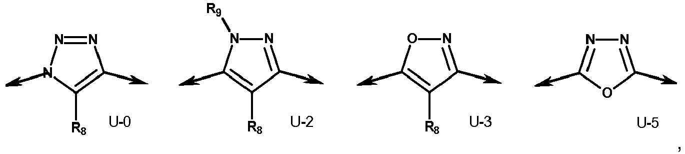 preferably G₆₋₂ is U-2,
wherein the arrow to the left of each group U denotes the point of attachment to the 6-membered ring containing G₁ and wherein the arrow to the right of each group U denotes the point of attachment to the radical R₇; and
wherein each R₈ is independently selected from hydrogen and C₁-C₄alkyl, preferably hydrogen and methyl; and wherein R₉ is C₁-C₄alkyl, preferably methyl; and wherein Qa₂ is selected from hydrogen, halogen, C₁-C₆haloalkoxy, C₁-C₆haloalkyl, C₁-C₆haloalkylsulfanyl, C₁-C₆haloalkylsulfinyl, C₁-C₆haloalkylsulfonyl, -C(O)C₁-C₄haloalkyl and from the group consisting of J-0 to J-48: 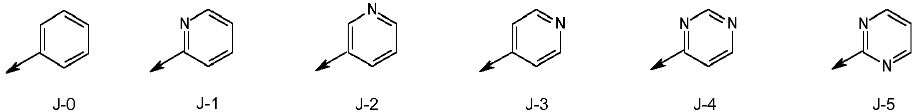 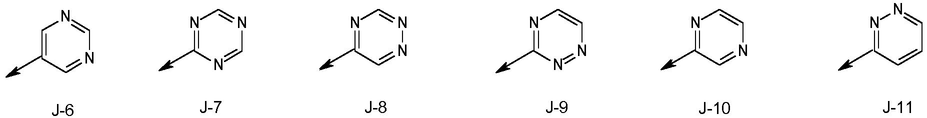 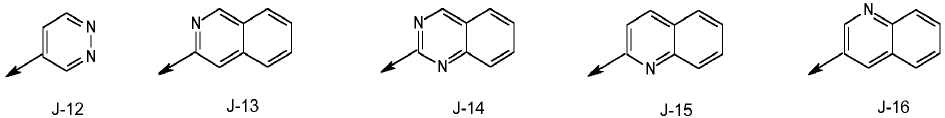 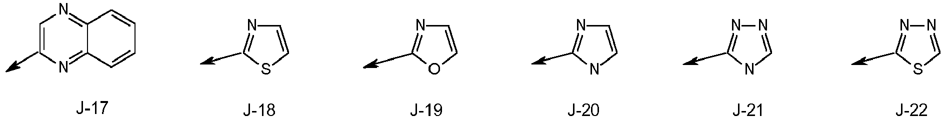 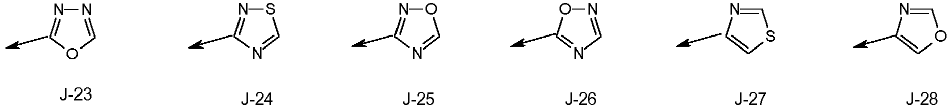 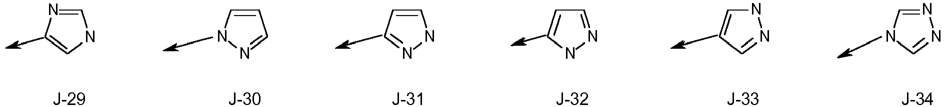 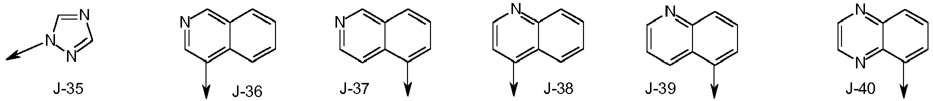 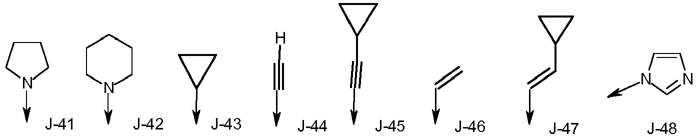 wherein each group J-0 to J-48 is mono-, di- or trisubstituted with Rx, wherein
   each Rx is, independently selected from hydrogen, halogen, cyano, C₁-C₄alkyl, C₁-C₄haloalkyl, C₁-C₄haloalkoxy, C₁-C₄alkoxy, C₁-C₄alkylsulfanyl, C₁-C₄alkylsulfinyl, C₁-C₄alkylsulfonyl, -C(O)C₁-C₄alkyl, C₁-C₄haloalkylsulfanyl, C₁-C₄haloalkylsulfinyl, C₁-C₄haloalkylsulfonyl and -C(O)C₁-C₄haloalkyl; and agrochemically acceptable salts, stereoismers, enantiomers, tautomers and N-oxides of those compounds.

More preferred are compounds of formula I-2p wherein Qa₂ is selected from hydrogen, halogen and the group J-35.

Even more preferred compounds of formula I-2p are those, in which Qa₂ is hydrogen. In said especially preferred group of compounds of formula I-2p, R₈ is preferably hydrogen or methyl, and R₉ is preferably methyl. A further preferred embodiment of said especially preferred group of compounds of formula I-2p comprises compounds wherein A is preferably N or CH, X₂ is preferably S or SO₂ and R₁₂ is preferably ethyl.

Other more preferred compounds of formula I-2p are those, in which Qa₂ is halogen, preferably chlorine. In said especially preferred group of compounds of formula I-2p, R₈ is preferably hydrogen or methyl, and R₉ is preferably methyl. A further preferred embodiment of said especially preferred group of compounds of formula I-2p comprises compounds wherein A is preferably N or CH, X₂ is preferably S or SO₂ and R₁₂ is preferably ethyl.

Other more preferred compounds of formula I-2p are those, in which Qa₂ is the group J-35 mono-substituted with Rx, wherein Rx is hydrogen or halogen, preferably hydrogen. In said especially preferred group of compounds of formula I-2p, R₈ is preferably hydrogen or methyl, and R₉ is preferably methyl. A further preferred embodiment of said especially preferred group of compounds of formula I-2p comprises compounds wherein A is preferably N or CH, X₂ is preferably S or SO₂ and R₁₂ is preferably ethyl.

In compounds of formula I-2p and all of the preferred embodiments of compounds of formula I-2p mentioned above, the radical 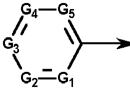 is preferably phenyl or pyridyl mono- or polysubstituted by substituents independently selected from C₁-C₄haloalkyl, C₁-C₄haloalkylsulfanyl, C₁-C₄haloalkylsulfinyl, C₁-C₄haloalkylsulfonyl, C₁-C₄haloalkoxy and -C(O)C₁-C₄haloalkyl.

In compounds of formula I-2p and all of the preferred embodiments of compounds of formula I-2p mentioned above, the radical 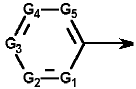 is even more preferably selected from the group consisting of formula G7, G8 and G9: 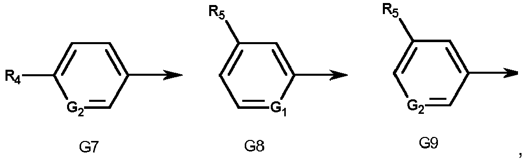
wherein R₄ and R₅ are independently selected from C₁-C₄haloalkyl, preferably trifluoromethyl; and
wherein G₁ and G₂ are independently selected from N and CH, preferably N.

A further preferred embodiment of the documentcomprises compounds of formula I represented by the compounds of formula I-3p 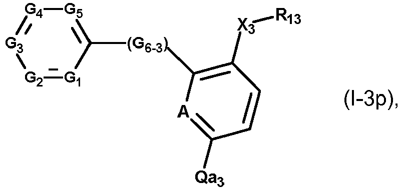 wherein
A is N or CH, preferably N;
the radical 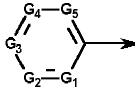 is phenyl or pyridyl, preferably pyridyl, both mono-substituted by substituents independently selected from the group consisting of C₁-C₄haloalkyl, in particular trifluoromethyl;
G₆₋₃ is represented by the formula U-2: 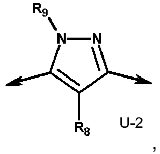
wherein the arrow to the left of each group U denotes the point of attachment to the 6-membered ring containing G₁ and wherein the arrow to the right of each group U denotes the point of attachment to the radical R₇; and
wherein R₈ is independently selected from hydrogen and C₁-C₄alkyl, in particular hydrogen and methyl; preferably R₈ is hydrogen;
R₉ is C₁-C₄alkyl, preferably methyl;
X₃ is S, SO or SO₂, in particular S or SO₂, preferably SO₂;
Qa₃ is selected from the group consisting of hydrogen, halogen, preferably chlorine and the group J-35 mono-substituted with Rx, wherein Rx is hydrogen or halogen, preferably hydrogen;
R₁₃ is C₁-C₄alkyl, in particular methyl or ethyl; and
agrochemically acceptable salts, stereoisomers, enantiomers, tautomers and N-oxides of the compounds of formula I-3p.

In said preferred embodiment comprising compounds of formula I-3p, the radical 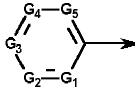 is preferably selected from the group consisting of formula G7, G8 and G9: 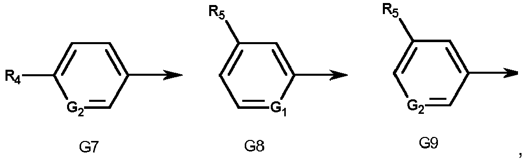
wherein R₄ and R₅ are independently selected from C₁-C₄haloalkyl, preferably trifluoromethyl; and
wherein G₁ and G₂ are independently selected from N and CH, preferably N.

This document alos discloses compounds of formula I represented by the compounds of formula I-4p 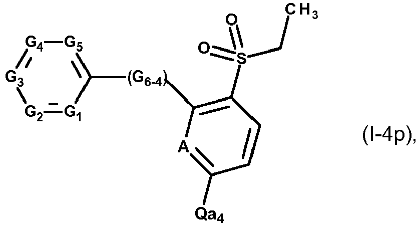 wherein
A is N;
the radical 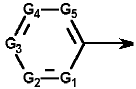 is selected from the group consisting of formula G7, G8 and G9: 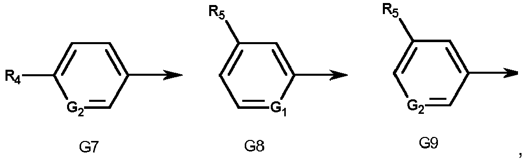
wherein R₄ and R₅ are independently selected from C₁-C₄haloalkyl, preferably trifluoromethyl; and
wherein G₁ and G₂ are independently selected from N;
G₆₋₄ is represented by the formula U-2: 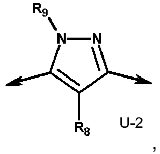
wherein the arrow to the left of each group U denotes the point of attachment to the 6-membered ring containing G₁ and wherein the arrow to the right of each group U denotes the point of attachment to the radical R₇; and
wherein R₈ is hydrogen; and
R₉ is C₁-C₄alkyl, preferably methyl; and
Qa₄ is selected from the group consisting of hydrogen, halogen, preferably chlorine and the group J-35 mono-substituted with Rx, wherein Rx is hydrogen or halogen, preferably hydrogen;
and agrochemically acceptable salts, stereoisomers, enantiomers, tautomers and N-oxides of the compounds of formula I-4.

In an outstanding group of compounds of formula I, the ring, which is formed by the groups G₁ to G₅, represents phenyl or pyridyl, which both can be substituted by C₁-C₄haloalkyl;
G₆ is pyrazolyl N-substituted by C₁-C₄alkyl, triazolyl, oxadiazolyl or isoxazolyl, or isoxazolyl substituted by C₁-C₄alkyl; in particular is triazolyl, oxadiazolyl or isoxazolyl, or isoxazolyl substituted by C₁-C₄alkyl; and
R₇ is phenyl or pyridyl, which both are substituted by ethylsulfonyl or ethylsufanyl, preferably substituted by ethylsulfonyl or ethylsufanyl at the ortho-position with respect to the bond joining said phenyl or pyridyl to the radical G₆; said phenyl or pyridyl can be further substituted by hydrogen, halogen, C₁-C₄haloalkyl, halophenyl and triazolyl, in particular said phenyl or pyridyl can be further substituted by C₁-C₄haloalkyl.

The group U is preferably different from U-5 and U-6.

The process for preparing compounds of formula I is carried out by methods known to those skilled in the art. More specifically, the subgroup of compounds of formula I, wherein X is SO (sulfoxide) and/or SO₂ (sulfone), may be obtained by means of an oxidation reaction of the corresponding sulfide compounds of formula I, wherein X is S, involving reagents such as, for example, m-chloroperoxybenzoic acid (mCPBA), hydrogen peroxide, oxone, sodium periodate, sodium hypochlorite or tert-butyl hypochlorite amongst other oxidants. The oxidation reaction is generally conducted in the presence of a solvent. Examples of the solvent to be used in the reaction include aliphatic halogenated hydrocarbons such as dichloromethane and chloroform; alcohols such as methanol and ethanol; acetic acid; water; and mixtures thereof. The amount of the oxidant to be used in the reaction is generally 1 to 3 moles, preferably 1 to 1.2 moles, relative to 1 mole of the sulfide compounds I to produce the sulfoxide compounds I, and preferably 2 to 2.2 moles of oxidant, relative to 1 mole of of the sulfide compounds I to produce the sulfone compounds I. Such oxidation reactions are disclosed, for example, in WO 2013/018928.

Compounds of formula I, wherein R₇, G₆, G₁, G₂, G₃, G₄, and G₅ are as defined in formula I and wherein X is S (sulfide), 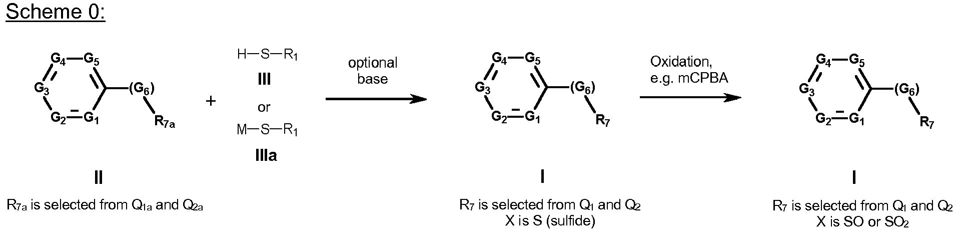 can be prepared (scheme 0) by reacting a compound of formula II, wherein G₆, G₁, G₂, G₃, G₄, and G₅ are as described in formula I and wherein R₇ₐ is a radical selected from the group consisting of formula Q₁ₐ to Q₂ₐ: 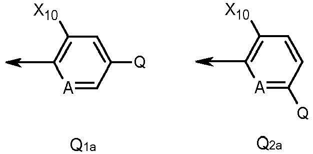 wherein A is as defined in formula I, and wherein X₁₀ is a halogen (preferably fluorine, chlorine or bromine), with a compound of formula III

R₁-SH (III),

or a salt thereof, wherein R₁ is as defined in formula I, optionally in the presence of a suitable base, such as alkali metal carbonates, for example sodium carbonate and potassium carbonate, or alkali metal hydrides such as sodium hydride, or alkali metal hydroxides such as sodium hydroxide and potassium hydroxide, in an inert solvent at temperatures preferably between 25-120°C. Examples of solvent to be used include ethers such as THF, ethylene glycol dimethyl ether, tert-butylmethyl ether, and 1,4-dioxane, aromatic hydrocarbons such as toluene and xylene, nitriles such as acetonitrile or polar aprotic solvents such as N,N-dimethylformamide, N,N-dimethylacetamide, N-methyl-2-pyrrolidone or dimethyl sulfoxide. Examples of salts of the compound of formula III include compounds of the formula IIIa

R₁-S-M (IIIa),

wherein R₁ is as defined above and wherein M is, for example, sodium or potassium.

The subgroup of compounds of formula I, wherein G₆, G₁, G₂, G₃, G₄, and G₅ are as defined above and wherein R₇ is defined as Q₁, in which A, Q, X and R₁ are as defined above, may be defined as compounds of formula I-Q₁. Compounds of formula I-Q₁, wherein X is SO or SO₂, 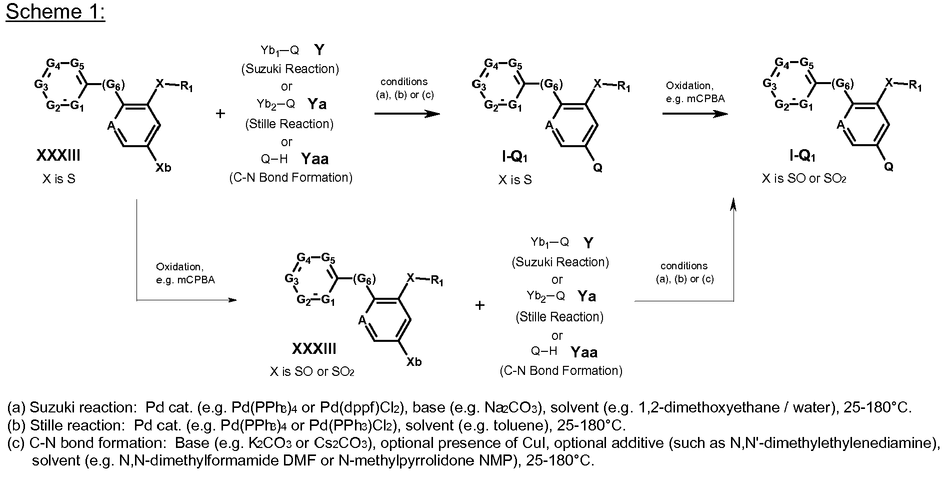 may be prepared by a Suzuki reaction (scheme 1), which involves for example, reacting compounds of formula XXXIII, wherein A, R₁, G₆, G₁, G₂, G₃, G₄, and G₅ are as defined above, and in which X is SO or SO₂, and wherein Xb is a leaving group like, for example, chlorine, bromine or iodine (preferably bromine), or an aryl- or alkylsulfonate such as trifluoromethanesulfonate, with compounds of formula Y, wherein Q is as defined above, and wherein Y_{b1} can be a boron-derived functional group, such as for example B(OH)₂ or B(OR_{b1})₂ wherein R_{b1} can be a C₁-C₄alkyl group or the two groups OR_{b1} can form together with the boron atom a five membered ring, as for example a pinacol boronic ester. The reaction may be catalyzed by a palladium based catalyst, for example tetrakis(triphenylphosphine)-palladium(0), (1,1'bis(diphenylphosphino)ferrocene)dichloro-palladium-dichloromethane (1:1 complex) or chloro(2-dicyclohexylphosphino-2',4',6'-triisopropyl-1,1'-biphenyl)[2-(2'-amino-1,1'-biphenyl)] palladium(II) (XPhos palladacycle), in presence of a base, like sodium carbonate, tripotassium phosphate or cesium fluoride, in a solvent or a solvent mixture, like, for example dioxane, acetonitrile, N,N-dimethylformamide, a mixture of 1,2-dimethoxyethane and water or of dioxane/water, or of toluene/water, preferably under inert atmosphere. The reaction temperature can preferentially range from room temperature to the boiling point of the reaction mixture, or the reaction may be performed under microwave irradiation. Such Suzuki reactions are well known to those skilled in the art and have been reviewed, for example, in J.Orgmet. Chem. 576, 1999, 147-168.

Alternatively compounds of formula I-Q₁, wherein X is SO or SO₂, may be prepared by a Stille reaction between compounds of formula Ya, wherein Q is as defined above, and wherein Y_{b2} is a trialkyl tin derivative, preferably tri-n-butyl tin or tri-methyl-tin, and compounds of formula XXXIII, wherein A, R₁, G₆, G₁, G₂, G₃, G₄, and G₅ are as defined above, and in which X is SO or SO₂, and wherein Xb is a leaving group like, for example, chlorine, bromine or iodine (preferably bromine), or an aryl- or alkylsulfonate such as trifluoromethanesulfonate. Such Stille reactions are usually carried out in the presence of a palladium catalyst, for example tetrakis(triphenylphosphine)palladium(0), or bis(triphenylphosphine) palladium(II) dichloride, in an inert solvent such as N,N-dimethylformamide, acetonitrile, toluene or dioxane, optionally in the presence of an additive, such as cesium fluoride, or lithium chloride, and optionally in the presence of a further catalyst, for example copper(I)iodide. Such Stille couplings are also well known to those skilled in the art, and have been described in for example J. Org. Chem., 2005, 70, 8601-8604, J. Org. Chem., 2009, 74, 5599-5602, and Angew. Chem. Int. Ed., 2004, 43, 1132-1136.

When Q is a five- to six-membered, aromatic, partially saturated or fully saturated ring system linked via a nitrogen atom to the ring which contains the group A, then compounds of formula I-Q₁, wherein X is SO or SO₂, may be prepared from compounds of formula XXXIII, wherein A, R₁, G₆, G₁, G₂, G₃, G₄, and G₅ are as defined above, and in which X is SO or SO₂, and wherein Xb is a leaving group like, for example, chlorine, bromine or iodine (preferably bromine), or an aryl- or alkylsulfonate such as trifluoromethanesulfonate, by reaction with a heterocycle Q-H (which contains an appropriate NH functionality) Yaa, wherein Q is as defined above, in the presence of a base, such as potassium carbonate K₂CO₃ or cesium carbonate Cs₂CO₃, optionally in the presence of a copper catalyst, for example copper(I) iodide, with or without an additive such as L-proline, N,N'-dimethylcyclohexane-1,2-diamine or N,N'-dimethylethylene-diamine, in an inert solvent such as N-methylpyrrolidone NMP or N,N-dimethylformamide DMF at temperatures between 30-150°C, optionally under microwave irradiation. Such a reaction (C-N Bond Formation) is illustrated below (scheme 2) for the heterocycle Q-H J-30, wherein J30 is as defined above, 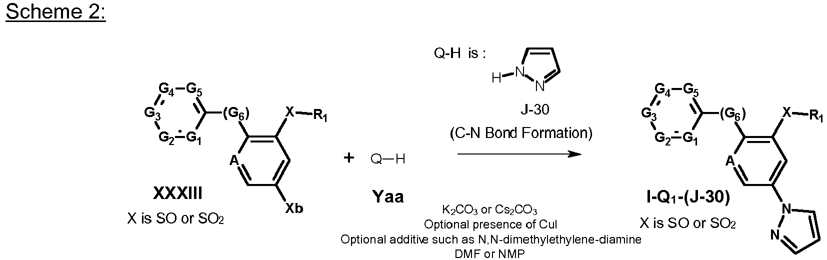 to give compounds of formula I-Q1-(J-30), a particular sub-group of compounds of formula I-Q₁, wherein A, R₁, G₆, G₁, G₂, G₃, G₄, and G₅ are as previously defined.

Oxidation of compounds of formula XXXIII, wherein A, R₁, G₆, G₁, G₂, G₃, G₄, and G₅ are as defined above, and in which X is S (sulfide), and wherein Xb is a leaving group like, for example, chlorine, bromine or iodine (preferably bromine), or an aryl- or alkylsulfonate such as trifluoromethanesulfonate, with a suitable oxidizing agent, into compounds of formula XXXIII, wherein X is SO or SO₂ may be achieved under conditions already described above.

A large number of compounds of the formula Y, Ya and Yaa are commercially available or can be prepared by those skilled in the art.

Alternatively, compounds of formula I-Q₁, wherein X is SO or SO₂, may be prepared from compounds of formula XXXIII, wherein X is S (sulfide) by involving the same chemistry as described above, but by changing the order of the steps (i.e. by running the sequence XXXIII (X is S) to I-Q₁ (X is S) via Suzuki, Stille or C-N bond formation, followed by an oxidation step to form I-Q₁ (X is SO or SO₂).

In the particular situation within scheme 1 when Q is an optionally substituted triazole linked via a nitrogen atom to the ring which contains the group A, then compounds of formula I-Q₁, wherein X is SO or SO₂, may be prepared from compounds of formula XXXIII, wherein A, R₁, G₆, G₁, G₂, G₃, G₄, and G₅ are as defined above, and in which X is S, SO or SO₂, and wherein Xb is a leaving group like, for example, chlorine, bromine or iodine (preferably bromine or chlorine), or an aryl- or alkylsulfonate such as trifluoromethanesulfonate, by reaction with an optionally substituted triazole Q-H (which contains an appropriate NH functionality) Yaa, wherein Q is N-linked triazolyl, in solvents such as alcohols (eg. methanol, ethanol, isopropanol, or higher boiling linear or branched alcohols), pyridine or acetic acid, optionally in the presence of an additional base, such as potassium carbonate K₂CO₃ or cesium carbonate Cs₂CO₃, optionally in the presence of a copper catalyst, for example copper(I) iodide, at temperatures between 30-180°C, optionally under microwave irradiation. The oxidation state of X in compounds of formula XXXIII and I-Q₁ may be adjusted by an additional oxidation step under conditions already described above.

In the particular situation within scheme 1 when Q is hydrogen, then compounds of formula I-Q₁, wherein X is S, SO or SO₂, may be prepared from compounds of formula XXXIII, wherein A, R₁, G₆, G₁, G₂, G₃, G₄, and G₅ are as defined above, and in which X is S, SO or SO₂, and wherein Xb is a leaving group like, for example, chlorine, bromine or iodine (preferably bromine or chlorine), by a dehalogenation step. Such a dehalogenation reaction of (het)arylhalides may be achieved under various conditions, such as, for example, by the use of zinc in a tetrahydrofuran/saturated aqueous ammonium chloride media system (J. Chinese Chem. Soc. 55 (3), 616-618, 2008), or by the use of catalytic hydrogenation conditions (known to a person skilled in the art, typically hydrogen under atmosphere or high pressure, in presence of a catalyst, such as palladium on charcoal or the like, optionally in presence of a base such as alkali hydroxide, ammonia or triethylamine), or by means of a palladium-catalyzed reduction using alkylammonium formates as a hydrogen donor (described, for example, by N. A. Cortese and R. F. Heck in J. Org. Chem. 42 (22), 3491,1977). Those catalytic hydrodehalogenation conditions by a transfer hydrogenation method may typically involve a palladium based catalyst, such as palladium on charcoal or for example palladium(II) dichloride or palladium(II) diacetate, an optional phosphine ligand such as triphenylphosphine or tri-ortho-tolylphosphine, formic acid, a trialkylamine such as triethylamine (when using triethylammonium formate as hydrogen donor), in an inert solvent such as N,N-dimethylformamide, toluene or dioxane. The reaction temperature can preferentially range from room temperature to the boiling point of the reaction mixture, or the reaction may be performed under microwave irradiation.

The subgroup of compounds of formula I, wherein G₆, G₁, G₂, G₃, G₄, and G₅ are as defined above and wherein R₇ is defined as Q₂, in which A, Q, X and R₁ are as defined above, may be defined as compounds of formula I-Q₂ (scheme 3).

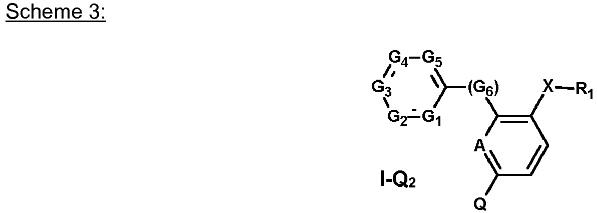

The chemistry described previously in schemes 0 to 3 to access compounds of formula I-Q₁, as well as relevant intermediates (see text, descriptions and preparation methods associated to schemes 0 to 3), may be applied analogously for the preparation of compounds of formula I-Q₂, possibly by changing the order of certain steps in a sequence and by slightly adapting reaction conditions in a manner known to a person skilled in the art. Such a transposition is illustrated in scheme 4 for the preparation of compounds of formula I-Q₂ from compounds of the formula XXXIII-p, wherein all substituent definitions mentioned previously are also valid for the compounds shown.

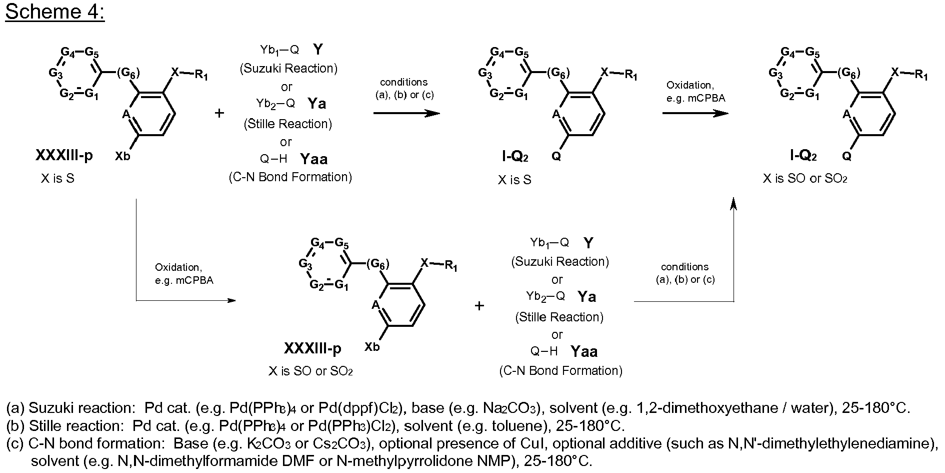

The chemistry in the particular situation when Q is an optionally substituted triazole linked via a nitrogen atom to the ring which contains the group A or when Q is hydrogen described above in the context of scheme 1 may be applied analogously for the preparation of compounds of formula I-Q2 wherein Q is defined as said optionally substituted triazole linked via a nitrogen atom to the ring which contains the group A or wherein Q is said hydrogen, and wherein all other substituent definitions mentioned previously are also valid for such compounds of the formula I-Q2.

The subgroup of compounds of formula II, wherein G₁, G₂, G₃, G₄ and G₅ are as described in formula I and wherein R₇ₐ is a radical selected from the group consisting of formula Q₁ₐ to Q₂ₐ, and in which G₆ is represented by the formula U-5, may be defined as compounds of formula II-U-5. Compounds of formula II-U-5, 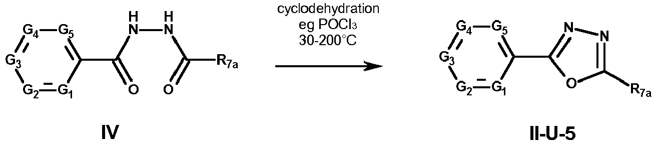 can be prepared by cyclodehydration of an N,N'-diacylhydrazine compounds of formula IV, for example by reacting a compound of formula IV, wherein R₇ₐ, G₁, G₂, G₃, G₄ and G₅ are as described above, in presence of phosphoryl chloride POCl₃ or thionyl chloride SOCl₂ (alternatively in presence of dehydrating agents, such as phosphorus pentoxide or the Burgess reagent), optionally in presence of an inert diluent, at temperatures between 30-200°C, preferably 50-180°C, with optional use of microwave heating. Examples of such a transformation may be found in, for example, Chemistry - A European Journal, 16(19), 5794-5802; 2010.

Compounds of formula IV, wherein R₇ₐ, G₁, G₂, G₃, G₄ and G₅ are as described above, 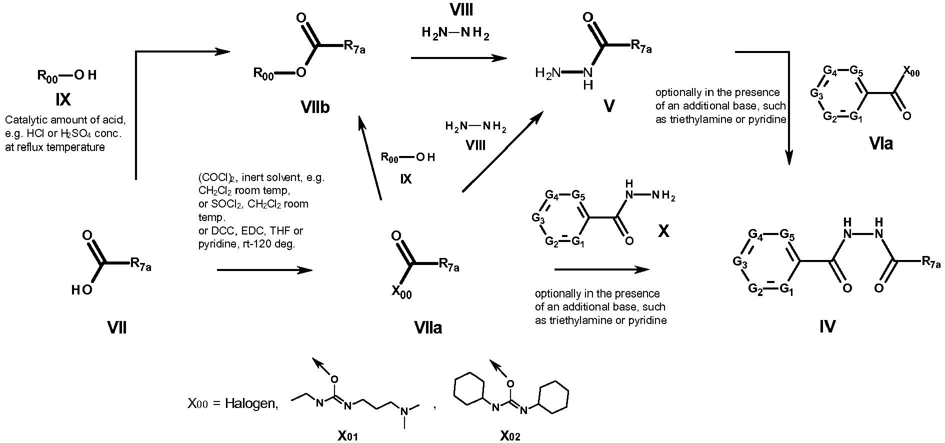 may be prepared by reacting an activated species Via, wherein G₁, G₂, G₃, G₄ and G₅ are as described above, and wherein X₀₀ is halogen, preferably chlorine, with a hydrazide compound of formula V (or a salt thereof), wherein R₇ₐ is as defined above, optionally in the presence of a base, such as triethylamine or pyridine, in an inert solvents such as dichloromethane, tetrahydrofuran, dioxane or toluene, at temperatures between 0 and 50°C.

Compounds of formula Via, wherein G₁, G₂, G₃, G₄ and G₅ are as described above, and wherein X₀₀ is halogen, preferably chlorine, 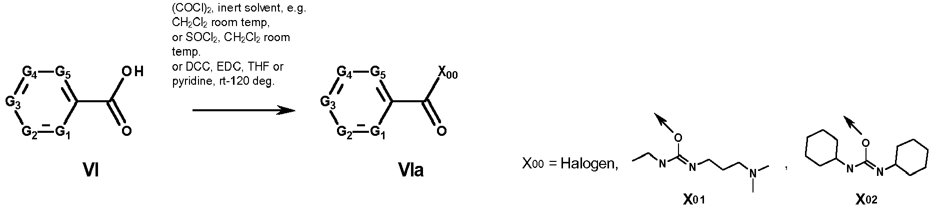 may be prepared by activation of a compound of formula VI, wherein G₁, G₂, G₃, G₄ and G₅ are as described above, by methods known to those skilled in the art and described in, for example, Tetrahedron, 2005, 61 (46), 10827-10852. For example, compounds VIa where X₀₀ is halogen, preferably chlorine, are formed by treatment of VI with, for example, oxallyl chloride (COCl)₂ or thionyl chloride SOCl₂ in the presence of catalytic quantities of N,N-dimethylformamide DMF in inert solvents such as methylene chloride CH₂Cl₂ or tetrahydrofuran THF at temperatures between 20 to 100°C, preferably 25°C. Alternatively, treatment of compounds of formula VI with, for example, 1-ethyl-3-(3-dimethylamino-propyl)carbodiimide EDC or dicyclohexyl carbodiimide DCC will generate an activated species Via, wherein X₀₀ is X₀₁ or X₀₂ respectively, in an inert solvent, such as pyridine or tetrahydrofuran THF, optionally in the presence of a base, such as triethylamine, at temperatures between 50-180°C.

Alternatively, compounds of formula IV, wherein R₇ₐ, G₁, G₂, G₃, G₄ and G₅ are as described above, may also be prepared by reacting an activated species VIIa, wherein R₇ₐ is as defined above, and wherein X₀₀ is halogen, preferably chlorine, with a hydrazide compound of formula X (or a salt thereof), wherein G₁, G₂, G₃, G₄ and G₅ are as described above, optionally in the presence of a base, such as triethylamine or pyridine, in an inert solvents such as dichloromethane, tetrahydrofuran, dioxane or toluene, at temperatures between 0 and 50°C.

Compounds of the formula V, or a salt thereof, wherein R₇ₐ is as defined above, may be prepared by either the direct action of hydrazine VIII (or a salt thereof), possibly in form of a hydrate, preferably hydrazine monohydrate, on an ester derivative VIIb of the compound of formula VII, wherein R₇ₐ is as defined above and wherein R₀₀ is C₁-C₄alkyl, preferably methyl or ethyl, at temperatures between 20 and 150°C, optionally under microwave irradiation. Such a process description may be found, for example, in M.H. Klingele et al, Eur.J. Org. Chem. 2004, 3422-3434. Alternatively, treament of the activated species VIIa with hydrazine VIII (or a salt thereof), possibly in form of a hydrate, preferably hydrazine monohydrate, optionally in the presence of a base, such as triethylamine or pyridine, in an inert solvents such as dichloromethane, tetrahydrofuran, dioxane or toluene, at temperatures between -50°C and 50°C, will also form the compounds of formula V.

Esters of formula Vllb, wherein R₇ₐ is as defined above and wherein R₀₀ is C₁-C₄alkyl, preferably methyl or ethyl, may be prepared by either the direct action of an alcohol R₀₀-OH of formula IX, wherein R₀₀ is C₁-C₄alkyl, on compounds of formula VIIa, wherein R₇ₐ is as defined above, or by means of an esterification reaction of compounds of formula VII with an alcohol R₀₀-OH of formula IX, wherein R₀₀ is C₁-C₄alkyl, in the presence of a catalytic amount of an acid, such as hydrochloric acid HCI or sulfuric acid H₂SO₄, under refluxing conditions. Both of these methods are well known to a person skilled in the art and precedented in the literature.

Compounds of formula VII and Vllb, wherein R₇ₐ is as defined above and wherein R₀₀ is C₁-C₄alkyl, are known compounds or can be prepared by known methods, described in the literature.

Hydrazide compounds of formula X (or a salt thereof), wherein G₁, G₂, G₃, G₄ and G₅ are as described above, may be prepared by treating an activated species Via, wherein G₁, G₂, G₃, G₄ and G₅ are as described above, and wherein X₀₀ is halogen, preferably chlorine, with hydrazine VIII (or a salt thereof), possibly in form of a hydrate, preferably hydrazine monohydrate, optionally in the presence of a base, such as triethylamine or pyridine, in an inert solvents such as dichloromethane, tetrahydrofuran, dioxane or toluene, at temperatures between -50°C and 50°C, in analogy to the transformation VIIa to V discussed above. Alternatively, compounds of formula X may also be prepared by the direct action of hydrazine VIII (or a salt thereof), possibly in form of a hydrate, preferably hydrazine monohydrate, on an ester derivative Vlb of the compound of formula VI, wherein G₁, G₂, G₃, G₄ and G₅ are as defined above and wherein R₀₀ is C₁-C₄alkyl, preferably methyl or ethyl, at temperatures between 20 and 150°C, optionally under microwave irradiation (in analogy to the transformation VIIb to V above).

Ester compounds of formula Vlb, wherein G₁, G₂, G₃, G₄ and G₅ are as defined above and wherein R₀₀ is C₁-C₄alkyl, preferably methyl or ethyl, 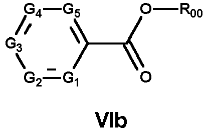 may be prepared from compounds of formula VI, wherein G₁, G₂, G₃, G₄ and G₅ are as described above, by involving an alcohol R₀₀-OH of formula IX, wherein R₀₀ is C₁-C₄alkyl, either directly (in analogy to the transformation VII to VIIb above) or via the activated species Via (in analogy to the transformation VII to VIIa to VIIb above).

Compounds of formula VI and Vlb, wherein G₁, G₂, G₃, G₄ and G₅ are as defined above and wherein R₀₀ is C₁-C₄alkyl, are possibly known compounds or can be prepared by known methods, described in the literature.

Those skilled in the art will realise that the compounds of formula I can be prepared in an alternative manner by switching the order of the previously described reactions. This alternative route is summarized below for the subgroup of compounds of formula I, wherein G₁, G₂, G₃, G₄ and G₅ are as described in formula I and wherein R₇ is a radical selected from the group consisting of formula Q₁ to Q₂, and in which G₆ is represented by the formula U-5, which may be defined as compounds of formula I-U-5. Such compounds of formula I-U-5 may be prepared from compounds of formula Vllb:

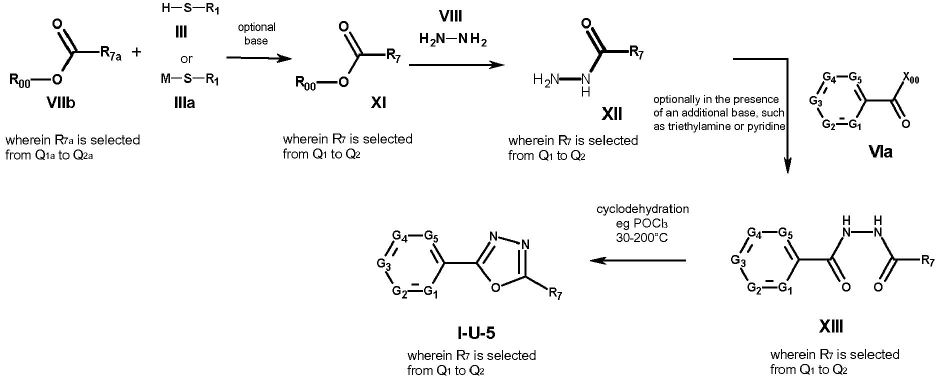

All substituent definitions shown in the scheme above are identical to those already described above.

The subgroup of compounds of formula II, wherein G₁, G₂, G₃, G₄ and G₅ are as described in formula I and wherein R₇ₐ is a radical selected from the group consisting of formula Q₁ₐ to Q₂ₐ, and in which G₆ is represented by the formula U-0, and wherein R₈ is as described in formula I, may be defined as compounds of formula II-U-0.
Such compounds of formula II-U-0, 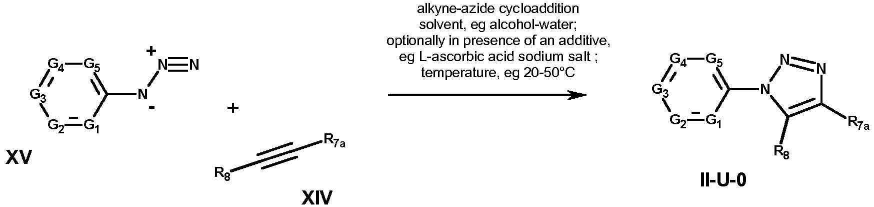 can be prepared by a cycloaddition reaction between alkyne compounds of formula XIV, wherein R₇ₐ and R₈ are as described above, and azide compounds of formula XV, or a tautomer form thereof, wherein G₁, G₂, G₃, G₄ and G₅ are as described above, in solvents such as alcohols (for example methanol, ethanol, propanol, isopropanol, butanol or ter-butanol), ethers (for example tetrahydrofuran or dioxane), acetonitrile and water, or mixtures thereof, at temperatures bewtween 20-120°C, preferably between 20-80°C, optionally in presence of additives, such as L-ascorbic acid sodium salt, optionally in presence of copper salts, such as copper sulfate CuSO₄ or copper acetate Cu(OAc)₂ and optionally in presence of further additives, such as tris(benzyltriazolylmethyl)amine TBTA. Examples of such a transformation may be found in, for example, European Journal of Organic Chemistry, 2014(36), 8167-8181; 2014 or Inorganica Chimica Acta, 374, 253-260; 2011.

Compounds of formula XV, or a tautomer form thereof, wherein G₁, G₂, G₃, G₄ and G₅ are as described above, 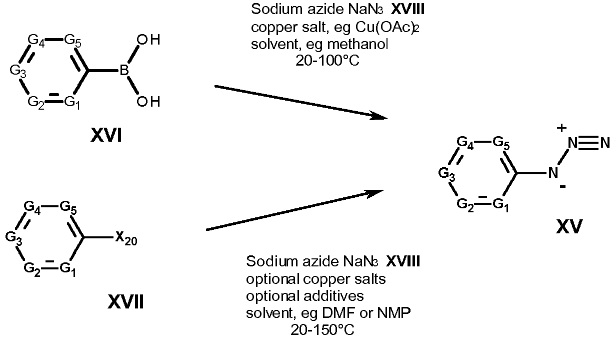 can be prepared by reaction of sodium azide XVIII with a boronic acid compound of formula XVI, wherein G₁, G₂, G₃, G₄ and G₅ are as described above, in presence of a copper derivative, like for example copper(II) acetate, or copper sulfate CuSO₄, optionally in presence of additives, such as L-ascorbic acid sodium salt, under an oxygen-containing atmosphere. The reaction can be run in solvents such as alcohols (for example methanol, ethanol, propanol, isopropanol, butanol or ter-butanol) at temperatures bewtween 20-100°C, preferably between 20-80°C.

Alternatively, compounds of formula XV, or a tautomer form thereof, may be prepared by reaction of sodium azide XVIII with a compound of formula XVII, wherein G₁, G₂, G₃, G₄ and G₅ are as described above, and wherein X₂₀ is a leaving group, such as a halogen, preferably fluorine, chlorine or bromine, or a pseudohalogen such as C₁₋₄haloalkylsulfonate, especially triflate, optionally in presence of additives, such as L-ascorbic acid sodium salt, optionally in presence of copper salts, such as copper sulfate CuSO₄ or copper acetate Cu(OAc)₂, in solvents such as acetonitrile, N,N-dimethylformamide DMF or N-methyl pyrrolidone NMP, at temperatures bewtween 20-150°C, preferably between 20-80°C.

Compounds of formula XIV, wherein R₇ₐ and R₈ are as described above, 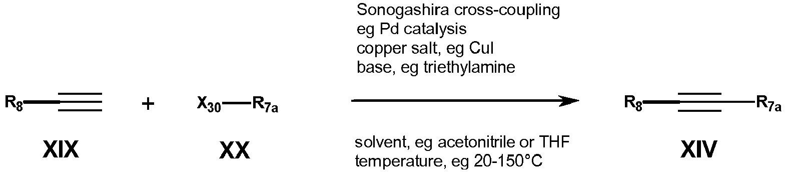 can be prepared by reacting compounds of formula XX, wherein R₇ₐ is as described above, and wherein X₃₀ is a leaving group, such as a halogen, preferably chlorine, bromine or iodine, or a pseudohalogen such as C₁₋₄haloalkylsulfonate, especially triflate, with terminal alkynes of formula XIX, wherein R₈ is as described above. This type of reaction is well known to a person skilled in the art and commonly described as the Sonogashira cross-coupling reaction. In this reaction, the substituted (het)aromatic component of formula XX is reacted with the terminal alkyne of formula XIX in the presence of a copper(I) salt, such as copper(I) iodide Cul, preferably in catalytic amount, and in the presence of a palladium based catalyst, for example bis(triphenyl-phosphine)palladium(II) dichloride or (1,1'bis(diphenylphosphino)ferrocene)dichloropalladium-dichloromethane (1:1 complex), preferably in catalytic amount, and in the presence of a base, such as a tertiary amine, for example triethylamine or Hünig's base (N,N-diisopropylethylamine), preferably in equivalent amount or in excess. The reaction can be performed in the amine as solvent or another compatible solvent can be used as diluant, as for example acetonitrile or an ether, such as dioxane or tetrahydrofuran. The reaction is best performed under inert atmosphere and can take place at temperatures in the range from below 0°C to the boiling point of the reaction mixture, optionally under microwave irradiation.

In the particular situation where R₈ in compounds of formula XIV is hydrogen, 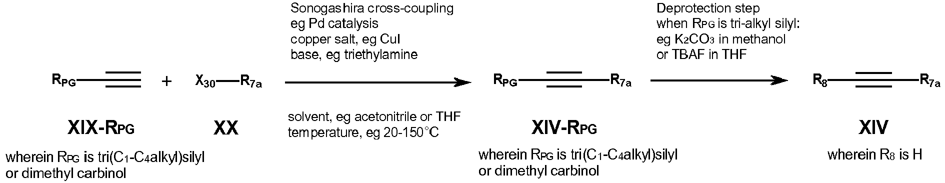 such compounds can be prepared via
i) a Sonogashira reaction (conditions as described above) between compounds of formula XX, wherein R₇ₐ is as described above, and wherein X₃₀ is a leaving group, such as a halogen, preferably chlorine, bromine or iodine, or a pseudohalogen such as C₁₋₄haloalkylsulfonate, especially triflate, with alkynes of formula XIX-R_{PG}, wherein R_{PG} is, for example, tri(C₁-C₄alkyl)silyl or dimethyl carbinol (in that later case, the reactant XIX-R_{PG} is then 2-methylbut-3-yn-2-ol), to form protected alkyne compounds of formula XIV-R_{PG} as intermediates, wherein R₇ₐ and R_{PG} are as described above; followed by
ii) a deprotection step, for example trimethylsilyl cleavage, when R_{PG} is tri(C₁-C₄alkyl)silyl, wherein C₁-C₄alkyl is methyl. Typically, compounds of formula XIV-R_{PG}, wherein R₇ₐ is as described above, and wherein R_{PG} is tri(C₁-C₄alkyl)silyl, are treated with a base, such as potassium carbonate K₂CO₃, or sodium- or potassium hydroxide, or tetrabutylammonium hydroxide, in toluene or in a protic solvent, such as an alcohol, preferably methanol or ethanol, at temperatures between 0°C and 50°C, to form the compounds of formula XIV, wherein R₃ is hydrogen. Alternatively, compounds of formula XIV-R_{PG}, wherein R₇ₐ is as described above, and wherein R_{PG} is tri(C₁-C₄alkyl)silyl, may also be treated with a fluoride source, such as for example, tetrabutyl ammonium fluoride TBAF or potassium fluoride, in solvents such as tetrahydrofuran THF or dioxane, at temperatures between 0°C and 50°C. Compounds of formula XIV-R_{PG}, wherein R₇ₐ is as described above, and wherein R_{PG} is dimethyl carbinol, may be deprotected similarly, by treatment with a base, such as potassium carbonate, or sodium- or potassium hydroxide, or tetrabutylammonium hydroxide, in toluene or in a protic solvent, such as an alcohol, preferably methanol or ethanol, at temperatures between 20°C and 120°C.

Advantageously, compounds of formula II-U-0, 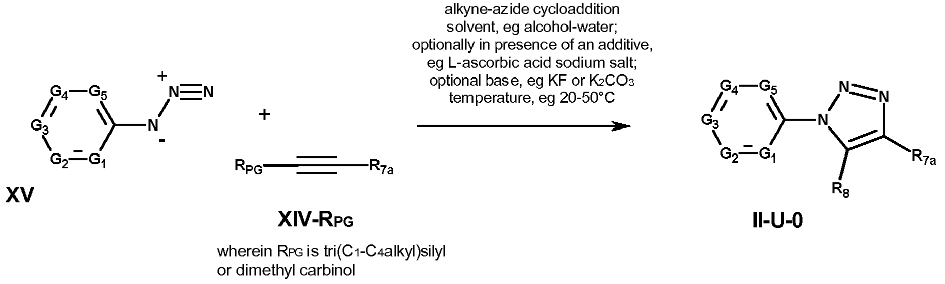 may be prepared by a direct cycloaddition reaction between alkyne compounds of formula XIV-R_{PG}, wherein R₇ₐ is as described above, and wherein R_{PG} is is tri(C₁-C₄alkyl)silyl, and azide compounds of formula XV, or a tautomer form thereof, wherein G₁, G₂, G₃, G₄ and G₅ are as described above, under conditions already described above, in presence of an optional base, such as or potassium fluoride or potassium carbonate K₂CO₃. Examples of such a transformation may be found in, for example, Organometallics, 27(21), 5430-5433; 2008.

Compounds of formula XVI, XVII, XIX, XIX-R_{PG} and XX (respective substituent definitions, see text above) are possibly known compounds or can be prepared by known methods, described in the literature.

Those skilled in the art will realise that the compounds of formula I can be prepared in an alternative manner by switching the order of the previously described reactions. This alternative route is summarized below for the subgroup of compounds of formula I, wherein G₁, G₂, G₃, G₄ and G₅ are as described in formula I and wherein R₇ is a radical selected from the group consisting of formula Q₁ to Q₂, and in which G₆ is represented by the formula U-0, which may be defined as compounds of formula I-U-0. Such compounds of formula I-U-0 may be prepared from compounds of formula XXI:

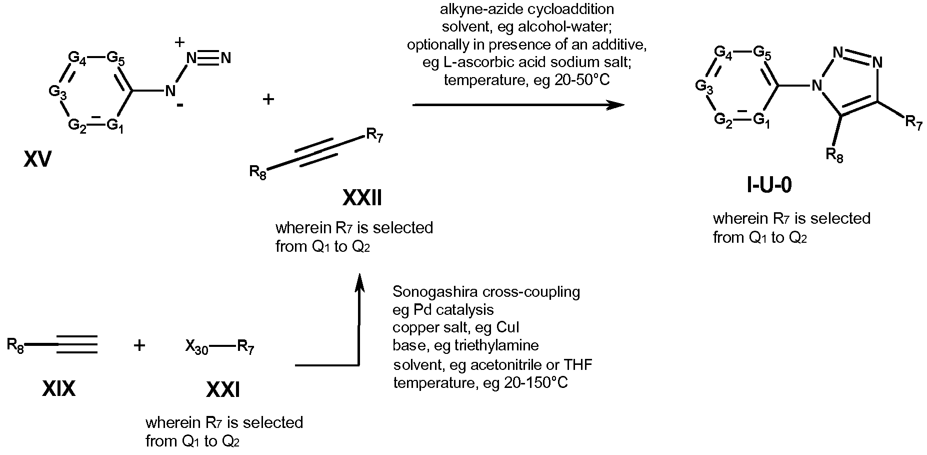

All substituent definitions shown in the scheme above are identical to those already described beforehand.

Alternatively, compounds of formula II-U-0, wherein the substituent definitions are as defined above, 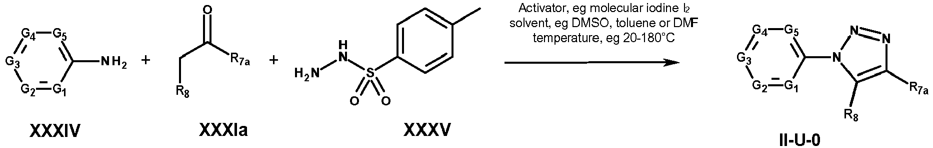 can be prepared by a one pot reaction between compounds of formula XXXIV, wherein G₁, G₂, G₃, G₄ and G₅ are as described above, and compounds of formula XXXIa, wherein R₇ₐ and R₈ are as described above, and *N*-tosyl hydrazine XXXV, in the presence of an activator, such as molecular iodine I₂ (between 0.5 and 3 equivalents, preferably 1.5 equivalent), optionally in the presence of an oxidant additive, such as oxone, potassium persulfate or an oxygen atmosphere, in a solvent such as dimethylsulfoxide DMSO, toluene or N,N-dimethylformamide (preferably DMSO), at temperatures bewtween 20-180°C, preferably at 80-120°C. Examples of such a transformation may be found in, for example, Chem. Eur. J. 20, 17635-17639; 2014.

Similarly, compounds of formula I-U-0, wherein the substituent definitions are as defined above, 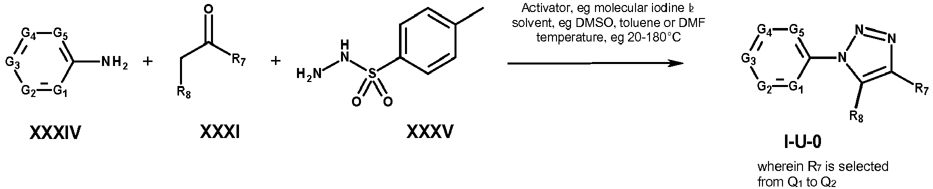 may be prepared by a one pot reaction between compounds of formula XXXIV, wherein G₁, G₂, G₃, G₄ and G₅ are as described above, and compounds of formula XXXI, wherein R₇ and R₈ are as described above, and *N*-tosyl hydrazine XXXV, in the presence of an activator, such as molecular iodine I₂ (between 0.5 and 3 equivalents, preferably 1.5 equivalent), optionally in the presence of an oxidant additive, such as oxone, potassium persulfate or an oxygen atmosphere, in a solvent such as dimethylsulfoxide DMSO, toluene or N,N-dimethylformamide (preferably DMSO), at temperatures bewtween 20-180°C, preferably at 80-120°C.

The subgroup of compounds of formula II, wherein G₁, G₂, G₃, G₄ and G₅ are as described in formula I and wherein R_{7b} is a radical selected from the group consisting of formula Q_{1b} to Q_{2b}, in which A, X and R₁ are as defined in formula I, and wherein Xb is a leaving group like, for example, fluorine, chlorine, bromine or iodine (preferably fluorine, chlorine or bromine), or an aryl- or alkylsulfonate such as trifluoromethanesulfonate, 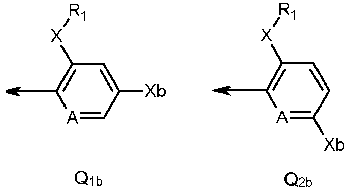 and in which G₆ is represented by the formula U-0, and wherein R₈ is as described in formula I, may be defined as compounds of formula II-U-0-b.
In analogy to above descriptions, such compounds of formula II-U-0-b, 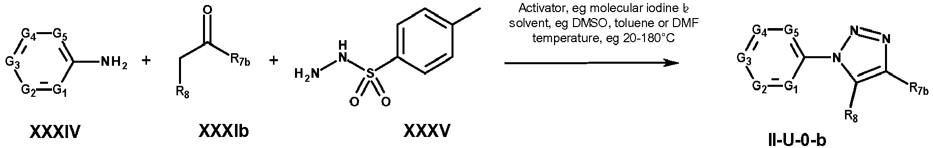 can be prepared by a one pot reaction between compounds of formula XXXIV, wherein G₁, G₂, G₃, G₄ and G₅ are as described above, and compounds of formula XXXIb, wherein R_{7b} and R₈ are as described above, and *N*-tosyl hydrazine XXXV, in the presence of an activator, such as molecular iodine I₂ (between 0.5 and 3 equivalents, preferably 1.5 equivalent), optionally in the presence of an oxidant additive, such as oxone, potassium persulfate or an oxygen atmosphere, in a solvent such as dimethylsulfoxide DMSO, toluene or N,N-dimethylformamide (preferably DMSO), at temperatures bewtween 20-180°C, preferably at 80-120°C.

Compounds of formula XXXIV and XXXV (respective substituent definitions, see text above) are either known compounds or can be prepared by known methods, described in the literature.

The subgroup of compounds of formula I, wherein G₁, G₂, G₃, G₄ and G₅ are as described in formula I and wherein R₇ is a radical selected from the group consisting of formula Q₁ to Q₂, and in which G₆ is represented by the formula U-3, and wherein R₈ is as described in formula I, may be defined as compounds of formula I-U-3.
Such compounds of formula I-U-3, 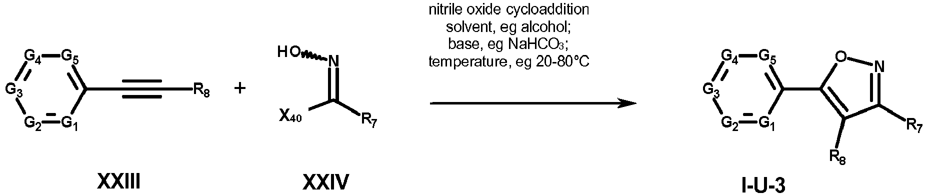 can be prepared by a cycloaddition reaction between alkyne compounds of formula XXIII, wherein R₈, G₁, G₂, G₃, G₄ and G₅ are as described above, and halo oxime compounds of formula XXIV, wherein R₇ is as described above, and wherein X₄₀ is halogen, preferably chlorine or bromine, in presence of a base such as sodium hydrogene carbonate NaHCO₃, potassium hydrogene carbonate, sodium- or potassium carbonate or triethylamine, in solvents such as alcohols (for example methanol, ethanol, propanol, isopropanol, butanol or ter-butanol), ethers (for example tetrahydrofuran or dioxane), ethyl acetate, or mixtures thereof, at temperatures bewtween 20-100°C, preferably between 20-80°C. This transformation is known to involve *in* situ formation of a nitrile oxide of formula XXV, wherein R₇ is as described above:

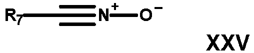

Examples of such a transformation may be found in, for example, Bioorganic & Medicinal Chemistry Letters, 14(16), 4307-4311; 2004.

Compounds of formula XXIV, wherein R₇ is as described above, and wherein X₄₀ is halogen, preferably chlorine or bromine, 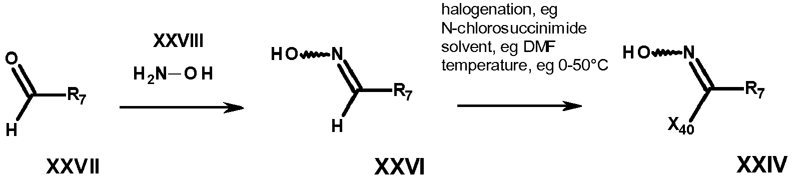 can be prepared by reacting oxime compounds of formula XXVI, wherein R₇ is as described above, with a halogenation reagent such as N-chlorosuccinimide (NCS) or N-bromosuccinimide (NBS). Such halogenation reactions are carried out in an inert solvent, such as chloroform, carbon tetrachloride, 1,2-dichloroethane, acetonitrile or N,N-dimethylformamide, at temperatures between 0-100°C, preferably 0-50°C.
Compounds of formula XXVI, wherein R₇ is as described above, can themselves be prepared by reacting compounds of formula XXVII, wherein R₇ is as described above, with hydroxylamine XXVIII, or a salt thereof (such as a hydrohalide salt, preferably a hydrochloride or a hydrobromide salt, or any other equivalent salt), in presence of a base such as sodium acetate, sodium- or potassium carbonate, triethylamine or piperidine, in solvents such as acetonitrile, N,N-dimethylformamide, alcohols (preferably methanol or ethanol) or water, or mixtures thereof, at temperatures between 20-120°C, preferably 20-80°C.

Compounds of formula XXIII, wherein R₈, G₁, G₂, G₃, G₄ and G₅ are as described above, 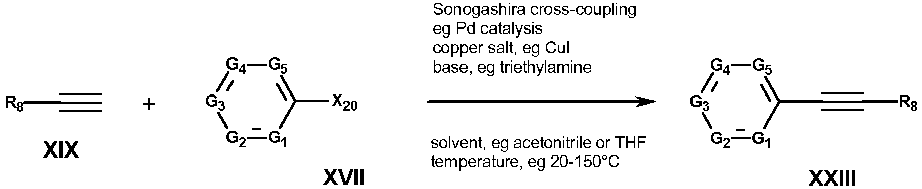 may be prepared by reacting previously described compounds of formula XIX, wherein R₈ is as described above, with previously described compounds of formula XVII, wherein G₁, G₂, G₃, G₄ and G₅ are as described above, and wherein X₂₀ is a leaving group, such as a halogen, preferably fluorine, chlorine or bromine, or a pseudohalogen such as C₁₋₄haloalkylsulfonate, especially triflate, under the previously described conditions of the Sonogashira cross-coupling reaction.
In the particular situation where R₈ in compounds of formula XXIII is hydrogen, 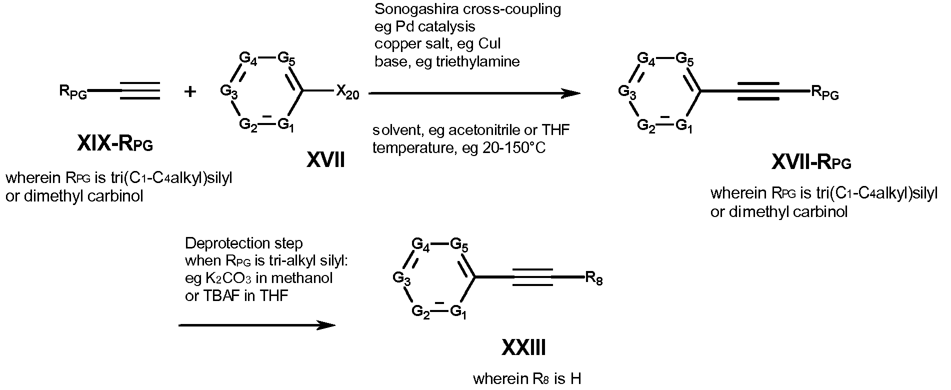 such compounds can be prepared via
i) a Sonogashira reaction (conditions as described above) between compounds of formula XVII, wherein G₁, G₂, G₃, G₄ and G₅ are as described above, and wherein X₂₀ is a leaving group, such as a halogen, preferably chlorine, bromine or iodine, or a pseudohalogen such as C₁₋₄haloalkylsulfonate, especially triflate, with alkynes of formula XIX-R_{PG}, wherein R_{PG} is, for example, tri(C₁-C₄alkyl)silyl or dimethyl carbinol (in that later case, the reactant XIX-R_{PG} is then 2-methylbut-3-yn-2-ol), to form protected alkyne compounds of formula XVII-R_{PG} as intermediates, wherein G₁, G₂, G₃, G₄, G₅ and R_{PG} are as described above; followed by
ii) a deprotection step, for example trimethylsilyl cleavage, when R_{PG} is tri(C₁-C₄alkyl)silyl, wherein C₁-C₄alkyl is methyl. Typically, compounds of formula XVII-R_{PG}, wherein G₁, G₂, G₃, G₄ and G₅ are as described above, and wherein R_{PG} is tri(C₁-C₄alkyl)silyl, are treated with a base, such as potassium carbonate K₂CO₃, or sodium- or potassium hydroxide, or tetrabutylammonium hydroxide, in toluene or in a protic solvent, such as an alcohol, preferably methanol or ethanol, at temperatures between 0°C and 50°C, to form the compounds of formula XXIII, wherein R₈ is hydrogen. Alternatively, compounds of formula XVII-R_{PG}, wherein G₁, G₂, G₃, G₄ and G₅ are as described above, and wherein R_{PG} is tri(C₁-C₄alkyl)silyl, may also be treated with a fluoride source, such as for example, tetrabutyl ammonium fluoride TBAF or potassium fluoride, in solvents such as tetrahydrofuran THF or dioxane, at temperatures between 0°C and 50°C.
   Compounds of formula XVII-R_{PG}, wherein G₁, G₂, G₃, G₄ and G₅ are as described above, and wherein R_{PG} is dimethyl carbinol, may be deprotected similarly, by treatment with a base, such as potassium carbonate, or sodium- or potassium hydroxide, or tetrabutylammonium hydroxide, in toluene or in a protic solvent, such as an alcohol, preferably methanol or ethanol, at temperatures between 20°C and 120°C.

The subgroup of compounds of formula II, wherein G₁, G₂, G₃, G₄ and G₅ are as described in formula I and wherein R₇ₐ is a radical selected from the group consisting of formula Q₁ₐ to Q₂ₐ, and in which G₆ is represented by the formula U-2, and wherein R₈ and R₉ are as described in formula I, may be defined as compounds of formula II-U-2-a.
Such compounds of formula II-U-2-a, 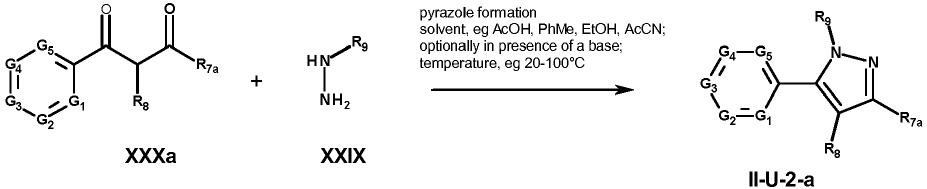 can be prepared by a reaction between a hydrazine compound of formula XXIX, or a salt thereof (such as a hydrohalide salt, preferably a hydrochloride or a hydrobromide salt, or any other equivalent salt), wherein R₉ is as described above, with a diketone compound of formula XXXa, or a tautomer form thereof, wherein G₁, G₂, G₃, G₄, G₅, R₇ₐ and R₈ are as described above, optionally in presence of a base such as sodium acetate, sodium- or potassium carbonate or triethylamine, in solvents such as acetic acid AcOH, toluene PhMe, tetrahydrofurane, dioxane, methanol, ethanol EtOH or acetonitrile, at temperatures between 20-120°C, preferably 20-100°C. Examples of such a transformation may be found in, for example, Catalysis Communications, 29, 194-197, 2012.

Compounds of formula XXXa, or a tautomer form thereof, wherein G₁, G₂, G₃, G₄, G₅, R₇ₐ and R₈ are as described above, 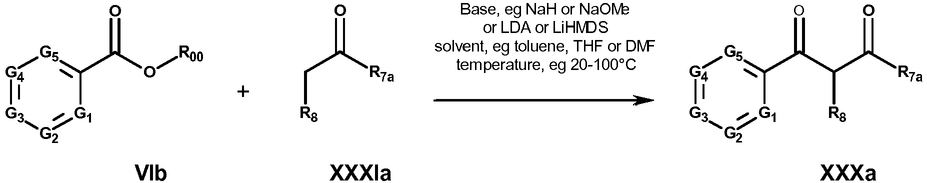 may be prepared by reacting a previously described compound of formula Vlb, wherein G₁, G₂, G₃, G₄ and G₅ are as defined above and wherein R₀₀ is C₁-C₄alkyl, with compounds of formula XXXIa, wherein R₇ₐ and R₈ are as described above, in presence of a base such as sodium hydride NaH, lithium diisopropylamide LDA, lithium hexamethyldisilazide LiHMDS, sodium methoxide NaOMe or sodium ethoxide, in solvents such as toluene, tetrahydrofuran THF or N,N-dimethylformamide DMF (or mixtures thereof), at temperatures between -78 to 120°C, preferably 0-100°C. Examples of such a transformation may be found in, for example, Synthetic Communications, 37(19), 3393-3402, 2007 or Polyhedron 9(23), 2839-2845, 1990.

Compounds of formula XXXa, or a tautomer form thereof, wherein G₁, G₂, G₃, G₄, G₅, R₇ₐ and R₈ are as described above, 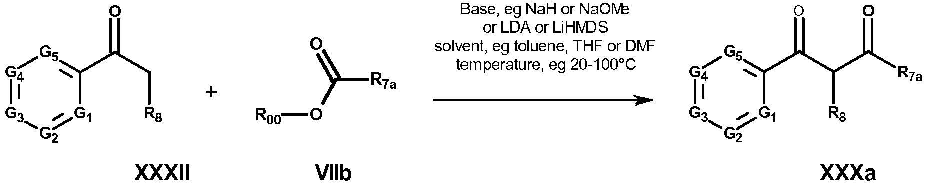 may be also prepared by inversing the polarity of the two reacting centers, ie by reacting a previously described compound of formula Vllb, wherein R₇ₐ is as described above, and in which R₀₀ is C₁-C₄alkyl, with compounds of formula XXXII, wherein R₈, G₁, G₂, G₃, G₄ and G₅ are as described above, in presence of a base such as sodium hydride NaH, lithium diisopropylamide LDA, lithium hexamethyldisilazide LiHMDS, sodium methoxide NaOMe or sodium ethoxide, in solvents such as toluene, tetrahydrofuran THF or N,N-dimethylformamide DMF (or mixtures thereof), at temperatures between -78 to 120°C, preferably 0-100°C.

The subgroup of compounds of formula I, wherein G₁, G₂, G₃, G₄ and G₅ are as described in formula I and wherein R₇ is a radical selected from the group consisting of formula Q₁ to Q₂, and in which G₆ is represented by the formula U-2, and wherein R₈ and R₉ are as described in formula I, may be defined as compounds of formula I-U-2.
Such compounds of formula I-U-2, 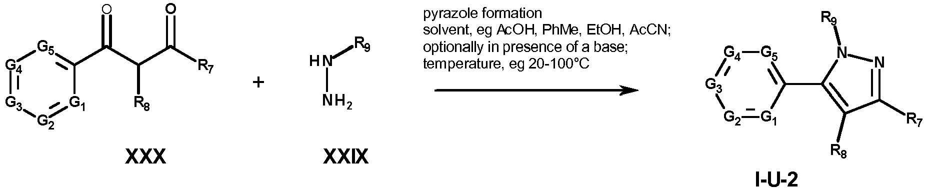 can be prepared by a reaction between a hydrazine compound of formula XXIX, or a salt thereof (such as a hydrohalide salt, preferably a hydrochloride or a hydrobromide salt, or any other equivalent salt), wherein R₉ is as described above, with a diketone compound of formula XXX, or a tautomer form thereof, wherein G₁, G₂, G₃, G₄, G₅, R₇ and R₈ are as described above, optionally in presence of a base such as sodium acetate, sodium- or potassium carbonate or triethylamine, in solvents such as acetic acid AcOH, toluene PhMe, tetrahydrofuran, dioxane, methanol, ethanol EtOH or acetonitrile, at temperatures between 20-120°C, preferably 20-100°C. Examples of such a transformation may be found in, for example, Catalysis Communications, 29, 194-197; 2012.

Compounds of formula XXX, or a tautomer form thereof, wherein G₁, G₂, G₃, G₄, G₅, R₇ and R₈ are as described above, 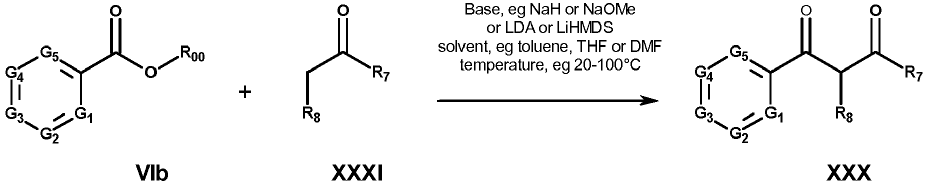 may be prepared by reacting a previously described compound of formula VIb, wherein G₁, G₂, G₃, G₄ and G₅ are as defined above and wherein R₀₀ is C₁-C₄alkyl, with compounds of formula XXXI, wherein R₇ and R₈ are as described above, in presence of a base such as sodium hydride NaH, lithium diisopropylamide LDA, lithium hexamethyldisilazide LiHMDS, sodium methoxide NaOMe or sodium ethoxide, in solvents such as toluene, tetrahydrofuran THF or N,N-dimethylformamide DMF (or mixtures thereof), at temperatures between -78 to 120°C, preferably 0-100°C. Examples of such a transformation may be found in, for example, Synthetic Communications, 37(19), 3393-3402; 2007 or Polyhedron 9(23), 2839-2845, 1990.

Compounds of formula XXX, or a tautomer form thereof, wherein G₁, G₂, G₃, G₄, G₅, R₇ and R₈ are as described above, 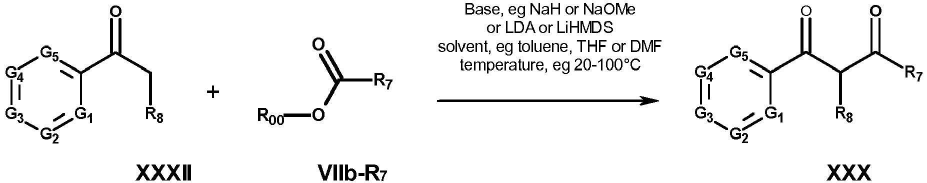 may be also prepared by inversing the polarity of the two reacting centers, ie by reacting compounds of formula VIIb-R₇, wherein R₇ is as described above, and in which R₀₀ is C₁-C₄alkyl, with compounds of formula XXXII, wherein R₈, G₁, G₂, G₃, G₄ and G₅ are as described above, in presence of a base such as sodium hydride NaH, lithium diisopropylamide LDA, lithium hexamethyldisilazide LiHMDS, sodium methoxide NaOMe or sodium ethoxide, in solvents such as toluene, tetrahydrofuran THF or N,N-dimethylformamide DMF (or mixtures thereof), at temperatures between -78 to 120°C, preferably 0-100°C.

The subgroup of compounds of formula II, wherein G₁, G₂, G₃, G₄ and G₅ are as described in formula I and wherein R_{7b} is a radical selected from the group consisting of formula Q_{1b} to Q_{2b}, in which A, X and R₁ are as defined in formula I, and wherein Xb is a leaving group like, for example, fluorine, chlorine, bromine or iodine (preferably chlorine or bromine, but preferably also fluorine when R_{7b} is Q_{2b}), or an aryl- or alkylsulfonate such as trifluoromethanesulfonate, 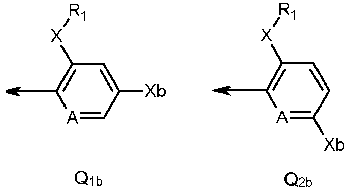 and in which G₆ is represented by the formula U-2, and wherein R₈ and R₉ are as described in formula I, may be defined as compounds of formula II-U-2-b.
Such compounds of formula II-U-2-b, 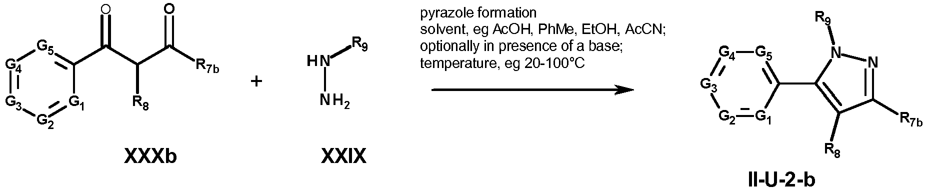 can be prepared by a reaction between a hydrazine compound of formula XXIX, or a salt thereof (such as a hydrohalide salt, preferably a hydrochloride or a hydrobromide salt, or any other equivalent salt), wherein R₉ is as described above, with a diketone compound of formula XXXb, or a tautomer form thereof, wherein G₁, G₂, G₃, G₄, G₅, R_{7b} and R₈ are as described above, optionally in presence of a base such as sodium acetate, sodium- or potassium carbonate or triethylamine, in solvents such as acetic acid AcOH, toluene PhMe, tetrahydrofuran, dioxane, methanol, ethanol EtOH or acetonitrile, at temperatures between 20-120°C, preferably 20-100°C. Examples of such a transformation may be found in, for example, Catalysis Communications, 29, 194-197, 2012.

Compounds of formula XXXb, or a tautomer form thereof, wherein G₁, G₂, G₃, G₄, G₅, R_{7b} and R₈ are as described above, 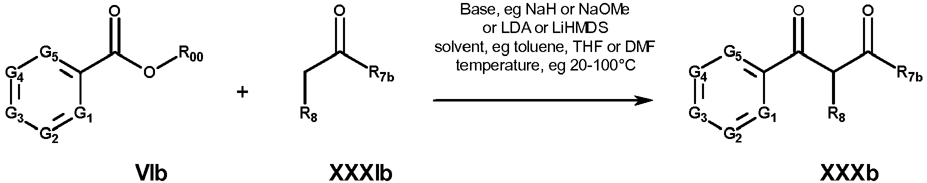 may be prepared by reacting a previously described compound of formula VIb, wherein G₁, G₂, G₃, G₄ and G₅ are as defined above and wherein R₀₀ is C₁-C₄alkyl, with compounds of formula XXXIb, wherein R_{7b} and R₈ are as described above, in presence of a base such as sodium hydride NaH, lithium diisopropylamide LDA, lithium hexamethyldisilazide LiHMDS, sodium methoxide NaOMe or sodium ethoxide, in solvents such as toluene, tetrahydrofuran THF or N,N-dimethylformamide DMF (or mixtures thereof), at temperatures between -78 to 120°C, preferably 0-100°C. Examples of such a transformation may be found in, for example, Synthetic Communications, 37(19), 3393-3402, 2007 or Polyhedron 9(23), 2839-2845, 1990.

Compounds of formula XXXb, or a tautomer form thereof, wherein G₁, G₂, G₃, G₄, G₅, R_{7b} and R₈ are as described above, 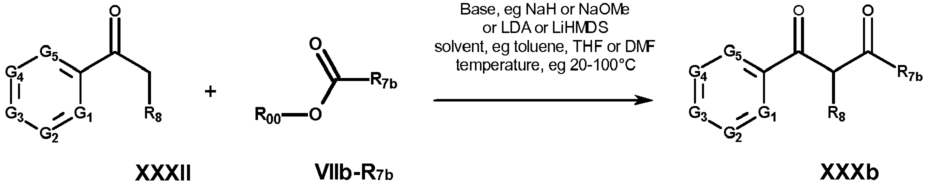 may be also prepared by inversing the polarity of the two reacting centers, ie by reacting compounds of formula VIIb-R_{7b}, wherein R_{7b} is as described above, and in which R₀₀ is C₁-C₄alkyl, with compounds of formula XXXII, wherein R₈, G₁, G₂, G₃, G₄ and G₅ are as described above, in presence of a base such as sodium hydride NaH, lithium diisopropylamide LDA, lithium hexamethyldisilazide LiHMDS, sodium methoxide NaOMe or sodium ethoxide, in solvents such as toluene, tetrahydrofuran THF or N,N-dimethylformamide DMF (or mixtures thereof), at temperatures between -78 to 120°C, preferably 0-100°C.

The compounds of formula (XXXb-i), a particular subgroup of the compounds of formula XXXb, 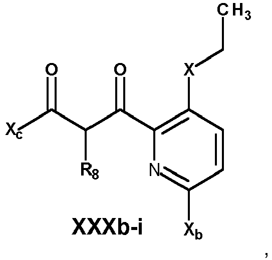 wherein
X and R₈ are as defined under formula I above; preferably R₈ is hydrogen or methyl, even more preferably R₈ is hydrogen;
X_{b} is halogen, preferably fluorine or chlorine;
X_{c} is the radical 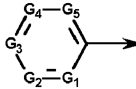 which is preferably selected from the group consisting of formula G7, G8 and G9: 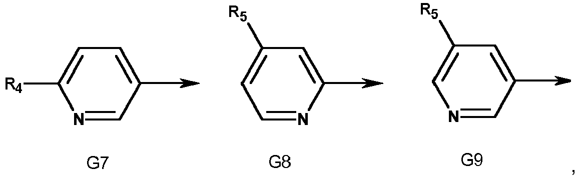
wherein R₄ and R₈ are independently selected from C₁-C₄haloalkyl, preferably trifluoromethyl;
are novel.. The preferences and preferred embodiments of the substituents of the compounds of formula I are also valid for the compounds of formula (XXXb-i).

Compounds of the formula II-U-2-b, wherein R_{7b} is the radical of formula Q_{1b}, and wherein all other substituent definitions are as described above, are equivalent to compounds of the formula XXXIII (see scheme 1), wherein G₆ is represented by the formula U-2, and wherein all other substituent definitions are as described above.
Hence, compounds of the formula II-U-2-b, wherein R_{7b} is the radical of formula Q_{1b}, can be converted to compounds of the formula I-Q₁, wherein G₆ is represented by the formula U-2, according to descriptions and illustrations found in schemes 1 and 2 (see text, descriptions and preparation methods associated to schemes 1 and 2 above).
Similarly, compounds of the formula II-U-2-b, wherein R_{7b} is the radical of formula Q_{2b}, and wherein all other substituent definitions are as described above, are equivalent to compounds of the formula XXXIII-p (see scheme 4), wherein G₆ is represented by the formula U-2, and wherein all other substituent definitions are as described above.
Hence, compounds of the formula II-U-2-b, wherein R_{7b} is the radical of formula Q_{2b}, can be converted to compounds of the formula I-Q₂, wherein G₆ is represented by the formula U-2, according to descriptions and illustrations found in scheme 4 (see text, descriptions and preparation methods associated to schemes 3 and 4 above).

Compounds of formula XXXII and XXIX (respective substituent definitions, see text above) are possibly known compounds or can be prepared by known methods, described in the literature.

Compounds of formula VIIb-R₇ and VIIb-R_{7b} (respective substituent definitions, see text above) can be prepared by analogous methods to those described above for the preparation of compounds of the formula VIIb.

Compounds of the formula XXXIa, XXXI and XXXIb (respective substituent definitions, see text above) may be grouped under a common formula XXXI-G_{R7}, wherein R₈ is as described above and wherein G_{R7} is either R₇ₐ, R₇ or R_{7b}, wherein R₇ₐ is a radical selected from the group consisting of formula Q₁ₐ to Q₂ₐ, R₇ is a radical selected from the group consisting of formula Q₁ and Q₂, and R_{7b} is a radical selected from the group consisting of formula Q_{1b} to Q_{2b}, in which Q₁ₐ, Q₂ₐ, Q₁, Q₂, Q_{1b} and Q_{2b}, are as defined above.
Such compounds of the formula XXXI-G_{R7}, 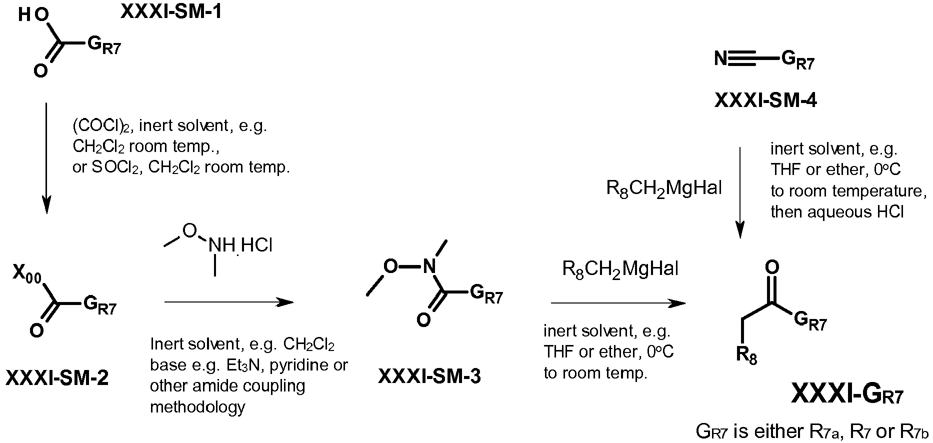 may be prepared from commonly available acid or nitrile starting materials of the formula XXXI-SM-1 and XXXI-SM-4, wherein G_{R7} is as defined above. Hence, compounds of the formula XXXI-G_{R7} may be prepared from nitrile compounds of the formula XXXI-SM-4 by reaction with a Grignard reagent of formula R₈CH₂MgHal, wherein Hal is a halogen, preferably chlorine or bromine, followed by acidic hydrolysis, as described, for example, in C. Ferri, "Reaktionen der Organischen Synthese", Georg Thieme Verlag, Stuttgart, 1978, page 223ff. This process is usually conducted at temperatures between -20°C and room temperature in inert solvents, such as ethers like diethyl ether, methyl t-butyl ether, tetrahydrofuran THF or dioxane. Alternatively, compounds of the formula XXXI-G_{R7} may be prepared from Weinreb amides of formula XXXI-SM-3 which are treated with a Grignard reagent of formula R₈CH₂MgHal described above, according to the method of Weinreb (Tetrahedron Letters 1981, 22, 3815-3818). An acyl halide of formula XXXI-SM-2, wherein X₀₀ is halogen, preferably chlorine (easily prepared from compounds of formula XXXI-SM-1 by methods known to those skilled in the art; see transformation VII to VIIa described above) is converted to a Weinreb amide of formula XXXI-SM-3 upon reaction with *N*,O-dimethylhydroxylamine by methods known to those skilled in the art.

Compounds of formula XXXI-SM-1 and XXXI-SM-4 (respective substituent definitions, see text above) are possibly known compounds or can be prepared by known methods, described in the literature.

The reactants can be reacted in the presence of a base. Examples of suitable bases are alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide and 1,8-diazabicyclo[5.4.0]undec-7-ene (DBU).

The reactants can be reacted with each other as such, i.e. without adding a solvent or diluent. In most cases, however, it is advantageous to add an inert solvent or diluent or a mixture of these. If the reaction is carried out in the presence of a base, bases which are employed in excess, such as triethylamine, pyridine, N-methylmorpholine or N,N-diethylaniline, may also act as solvents or diluents.

The reaction is advantageously carried out in a temperature range from approximately -80°C to approximately +140°C, preferably from approximately -30°C to approximately +100°C, in many cases in the range between ambient temperature and approximately +80°C.

A compound of formula I can be converted in a manner known per se into another compound of formula I by replacing one or more substituents of the starting compound of formula I in the customary manner by (an)other substituent(s).

Depending on the choice of the reaction conditions and starting materials which are suitable in each case, it is possible, for example, in one reaction step only to replace one substituent by another substituent, or a plurality of substituents can be replaced by other substituents in the same reaction step.

Salts of compounds of formula I can be prepared in a manner known per se. Thus, for example, acid addition salts of compounds of formula I are obtained by treatment with a suitable acid or a suitable ion exchanger reagent and salts with bases are obtained by treatment with a suitable base or with a suitable ion exchanger reagent.

Salts of compounds of formula I can be converted in the customary manner into the free compounds I, acid addition salts, for example, by treatment with a suitable basic compound or with a suitable ion exchanger reagent and salts with bases, for example, by treatment with a suitable acid or with a suitable ion exchanger reagent.

Salts of compounds of formula I can be converted in a manner known per se into other salts of compounds of formula I, acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds of formula I, which have salt-forming properties can be obtained in free form or in the form of salts.

The compounds of formula I and, where appropriate, the tautomers thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule; this document relates to the pure isomers and also to all isomer mixtures which are possible and is to be understood in each case in this sense hereinabove and hereinbelow, even when stereochemical details are not mentioned specifically in each case.

Diastereomer mixtures or racemate mixtures of compounds of formula I, in free form or in salt form, which can be obtained depending on which starting materials and procedures have been chosen can be separated in a known manner into the pure diasteromers or racemates on the basis of the physicochemical differences of the components, for example by fractional crystallization, distillation and/or chromatography.

Enantiomer mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl celulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained not only by separating suitable isomer mixtures, but also by generally known methods of diastereoselective or enantioselective synthesis, for example by carrying out the process with starting materials of a suitable stereochemistry.
N-oxides can be prepared by reacting a compound of the formula I with a suitable oxidizing agent, for example the H₂O₂/urea adduct in the presence of an acid anhydride, e.g. trifluoroacetic anhydride. Such oxidations are known from the literature, for example from J. Med. Chem., 32 (12), 2561-73, 1989 or WO 00/15615.
It is advantageous to isolate or synthesize in each case the biologically more effective isomer, for example enantiomer or diastereomer, or isomer mixture, for example enantiomer mixture or diastereomer mixture, if the individual components have a different biological activity.
The compounds of formula I and, where appropriate, the tautomers thereof, in each case in free form or in salt form, can, if appropriate, also be obtained in the form of hydrates and/or include other solvents, for example those which may have been used for the crystallization of compounds which are present in solid form.

The compounds according to the following Tables 1 to 27 below can be prepared according to the methods described above.
Table 1: This table discloses the 12 compounds 1.001 to 1.012 of the formula I-1a-U0: 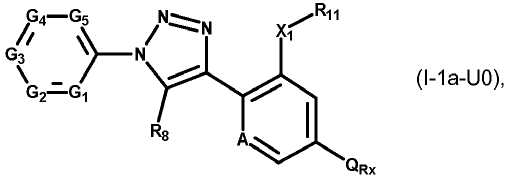 wherein X₁ is S, R₃ is hydrogen, and Q_{Rx}, A, R₁₁, G₁, G₂, G₃, G₄ and G₅ are as defined below:

**Table 1:**

| Comp. No | Q_{Rx} | A | R₁₁ | G₁ | G₂ | G₃ | G₄ | G₅ |
|---|---|---|---|---|---|---|---|---|
| 1.001 | H | N | CH₂CH₃ | CH | CH | C(CF₃) | CH | CH |
| 1.002 | CF₃ | N | CH₂CH₃ | CH | CH | C(CF₃) | CH | CH |
| 1.003 | H | N | CH₂CH₃ | CH | CH | CH | C(CF₃) | CH |
| 1.004 | CF₃ | N | - CH₂CH₃ | CH | CH | CH | C(CF₃) | CH |
| 1.005 | H | N | CH₂CH₃ | N | CH | CH | C(CF₃) | CH |
| 1.006 | CF₃ | N | - CH₂CH₃ | N | CH | CH | C(CF₃) | CH |
| 1.007 | H | N | CH₂CH₃ | CH | N | C(CF₃) | CH | CH |
| 1.008 | CF₃ | N | - CH₂CH₃ | CH | N | C(CF₃) | CH | CH |
| 1.009 | Br | N | CH₂CH₃ | CH | CH | CH | C(CF₃) | CH |
| 1.010 | 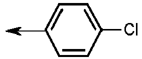 | N | CH₂CH₃ | CH | CH | CH | C(CF₃) | CH |
| 1.011 | H | N | CH₂CH₃ | CH | N | CH | C(CF₃) | CH |
| 1.012 | CF₃ | N | CH₂CH₃ | CH | N | CH | C(CF₃) | CH |

and the N-oxides of the compounds of Table 1.
Table 2: This table discloses the 12 compounds 2.001 to 2.012 of the formula I-1a-U0, wherein X₁ is SO, R₈ is hydrogen, and Q_{Rx}, A, R₁₁, G₁, G₂, G₃, G₄ and G₅ are as defined in Table 1.
Table 3: This table discloses the 12 compounds 3.001 to 3.012 of the formula I-1a-U0, wherein X₁ is SO₂, R₈ is hydrogen, and Q_{Rx}, A, R₁₁, G₁, G₂, G₃, G₄ and G₅ are as defined in Table 1.
Table 4: This table discloses the 8 compounds 4.001 to 4.008 of the formula I-2a-U3: 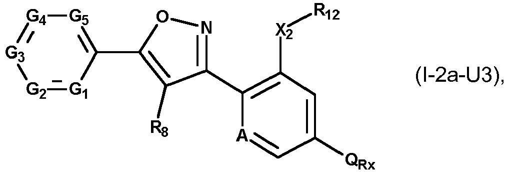 wherein X₂ is S, R₈ is hydrogen, and Q_{Rx}, A, R₁₂, G₁, G₂, G₃, G₄ and G₅ are as defined below:

**Table 4:**

| Comp. No | Q_{Rx} | A | R₁₂ | G₁ | G₂ | G₃ | G₄ | G₅ |
|---|---|---|---|---|---|---|---|---|
| 4.001 | H | N | -CH₂CH₃ | CH | CH | C(CF₃) | CH | CH |
| 4.002 | CF₃ | N | -CH₂CH₃ | CH | CH | C(CF₃) | CH | CH |
| 4.003 | H | N | -CH₂CH₃ | CH | CH | CH | C(CF₃) | CH |
| 4.004 | CF₃ | N | -CH₂CH₃ | CH | CH | CH | C(CF₃) | CH |
| 4.005 | H | N | -CH₂CH₃ | N | CH | CH | C(CF₃) | CH |
| 4.006 | CF₃ | N | -CH₂CH₃ | N | CH | CH | C(CF₃) | CH |
| 4.007 | H | N | -CH₂CH₃ | CH | N | C(CF₃) | CH | CH |
| 4.008 | CF₃ | N | -CH₂CH₃ | CH | N | C(CF₃) | CH | CH |

and the N-oxides of the compounds of Table 4.
Table 5: This table discloses the 8 compounds 5.001 to 5.008 of the formula I-2a-U3, wherein X₂ is SO, R₈ is hydrogen, and Q_{Rx}, A, R₁₂, G₁, G₂, G₃, G₄ and G₅ are as defined in Table 4.
Table 6: This table discloses the 8 compounds 6.001 to 6.008 of the formula I-2a-U3, wherein X₂ is SO₂, R₈ is hydrogen, and Q_{Rx}, A, R₁₂, G₁, G₂, G₃, G₄ and G₅ are as defined in Table 4.
Table 7: This table discloses the 9 compounds 7.001 to 7.009 of the formula I-3a-U5: 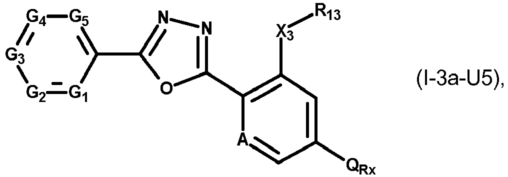 wherein X₃ is S, and Q_{Rx}, A, R₁₃, G₁, G₂, G₃, G₄ and G₅ are as defined below:

**Table 7:**

| Comp. No | Q_{Rx} | A | R₁₃ | G₁ | G₂ | G₃ | G₄ | G₅ |
|---|---|---|---|---|---|---|---|---|
| 7.001 | H | N | -CH₂CH₃ | CH | CH | C(CF₃) | CH | CH |
| 7.002 | CF₃ | N | -CH₂CH₃ | CH | CH | C(CF₃) | CH | CH |
| 7.003 | H | N | -CH₂CH₃ | CH | CH | CH | C(CF₃) | CH |
| 7.004 | CF₃ | N | -CH₂CH₃ | CH | CH | CH | C(CF₃) | CH |
| 7.005 | H | N | -CH₂CH₃ | N | CH | CH | C(CF₃) | CH |
| 7.006 | CF₃ | N | -CH₂CH₃ | N | CH | CH | C(CF₃) | CH |
| 7.007 | H | N | -CH₂CH₃ | CH | N | C(CF₃) | CH | CH |
| 7.008 | CF₃ | N | -CH₂CH₃ | CH | N | C(CF₃) | CH | CH |
| 7.009 | CF₃ | CH | -CH₂CH₃ | CH | N | C(CF₃) | CH | CH |

and the N-oxides of the compounds of Table 7.
Table 8: This table discloses the 9 compounds 8.001 to 8.009 of the formula I-3a-U5, wherein X₃ is SO, and Q_{Rx}, A, R₁₃, G₁, G₂, G₃, G₄ and G₅ are as defined in Table 7.
Table 9: This table discloses the 9 compounds 9.001 to 9.009 of the formula I-3a-U5, wherein X₃ is SO₂, and Q_{Rx}, A, R₁₃, G₁, G₂, G₃, G₄ and G₅ are as defined in Table 7.
Table 10: This table discloses the 9 compounds 10.001 to 10.009 of the formula I-4a-U2: 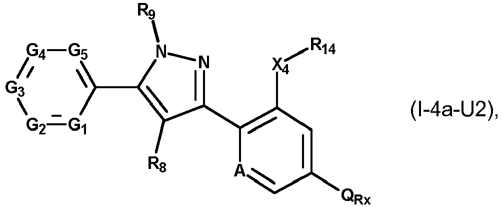 wherein X₄ is S, R₈ is hydrogen, R₉ is methyl, and Q_{Rx}, A, R₁₄, G₁, G₂, G₃, G₄ and G₅ are as defined below:

**Table 10:**

| Comp. No | Q_{Rx} | A | R₁₄ | G₁ | G₂ | G₃ | G₄ | G₅ |
|---|---|---|---|---|---|---|---|---|
| 10.001 | H | N | -CH₂CH₃ | CH | CH | C(CF₃) | CH | CH |
| 10.002 | CF₃ | N | -CH₂CH₃ | CH | CH | C(CF₃) | CH | CH |
| 10.003 | H | N | -CH₂CH₃ | CH | CH | CH | C(CF₃) | CH |
| 10.004 | CF₃ | N | -CH₂CH₃ | CH | CH | CH | C(CF₃) | CH |
| 10.005 | H | N | -CH₂CH₃ | N | CH | CH | C(CF₃) | CH |
| 10.006 | CF₃ | N | -CH₂CH₃ | N | CH | CH | C(CF₃) | CH |
| 10.007 | H | N | -CH₂CH₃ | CH | N | C(CF₃) | CH | CH |
| 10.008 | CF₃ | N | -CH₂CH₃ | CH | N | C(CF₃) | CH | CH |
| 10.009 | H | N | -CH₂CH₃ | CH | N | CH | C(CF₃) | CH |

and the N-oxides of the compounds of Table 10.
Table 11: This table discloses the 9 compounds 11.001 to 11.009 of the formula I-4a-U2, wherein X₄ is SO, R₈ is hydrogen, R₉ is methyl, and Q_{Rx}, A, R₁₄, G₁, G₂, G₃, G₄ and G₅ are as defined in Table 10.
Table 12: This table discloses the 9 compounds 12.001 to 12.009 of the formula I-4a-U2, wherein X₄ is SO₂, R₈ is hydrogen, R₉ is methyl, and Q_{Rx}, A, R₁₄, G₁, G₂, G₃, G₄ and G₅ are as defined in Table 10.
Table 13: This table discloses the 4 compounds 13.001 to 13.004 of the formula I-1b-U0: 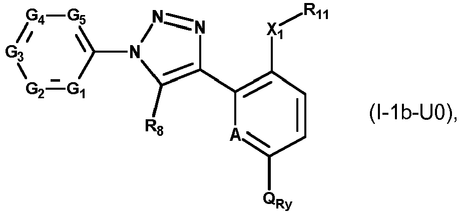 wherein X₁ is S, R₈ is hydrogen, and Q_{Ry}, A, R₁₁, G₁, G₂, G₃, G₄ and G₅ are as defined below:

**Table 13:**

| Comp. No | Q_{Ry} | A | R₁₁ | G₁ | G₂ | G₃ | G₄ | G₅ |
|---|---|---|---|---|---|---|---|---|
| 13.001 | CF₃ | N | -CH₂CH₃ | CH | CH | C(CF₃) | CH | CH |
| 13.002 | CF₃ | N | -CH₂CH₃ | CH | CH | CH | C(CF₃) | CH |
| 13.003 | CF₃ | N | -CH₂CH₃ | N | CH | CH | C(CF₃) | CH |
| 13.004 | CF₃ | N | -CH₂CH₃ | CH | N | C(CF₃) | CH | CH |

and the N-oxides of the compounds of Table 13.
Table 14: This table discloses the 4 compounds 14.001 to 14.004 of the formula I-1b-U0, wherein X₁ is SO, R₈ is hydrogen, and Q_{Ry}, A, R₁₁, G₁, G₂, G₃, G₄ and G₅ are as defined in Table 13.
Table 15: This table discloses the 4 compounds 15.001 to 15.004 of the formula I-1b-U0, wherein X₁ is SO₂, R₈ is hydrogen, and Q_{Ry}, A, R₁₁, G₁, G₂, G₃, G₄ and G₅ are as defined in Table 13.
Table 16: This table discloses the 4 compounds 16.001 to 16.004 of the formula I-2b-U3: 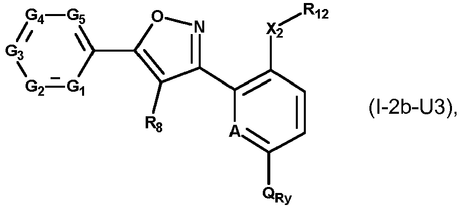 wherein X₂ is S, R₈ is hydrogen, and Q_{Ry}, A, R₁₂, G₁, G₂, G₃, G₄ and G₅ are as defined below:

**Table 16:**

| Comp. No | Q_{Ry} | A | R₁₂ | G₁ | G₂ | G₃ | G₄ | G₅ |
|---|---|---|---|---|---|---|---|---|
| 16.001 | CF₃ | N | -CH₂CH₃ | CH | CH | C(CF₃) | CH | CH |
| 16.002 | CF₃ | N | -CH₂CH₃ | CH | CH | CH | C(CF₃) | CH |
| 16.003 | CF₃ | N | -CH₂CH₃ | N | CH | CH | C(CF₃) | CH |
| 16.004 | CF₃ | N | -CH₂CH₃ | CH | N | C(CF₃) | CH | CH |

and the N-oxides of the compounds of Table 16.
Table 17: This table discloses the 4 compounds 17.001 to 17.004 of the formula I-2b-U3, wherein X₂ is SO, R₈ is hydrogen, and Q_{Ry}, A, R₁₂, G₁, G₂, G₃, G₄ and G₅ are as defined in Table 16.
Table 18: This table discloses the 4 compounds 18.001 to 18.004 of the formula I-2b-U3, wherein X₂ is SO₂, R₈ is hydrogen, and Q_{Ry}, A, R₁₂, G₁, G₂, G₃, G₄ and G₅ are as defined in Table 16.
Table 19: This table discloses the 4 compounds 19.001 to 19.004 of the formula I-3b-U5: 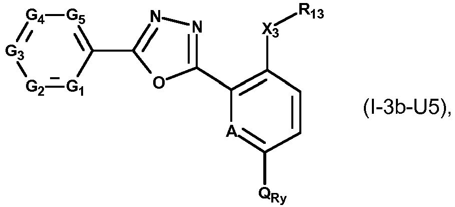 wherein X₃ is S, and Q_{Ry}, A, R₁₃, G₁, G₂, G₃, G₄ and G₅ are as defined below:

**Table 19:**

| Comp. No | Q_{Ry} | A | R₁₃ | G₁ | G₂ | G₃ | G₄ | G₅ |
|---|---|---|---|---|---|---|---|---|
| 19.001 | CF₃ | N | -CH₂CH₃ | CH | CH | C(CF₃) | CH | CH |
| 19.002 | CF₃ | N | -CH₂CH₃ | CH | CH | CH | C(CF₃) | CH |
| 19.003 | CF₃ | N | -CH₂CH₃ | N | CH | CH | C(CF₃) | CH |
| 19.004 | CF₃ | N | -CH₂CH₃ | CH | N | C(CF₃) | CH | CH |

and the N-oxides of the compounds of Table 19.
Table 20: This table discloses the 4 compounds 20.001 to 20.004 of the formula I-3b-U5, wherein X₃ is SO, and Q_{Ry}, A, R₁₃, G₁, G₂, G₃, G₄ and G₅ are as defined in Table 19.
Table 21: This table discloses the 4 compounds 21.001 to 21.004 of the formula I-3b-U5, wherein X₃ is SO₂, and Q_{Ry}, A, R₁₃, G₁, G₂, G₃, G₄ and G₅ are as defined in Table 19.
Table 22: This table discloses the 10 compounds 22.001 to 22.010 of the formula I-4b-U2: 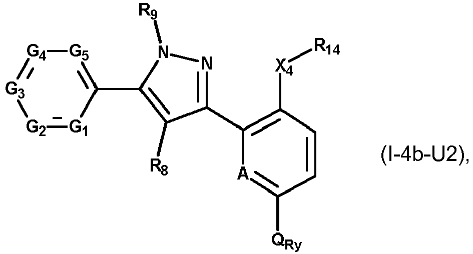 wherein X₄ is S, R₈ is hydrogen, R₉ is methyl, and Q_{Ry}, A, R₁₄, G₁, G₂, G₃, G₄ and G₅ are as defined below:

**Table 22:**

| Comp. No | Q_{Ry} | A | R₁₄ | G₁ | G₂ | G₃ | G₄ | G₅ |
|---|---|---|---|---|---|---|---|---|
| 22.001 | CF₃ | N | -CH₂CH₃ | CH | CH | C(CF₃) | CH | CH |
| 22.002 | CF₃ | N | -CH₂CH₃ | CH | CH | CH | C(CF₃) | CH |
| 22.003 | CF₃ | N | -CH₂CH₃ | N | CH | CH | C(CF₃) | CH |
| 22.004 | CF₃ | N | -CH₂CH₃ | CH | N | C(CF₃) | CH | CH |
| 22.005 | Cl | N | -CH₂CH₃ | N | CH | CH | C(CF₃) | CH |
| 22.006 | 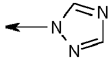 | N | -CH₂CH₃ | N | CH | CH | C(CF₃) | CH |
| 22.007 | Cl | N | -CH₂CH₃ | CH | N | C(CF₃) | CH | CH |
| 22.008 | 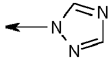 | N | -CH₂CH₃ | CH | N | C(CF₃) | CH | CH |
| 22.009 | Cl | N | -CH₂CH₃ | CH | N | CH | C(CF₃) | CH |
| 22.010 | 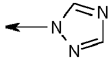 | N | -CH₂CH₃ | CH | N | CH | C(CF₃) | CH |

and the N-oxides of the compounds of Table 22.
Table 23: This table discloses the 10 compounds 23.001 to 23.010 of the formula I-4b-U2, wherein X₄ is SO, R₈ is hydrogen, R₉ is methyl, and Q_{Ry}, A, R₁₄, G₁, G₂, G₃, G₄ and G₅ are as defined in Table 22.
Table 24: This table discloses the 10 compounds 24.001 to 24.010 of the formula I-4b-U2, wherein X₄ is SO₂, R₈ is hydrogen, R₉ is methyl, and Q_{Ry}, A, R₁₄, G₁, G₂, G₃, G₄ and G₅ are as defined in Table 22.
Table 25: This table discloses the 2 compounds 25.001 to 25.002 of the formula I-2a-U3: 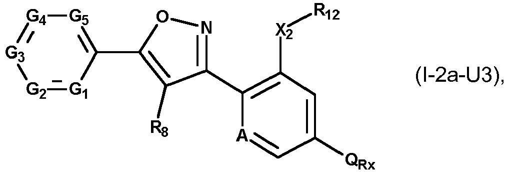 wherein X₂ is S, R₈ is methyl, and Q_{Rx}, A, R₁₂, G₁, G₂, G₃, G₄ and G₅ are as defined below:

**Table 25:**

| Comp. No | Q_{Rx} | A | R₁₂ | G₁ | G₂ | G₃ | G₄ | G₅ |
|---|---|---|---|---|---|---|---|---|
| 25.001 | H | N | -CH₂CH₃ | CH | CH | C(CF₃) | CH | CH |
| 25.002 | CF₃ | N | -CH₂CH₃ | CH | CH | C(CF₃) | CH | CH |

and the N-oxides of the compounds of Table 25.
Table 26: This table discloses the 2 compounds 26.001 to 26.002 of the formula I-2a-U3, wherein X₂ is SO, R₈ is methyl, and Q_{Rx}, A, R₁₂, G₁, G₂, G₃, G₄ and G₅ are as defined in Table 25.
Table 27: This table discloses the 2 compounds 27.001 to 27.002 of the formula I-2a-U3, wherein X₂ is SO₂, R₈ is methyl, and Q_{Rx}, A, R₁₂, G₁, G₂, G₃, G₄ and G₅ are as defined in Table 25.

The compounds of formula I are preventively and/or curatively valuable active ingredients in the field of pest control, even at low rates of application, which have a very favorable biocidal spectrum and are well tolerated by warm-blooded species, fish and plants. The active ingredients according to the invention act against all or individual developmental stages of normally sensitive, but also resistant, animal pests, such as insects or representatives of the order Acarina. The insecticidal or acaricidal activity of the active ingredients according to the invention can manifest itself directly, i. e. in destruction of the pests, which takes place either immediately or only after some time has elapsed, for example during ecdysis, or indirectly, for example in a reduced oviposition and/or hatching rate, a good activity corresponding to a destruction rate (mortality) of at least 50 to 60%.

Examples of the abovementioned animal pests are:
from the order *Acarina,* for example,
   Acalitus spp, Aculus spp, Acaricalus spp, Aceria spp, Acarus siro, Amblyomma spp., Argas spp., Boophilus spp., Brevipalpus spp., Bryobia spp, Calipitrimerus spp., Chorioptes spp., Dermanyssus gallinae, Dermatophagoides spp, Eotetranychus spp, Eriophyes spp., Hemitarsonemus spp, Hyalomma spp., Ixodes spp., Olygonychus spp, Ornithodoros spp., Polyphagotarsone latus, Panonychus spp., Phyllocoptruta oleivora, Phytonemus spp, Polyphagotarsonemus spp, Psoroptes spp., Rhipicephalus spp., Rhizoglyphus spp., Sarcoptes spp., Steneotarsonemus spp, Tarsonemus spp. and Tetranychus spp.;
from the order *Anoplura,* for example,
   Haematopinus spp., Linognathus spp., Pediculus spp., Pemphigus spp. and Phylloxera spp.;
from the order *Coleoptera,* for example,
   Agriotes spp., Amphimallon majale, Anomala orientalis, Anthonomus spp., Aphodius spp, Astylus atromaculatus, Ataenius spp, Atomaria linearis, Chaetocnema tibialis, Cerotoma spp, Conoderus spp, Cosmopolites spp., Cotinis nitida, Curculio spp., Cyclocephala spp, Dermestes spp., Diabrotica spp., Diloboderus abderus, Epilachna spp., Eremnus spp., Heteronychus arator, Hypothenemus hampei, Lagria vilosa, Leptinotarsa decemLineata, Lissorhoptrus spp., Liogenys spp, Maecolaspis spp, Maladera castanea, Megascelis spp, Melighetes aeneus, Melolontha spp., Myochrous armatus, Orycaephilus spp., Otiorhynchus spp., Phyllophaga spp, Phlyctinus spp., Popillia spp., Psylliodes spp., Rhyssomatus aubtilis, Rhizopertha spp., Scarabeidae, Sitophilus spp., Sitotroga spp., Somaticus spp, Sphenophorus spp, Sternechus subsignatus, Tenebrio spp., Tribolium spp. and Trogoderma spp.;
from the order *Diptera,* for example,
   Aedes spp., Anopheles spp, Antherigona soccata,Bactrocea oleae, Bibio hortulanus, Bradysia spp, Calliphora erythrocephala, Ceratitis spp., Chrysomyia spp., Culex spp., Cuterebra spp., Dacus spp., Delia spp, Drosophila melanogaster, Fannia spp., Gastrophilus spp., Geomyza tripunctata, Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis spp, Rivelia quadrifasciata, Scatella spp, Sciara spp., Stomoxys spp., Tabanus spp., Tannia spp. and Tipula spp.;
from the order *Hemiptera,* for example,
   Acanthocoris scabrator, Acrosternum spp, Adelphocoris lineolatus, Amblypelta nitida, Bathycoelia thalassina, Blissus spp, Cimex spp., Clavigralla tomentosicollis, Creontiades spp, Distantiella theobroma, Dichelops furcatus, Dysdercus spp., Edessa spp, Euchistus spp., Eurydema pulchrum, Eurygaster spp., Halyomorpha halys, Horcias nobilellus, Leptocorisa spp., Lygus spp, Margarodes spp, Murgantia histrionic, Neomegalotomus spp, Nesidiocoris tenuis, Nezara spp., Nysius simulans, Oebalus insularis, Piesma spp., Piezodorus spp, Rhodnius spp., Sahlbergella singularis, Scaptocoris castanea, Scotinophara spp. , Thyanta spp , Triatoma spp., Vatiga illudens;
   Acyrthosium pisum, Adalges spp, Agalliana ensigera, Agonoscena targionii, Aleurodicus spp, Aleurocanthus spp, Aleurolobus barodensis, Aleurothrixus floccosus, Aleyrodes brassicae, Amarasca biguttula, Amritodus atkinsoni, Aonidiella spp., Aphididae, Aphis spp., Aspidiotus spp., Aulacorthum solani, Bactericera cockerelli, Bemisia spp, Brachycaudus spp, Brevicoryne brassicae, Cacopsylla spp, Cavariella aegopodii Scop., Ceroplaster spp., Chrysomphalus aonidium, Chrysomphalus dictyospermi, Cicadella spp, Cofana spectra, Cryptomyzus spp, Cicadulina spp, Coccus hesperidum, Dalbulus maidis, Dialeurodes spp, Diaphorina citri, Diuraphis noxia, Dysaphis spp, Empoasca spp., Eriosoma larigerum, Erythroneura spp., Gascardia spp., Glycaspis brimblecombei, Hyadaphis pseudobrassicae, Hyalopterus spp, Hyperomyzus pallidus, Idioscopus clypealis, Jacobiasca lybica, Laodelphax spp., Lecanium corni, Lepidosaphes spp., Lopaphis erysimi, Lyogenys maidis, Macrosiphum spp., Mahanarva spp, Metcalfa pruinosa, Metopolophium dirhodum, Myndus crudus, Myzus spp., Neotoxoptera sp, Nephotettix spp., Nilaparvata spp., Nippolachnus piri Mats, Odonaspis ruthae, Oregma lanigera Zehnter, Parabemisia myricae, Paratrioza cockerelli, Parlatoria spp., Pemphigus spp., Peregrinus maidis, Perkinsiella spp, Phorodon humuli, Phylloxera spp, Planococcus spp., Pseudaulacaspis spp., Pseudococcus spp., Pseudatomoscelis seriatus, Psylla spp., Pulvinaria aethiopica, Quadraspidiotus spp., Quesada gigas, Recilia dorsalis, Rhopalosiphum spp., Saissetia spp., Scaphoideus spp., Schizaphis spp., Sitobion spp., Sogatella furcifera, Spissistilus festinus, Tarophagus Proserpina, Toxoptera spp, Trialeurodes spp, Tridiscus sporoboli, Trionymus spp, Trioza erytreae , Unaspis citri, Zygina flammigera, Zyginidia scutellaris, ;
from the order *Hymenoptera,* for example,
   Acromyrmex, Arge spp, Atta spp., Cephus spp., Diprion spp., Diprionidae, Gilpinia polytoma, Hoplocampa spp., Lasius spp., Monomorium pharaonis, Neodiprion spp., Pogonomyrmex spp, Slenopsis invicta, Solenopsis spp. and Vespa spp.;
from the order *Isoptera,* for example,
   Coptotermes spp, Corniternes cumulans, Incisitermes spp, Macrotermes spp, Mastotermes spp, Microtermes spp, Reticulitermes spp.; Solenopsis geminate
from the order *Lepidoptera,* for example,
   Acleris spp., Adoxophyes spp., Aegeria spp., Agrotis spp., Alabama argillaceae, Amylois spp., Anticarsia gemmatalis, Archips spp., Argyresthia spp, Argyrotaenia spp., Autographa spp., Bucculatrix thurberiella, Busseola fusca, Cadra cautella, Carposina nipponensis, Chilo spp., Choristoneura spp., Chrysoteuchia topiaria, Clysia ambiguella, Cnaphalocrocis spp., Cnephasia spp., Cochylis spp., Coleophora spp., Colias lesbia, Cosmophila flava, Crambus spp, Crocidolomia binotalis, Cryptophlebia leucotreta, Cydalima perspectalis, Cydia spp., Diaphania perspectalis, Diatraea spp., Diparopsis castanea, Earias spp., Eldana saccharina, Ephestia spp., Epinotia spp, Estigmene acrea, Etiella zinckinella, Eucosma spp., Eupoecilia ambiguella, Euproctis spp., Euxoa spp., Feltia jaculiferia, Grapholita spp., Hedya nubiferana, Heliothis spp., Hellula undalis, Herpetogramma spp, Hyphantria cunea, Keiferia lycopersicella, Lasmopalpus lignosellus, Leucoptera scitella, Lithocollethis spp., Lobesia botrana, Loxostege bifidalis, Lymantria spp., Lyonetia spp., Malacosoma spp., Mamestra brassicae, Manduca sexta, Mythimna spp, Noctua spp, Operophtera spp., Orniodes indica, Ostrinia nubilalis, Pammene spp., Pandemis spp., Panolis flammea, Papaipema nebris, Pectinophora gossypiela, Perileucoptera coffeella, Pseudaletia unipuncta, Phthorimaea operculella, Pieris rapae, Pieris spp., Plutella xylostella, Prays spp., Pseudoplusia spp, Rachiplusia nu, Richia albicosta, Scirpophaga spp., Sesamia spp., Sparganothis spp., Spodoptera spp., Sylepta derogate, Synanthedon spp., Thaumetopoea spp., Tortrix spp., Trichoplusia ni, Tuta absoluta, and Yponomeuta spp.;
from the order *Mallophaga,* for example,
   Damalinea spp. and Trichodectes spp.;
from the order *Orthoptera,* for example,
   Blatta spp., Blattella spp., Gryllotalpa spp., Leucophaea maderae, Locusta spp., Neocurtilla hexadactyla, Periplaneta spp. , Scapteriscus spp, and Schistocerca spp.;
from the order *Psocoptera,* for example,
   Liposcelis spp.;
from the order *Siphonaptera,* for example,
   Ceratophyllus spp., Ctenocephalides spp. and Xenopsylla cheopis;
from the order *Thysanoptera,* for example,
   Calliothrips phaseoli, Frankliniella spp., Heliothrips spp, Hercinothrips spp., Parthenothrips spp, Scirtothrips aurantii, Sericothrips variabilis, Taeniothrips spp., Thrips spp;
from the order *Thysanura,* for example, Lepisma saccharina.

The active ingredients according to the invention can be used for controlling, i. e. containing or destroying, pests of the abovementioned type which occur in particular on plants, especially on useful plants and ornamentals in agriculture, in horticulture and in forests, or on organs, such as fruits, flowers, foliage, stalks, tubers or roots, of such plants, and in some cases even plant organs which are formed at a later point in time remain protected against these pests.

Suitable target crops are, in particular, cereals, such as wheat, barley, rye, oats, rice, maize or sorghum; beet, such as sugar or fodder beet; fruit, for example pomaceous fruit, stone fruit or soft fruit, such as apples, pears, plums, peaches, almonds, cherries or berries, for example strawberries, raspberries or blackberries; leguminous crops, such as beans, lentils, peas or soya; oil crops, such as oilseed rape, mustard, poppies, olives, sunflowers, coconut, castor, cocoa or ground nuts; cucurbits, such as pumpkins, cucumbers or melons; fibre plants, such as cotton, flax, hemp or jute; citrus fruit, such as oranges, lemons, grapefruit or tangerines; vegetables, such as spinach, lettuce, asparagus, cabbages, carrots, onions, tomatoes, potatoes or bell peppers; Lauraceae, such as avocado, Cinnamonium or camphor; and also tobacco, nuts, coffee, eggplants, sugarcane, tea, pepper, grapevines, hops, the plantain family and latex plants.

The compositions and/or methods of the present invention may be also used on any ornamental and/or vegetable crops, including flowers, shrubs, broad-leaved trees and evergreens.

For example the invention may be used on any of the following ornamental species: *Ageratum* spp., *Alonsoa* spp., *Anemone* spp., *Anisodontea capsenisis, Anthemis* spp., *Antirrhinum* spp., *Aster* spp., *Begonia* spp. (e.g. *B. elatior, B. semperflorens, B. tubéreux), Bougainvillea* spp., *Brachycome* spp., *Brassica* spp. (ornamental), *Calceolaria* spp., *Capsicum annuum, Catharanthus roseus, Canna* spp., *Centaurea* spp., *Chrysanthemum* spp., *Cineraria* spp. (*C*. *maritime*), *Coreopsis* spp., *Crassula coccinea, Cuphea ignea, Dahlia* spp., *Delphinium* spp., *Dicentra spectabilis, Dorotheantus* spp., *Eustoma grandiflorum, Forsythia* spp., *Fuchsia* spp., *Geranium gnaphalium, Gerbera* spp., *Gomphrena globosa, Heliotropium* spp., *Helianthus* spp., *Hibiscus* spp., *Hortensia* spp., *Hydrangea* spp., *Hypoestes phyllostachya, Impatiens* spp. (*I. Walleriana*), *Iresines* spp., *Kalanchoe* spp., *Lantana camara, Lavatera trimestris, Leonotis leonurus, Lilium* spp., *Mesembryanthemum* spp., *Mimulus* spp., *Monarda* spp., *Nemesia* spp., *Tagetes* spp., *Dianthus* spp. (carnation), *Canna* spp., *Oxalis* spp., *Bellis* spp., *Pelargonium* spp. *(P. peltatum, P. Zonale*), *Viola* spp. (pansy), *Petunia* spp., *Phlox* spp., *Plecthranthus* spp., *Poinsettia* spp., *Parthenocissus* spp. *(P. quinquefolia, P. tricuspidata), Primula* spp., *Ranunculus* spp., *Rhododendron* spp., Rosa spp. (rose), *Rudbeckia* spp., *Saintpaulia* spp., *Salvia* spp., *Scaevola aemola, Schizanthus wisetonensis, Sedum* spp., *Solanum* spp., *Surfinia* spp., *Tagetes* spp., *Nicotinia* spp., *Verbena* spp., *Zinnia* spp. and other bedding plants.

For example the invention may be used on any of the following vegetable species: *Allium* spp. (A. *sativum, A.. cepa, A. oschaninii, A. Porrum, A. ascalonicum, A. fistulosum), Anthriscus cerefolium, Apium graveolus, Asparagus officinalis, Beta vulgarus, Brassica* spp. (*B. Oleracea, B. Pekinensis, B. rapa*), *Capsicum annuum, Cicer arietinum, Cichorium endivia, Cichorum* spp. (*C*. *intybus, C. endivia*), *Citrillus lanatus, Cucumis* spp. (*C*. *sativus, C. melo*), *Cucurbita* spp. (*C. pepo, C. maxima*), *Cyanara* spp. (*C. scolymus, C. cardunculus*), *Daucus carota, Foeniculum vulgare, Hypericum* spp., *Lactuca sativa, Lycopersicon* spp. (*L. esculentum, L. lycopersicum*), *Mentha* spp., *Ocimum basilicum, Petroselinum crispum, Phaseolus* spp. (*P. vulgaris, P. coccineus*), *Pisum sativum, Raphanus sativus, Rheum rhaponticum, Rosemarinus* spp., *Salvia* spp., *Scorzonera hispanica, Solanum melongena, Spinacea oleracea, Valerianella* spp. (*V. locusta, V. eriocarpa*) and *Vicia faba.*

Preferred ornamental species include African violet, *Begonia, Dahlia, Gerbera, Hydrangea, Verbena, Rosa, Kalanchoe, Poinsettia, Aster, Centaurea, Coreopsis, Delphinium, Monarda, Phlox, Rudbeckia, Sedum, Petunia, Viola, Impatiens, Geranium, Chrysanthemum, Ranunculus, Fuchsia, Salvia, Hortensia,* rosemary, sage, St. Johnswort, mint, sweet pepper, tomato and cucumber.

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca(preferably in vegetables, vineyards), Leptinotarsa (preferably in potatos) and Chilo supressalis (preferably in rice).

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca(preferably in vegetables, vineyards), Leptinotarsa (preferably in potatos) and Chilo supressalis (preferably in rice).

In a further aspect, the invention may also relate to a method of controlling damage to plant and parts thereof by plant parasitic nematodes (Endoparasitic-, Semiendoparasitic- and Ectoparasitic nematodes), especially plant parasitic nematodes such as root knot nematodes, Meloidogyne hapla, Meloidogyne incognita, Meloidogyne javanica, Meloidogyne arenaria and other Meloidogyne species; cyst-forming nematodes, Globodera rostochiensis and other Globodera species; Heterodera avenae, Heterodera glycines, Heterodera schachtii, Heterodera trifolii, and other Heterodera species; Seed gall nematodes, Anguina species; Stem and foliar nematodes, Aphelenchoides species; Sting nematodes, Belonolaimus longicaudatus and other Belonolaimus species; Pine nematodes, Bursaphelenchus xylophilus and other Bursaphelenchus species; Ring nematodes, Criconema species, Criconemella species, Criconemoides species, Mesocriconema species; Stem and bulb nematodes, Ditylenchus destructor, Ditylenchus dipsaci and other Ditylenchus species; Awl nematodes, Dolichodorus species; Spiral nematodes, Heliocotylenchus multicinctus and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, Longidorus elongatus and other Longidorus species; Pin nematodes, Pratylenchus species; Lesion nematodes, Pratylenchus neglectus, Pratylenchus penetrans, Pratylenchus curvitatus, Pratylenchus goodeyi and other Pratylenchus species; Burrowing nematodes, Radopholus similis and other Radopholus species; Reniform nematodes, Rotylenchus robustus, Rotylenchus reniformis and other Rotylenchus species; Scutellonema species; Stubby root nematodes, Trichodorus primitivus and other Trichodorus species, Paratrichodorus species; Stunt nematodes, Tylenchorhynchus claytoni, Tylenchorhynchus dubius and other Tylenchorhynchus species; Citrus nematodes, Tylenchulus species; Dagger nematodes, Xiphinema species; and other plant parasitic nematode species, such as Subanguina spp., Hypsoperine spp., Macroposthonia spp., Melinius spp., Punctodera spp., and Quinisulcius spp..

The compounds of the invention may also have activity against the molluscs. Examples of which include, for example, Ampullariidae; Arion (A. ater, A. circumscriptus, A. hortensis, A. rufus); Bradybaenidae (Bradybaena fruticum); Cepaea (C. hortensis, C. Nemoralis); ochlodina; Deroceras (D. agrestis, D. empiricorum, D. laeve, D. reticulatum); Discus (D. rotundatus); Euomphalia; Galba (G. trunculata); Helicelia (H. itala, H. obvia); Helicidae Helicigona arbustorum); Helicodiscus; Helix (H. aperta); Limax (L. cinereoniger, L. flavus, L. marginatus, L. maximus, L. tenellus); Lymnaea; Milax (M. gagates, M. marginatus, M. sowerbyi); Opeas; Pomacea (P. canaticulata); Vallonia and Zanitoides.

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins, for example insecticidal proteins from Bacillus cereus or Bacillus popilliae; or insecticidal proteins from Bacillus thuringiensis, such as δ-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.
In the context of the present invention there are to be understood by δ-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 02/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 03/018810). Examples of such toxins or transgenic plants capable of synthesising such toxins are disclosed, for example, in EP-A-0 374 753, WO 93/07278, WO 95/34656, EP-A-0 427 529, EP-A-451 878 and WO 03/052073.
The processes for the preparation of such transgenic plants are generally known to the person skilled in the art and are described, for example, in the publications mentioned above. Cryl-type deoxyribonucleic acids and their preparation are known, for example, from WO 95/34656, EP-A-0 367 474, EP-A-0 401 979 and WO 90/13651.
The toxin contained in the transgenic plants imparts to the plants tolerance to harmful insects. Such insects can occur in any taxonomic group of insects, but are especially commonly found in the beetles (Coleoptera), two-winged insects (Diptera) and moths (Lepidoptera).
Transgenic plants containing one or more genes that code for an insecticidal resistance and express one or more toxins are known and some of them are commercially available. Examples of such plants are: YieldGard® (maize variety that expresses a Cry1Ab toxin); YieldGard Rootworm® (maize variety that expresses a Cry3Bb1 toxin); YieldGard Plus® (maize variety that expresses a Cry1Ab and a Cry3Bb1 toxin); Starlink® (maize variety that expresses a Cry9C toxin); Herculex I® (maize variety that expresses a Cry1Fa2 toxin and the enzyme phosphinothricine N-acetyltransferase (PAT) to achieve tolerance to the herbicide glufosinate ammonium); NuCOTN 33B® (cotton variety that expresses a Cry1Ac toxin); Bollgard I® (cotton variety that expresses a Cry1Ac toxin); Bollgard II® (cotton variety that expresses a Cry1Ac and a Cry2Ab toxin); VipCot® (cotton variety that expresses a Vip3A and a Cry1Ab toxin); NewLeaf® (potato variety that expresses a Cry3A toxin); NatureGard®, Agrisure® GT Advantage (GA21 glyphosate-tolerant trait), Agrisure® CB Advantage (Bt11 corn borer (CB) trait) and Protecta®.
Further examples of such transgenic crops are:
1. **Bt11 Maize** from Syngenta Seeds SAS, Chemin de I'Hobit 27, F-31 790 St. Sauveur, France, registration number C/FR/96/05/10. Genetically modified Zea *mays* which has been rendered resistant to attack by the European corn borer (*Ostrinia nubilalis* and *Sesamia nonagrioides*) by transgenic expression of a truncated toxin. Bt11 maize also transgenically expresses the enzyme PAT to achieve tolerance to the herbicide glufosinate ammonium.
2. **Bt176 Maize** from Syngenta Seeds SAS, Chemin de I'Hobit 27, F-31 790 St. Sauveur, France, registration number C/FR/96/05/10. Genetically modified Zea *mays* which has been rendered resistant to attack by the European corn borer (*Ostrinia nubilalis* and *Sesamia nonagrioides*) by transgenic expression of a toxin. Bt176 maize also transgenically expresses the enzyme PAT to achieve tolerance to the herbicide glufosinate ammonium.
3. **MIR604 Maize** from Syngenta Seeds SAS, Chemin de I'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
4. **MON 863 Maize** from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
**6. 1507 Maize** from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. **NK603** × **MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup® (contains glyphosate), and also a Cry1Ab toxin obtained from *Bacillus thuringiensis subsp. kurstaki* which brings about tolerance to certain Lepidoptera, include the European corn borer.
   Transgenic crops of insect-resistant plants are also described in BATS (Zentrum für Biosicherheit und Nachhaltigkeit, Zentrum BATS, Clarastrasse 13, 4058 Basel, Switzerland) Report 2003, (http://bats.ch).
   The term "crops" is to be understood as including also crop plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818 and EP-A-0 353 191. The methods of producing such transgenic plants are generally known to the person skilled in the art and are described, for example, in the publications mentioned above.

Antipathogenic substances which can be expressed by such transgenic plants include, for example, ion channel blockers, such as blockers for sodium and calcium channels, for example the viral KP1, KP4 or KP6 toxins; stilbene synthases; bibenzyl synthases; chitinases; glucanases; the so-called "pathogenesis-related proteins" (PRPs; see e.g. EP-A-0 392 225); antipathogenic substances produced by microorganisms, for example peptide antibiotics or heterocyclic antibiotics (see e.g. WO 95/33818) or protein or polypeptide factors involved in plant pathogen defence (so-called "plant disease resistance genes", as described in WO 03/000906).
Further areas of use of the compositions according to the invention are the protection of stored goods and store ambients and the protection of raw materials, such as wood, textiles, floor coverings or buildings, and also in the hygiene sector, especially the protection of humans, domestic animals and productive livestock against pests of the mentioned type.

The present invention also provides a method for controlling pests (such as mosquitoes and other disease vectors; see also http://www.who.int/malaria/vector_control/irs/en/). In one embodiment, the method for controlling pests comprises applying the compositions of the invention to the target pests, to their locus or to a surface or substrate by brushing, rolling, spraying, spreading or dipping. By way of example, an IRS (indoor residual spraying) application of a surface such as a wall, ceiling or floor surface is contemplated by the method of the invention. In another embodiment, it is contemplated to apply such compositions to a substrate such as non-woven or a fabric material in the form of (or which can be used in the manufacture of) netting, clothing, bedding, curtains and tents.

In one embodiment, the method for controlling such pests comprises applying a pesticidally effective amount of the compositions of the invention to the target pests, to their locus, or to a surface or substrate so as to provide effective residual pesticidal activity on the surface or substrate. Such application may be made by brushing, rolling, spraying, spreading or dipping the pesticidal composition of the invention. By way of example, an IRS application of a surface such as a wall, ceiling or floor surface is contemplated by the method of the invention so as to provide effective residual pesticidal activity on the surface. In another embodiment, it is contemplated to apply such compositions for residual control of pests on a substrate such as a fabric material in the form of (or which can be used in the manufacture of) netting, clothing, bedding, curtains and tents.

Substrates including non-woven, fabrics or netting to be treated may be made of natural fibres such as cotton, raffia, jute, flax, sisal, hessian, or wool, or synthetic fibres such as polyamide, polyester, polypropylene, polyacrylonitrile or the like. The polyesters are particularly suitable. The methods of textile treatment are known, e.g. WO 2008/151984, WO 2003/034823, US 5631072, WO 2005/64072, WO2006/128870, EP 1724392, WO2005113886 or WO 2007/090739.

Further areas of use of the compositions according to the invention are the field of tree injection/trunk treatment for all ornamental trees as well all sort of fruit and nut trees.

In the field of tree injection/trunk treatment, the compounds according to the present invention are especially suitable against wood-boring insects from the order *Lepidoptera* as mentioned above and from the order *Coleoptera,* especially against woodborers listed in the following tables A and B:

**Table A. Examples of exotic woodborers of economic importance.**

| Family | Species | Host or Crop Infested |
|---|---|---|
| Buprestidae | *Agrilus planipennis* | Ash |
| Cerambycidae | *Anoplura glabripennis* | Hardwoods |
| Scolytidae | *Xylosandrus crassiusculus* | Hardwoods |
| | *X. mutilatus* | Hardwoods |
| | *Tomicus piniperda* | Conifers |

**Table B. Examples of native woodborers of economic importance.**

| Family | Species | Host or Crop Infested |
|---|---|---|
| Buprestidae | *Agrilus anxius* | Birch |
| | *Agrilus politus* | Willow, Maple |
| | *Agrilus sayi* | Bayberry, Sweetfern |
| | *Agrilus vittaticolllis* | Apple, Pear, Cranberry, Serviceberry, Hawthorn |
| | *Chrysobothris femorata* | Apple, Apricot, Beech, Boxelder, Cherry, Chestnut, Currant, Elm, Hawthorn, Hackberry, Hickory, Horsechestnut, Linden, Maple, Mountain-ash, Oak, Pecan, Pear, Peach, Persimmon, Plum, Poplar, Quince, Redbud, Serviceberry, Sycamore, Walnut, Willow |
| | *Texania campestris* | Basswood, Beech, Maple, Oak, Sycamore, Willow, Yellow-poplar |
| Cerambycidae | *Goes pulverulentus* | Beech, Elm, Nuttall, Willow, Black oak, Cherrybark oak, Water oak, Sycamore |
| | *Goes tigrinus* | Oak |
| | *Neoclytus acuminatus* | Ash, Hickory, Oak, Walnut, Birch, Beech, Maple, Eastern hophornbeam, Dogwood, Persimmon, Redbud, Holly, Hackberry, Black locust, Honeylocust, Yellow-poplar, Chestnut, Osage-orange, Sassafras, Lilac, Mountain-mahogany, Pear, Cherry, Plum, Peach, Apple, Elm, Basswood, Sweetgum |
| | *Neoptychodes trilineatus* | Fig, Alder, Mulberry, Willow, Netleaf hackberry |
| | *Oberea ocellata* | Sumac, Apple, Peach, Plum, Pear, Currant, Blackberry |
| | *Oberea tripunctata* | Dogwood, Viburnum, Elm, Sourwood, Blueberry, Rhododendron, Azalea, Laurel, Poplar, Willow, Mulberry |
| | *Oncideres cingulata* | Hickory, Pecan, Persimmon, Elm, Sourwood, Basswood, Honeylocust, Dogwood, Eucalyptus, Oak, Hackberry, Maple, Fruit trees |
| | *Saperda calcarata* | Poplar |
| | *Strophiona nitens* | Chestnut, Oak, Hickory, Walnut, Beech, Maple |
| Scolytidae | *Corthylus columbianus* | Maple, Oak, Yellow-poplar, Beech, Boxelder, Sycamore, Birch, Basswood, Chestnut, Elm |
| | *Dendroctonus frontalis* | Pine |
| | *Dryocoetes betulae* | Birch, Sweetgum, Wild cherry, Beech, Pear |
| | *Monarthrum fasciatum* | Oak, Maple, Birch, Chestnut, Sweetgum, Blackgum, Poplar, Hickory, Mimosa, Apple, Peach, Pine |
| | *Phloeotribus liminaris* | Peach, Cherry, Plum, Black cherry, Elm, Mulberry, Mountain-ash |
| | *Pseudopityophthorus pruinosus* | Oak, American beech, Black cherry, Chickasaw plum, Chestnut, Maple, Hickory, Hornbeam, Hophornbeam |
| Sesiidae | *Paranthrene simulans* | Oak, American chestnut |
| | *Sannina uroceriformis* | Persimmon |
| | *Synanthedon exitiosa* | Peach, Plum, Nectarine, Cherry, Apricot, Almond, Black cherry |
| | *Synanthedon pictipes* | Peach, Plum, Cherry, Beach, Black Cherry |
| | *Synanthedon rubrofascia* | Tupelo |
| | *Synanthedon scitula* | Dogwood, Pecan, Hickory, Oak, Chestnut, Beech, Birch, Black cherry, Elm, Mountain-ash, Viburnum, Willow, Apple, Loquat, Ninebark, Bayberry |
| | *Vitacea polistiformis* | Grape |

The present invention may be also used to control any insect pests that may be present in turfgrass, including for example beetles, caterpillars, fire ants, ground pearls, millipedes, sow bugs, mites, mole crickets, scales, mealybugs ticks, spittlebugs, southern chinch bugs and white grubs. The present invention may be used to control insect pests at various stages of their life cycle, including eggs, larvae, nymphs and adults.
In particular, the present invention may be used to control insect pests that feed on the roots of turfgrass including white grubs (such as *Cyclocephala spp.* (e.g. masked chafer, *C. lurida*), *Rhizotrogus spp.* (e.g. European chafer, *R. majalis*), *Cotinus spp.* (e.g. Green June beetle, *C. nitida*), *Popillia spp.* (e.g. Japanese beetle, *P. japonica*), *Phyllophaga spp.* (e.g. May/June beetle), *Ataenius spp.* (e.g. Black turfgrass ataenius, *A. spretulus*), *Maladera spp.* (e.g. Asiatic garden beetle, *M. castanea*) and *Tomarus spp.*), ground pearls (*Margarodes* spp.), mole crickets (tawny, southern, and short-winged; *Scapteriscus* spp., *Gryllotalpa africana*) and leatherjackets (European crane fly, *Tipula spp*.).
The present invention may also be used to control insect pests of turfgrass that are thatch dwelling, including armyworms (such as fall armyworm *Spodoptera frugiperda,* and common armyworm *Pseudaletia unipuncta*), cutworms, billbugs (*Sphenophorus spp.,* such as S. *venatus verstitus* and S. *parvulus*), and sod webworms (such as *Crambus spp.* and the tropical sod webworm, *Herpetogramma phaeopteralis*).
The present invention may also be used to control insect pests of turfgrass that live above the ground and feed on the turfgrass leaves, including chinch bugs (such as southern chinch bugs, *Blissus insularis*), Bermudagrass mite (*Eriophyes cynodoniensis*), rhodesgrass mealybug (*Antonina graminis*), two-lined spittlebug (*Propsapia bicincta*), leafhoppers, cutworms (*Noctuidae* family), and greenbugs. The present invention may also be used to control other pests of turfgrass such as red imported fire ants (*Solenopsis invicta*) that create ant mounds in turf.

In the hygiene sector, the compositions according to the invention are active against ectoparasites such as hard ticks, soft ticks, mange mites, harvest mites, flies (biting and licking), parasitic fly larvae, lice, hair lice, bird lice and fleas.
Examples of such parasites are:
Of the order Anoplurida: Haematopinus spp., Linognathus spp., Pediculus spp. and Phtirus spp., Solenopotes spp..

Of the order Mallophagida: Trimenopon spp., Menopon spp., Trinoton spp., Bovicola spp., Werneckiella spp., Lepikentron spp., Damalina spp., Trichodectes spp. and Felicola spp..

Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp..

Of the order Siphonapterida, for example Pulex spp., Ctenocephalides spp., Xenopsylla spp., Ceratophyllus spp..

Of the order Heteropterida, for example Cimex spp., Triatoma spp., Rhodnius spp., Panstrongylus spp..

Of the order Blattarida, for example Blatta orientalis, Periplaneta americana, Blattelagermanica and Supella spp..

Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp..

Of the orders Actinedida (Prostigmata) and Acaridida (Astigmata), for example Acarapis spp., Cheyletiella spp., Ornithocheyletia spp., Myobia spp., Psorergatesspp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp..

The compositions according to the invention are also suitable for protecting against insect infestation in the case of materials such as wood, textiles, plastics, adhesives, glues, paints, paper and card, leather, floor coverings and buildings.
The compositions according to the invention can be used, for example, against the following pests: beetles such as Hylotrupes bajulus, Chlorophorus pilosis, Anobium punctatum, Xestobium rufovillosum, Ptilinuspecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthesrugicollis, Xyleborus spec.,Tryptodendron spec., Apate monachus, Bostrychus capucins, Heterobostrychus brunneus, Sinoxylon spec. and Dinoderus minutus, and also hymenopterans such as Sirex juvencus, Urocerus gigas, Urocerus gigas taignus and Urocerus augur, and termites such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis and Coptotermes formosanus, and bristletails such as Lepisma saccharina.

The compounds according to the invention can be used as pesticidal agents in unmodified form, but they are generally formulated into compositions in various ways using formulation adjuvants, such as carriers, solvents and surface-active substances. The formulations can be in various physical forms, e.g. in the form of dusting powders, gels, wettable powders, water-dispersible granules, water-dispersible tablets, effervescent pellets, emulsifiable concentrates, microemulsifiable concentrates, oil-in-water emulsions, oil-flowables, aqueous dispersions, oily dispersions, suspo-emulsions, capsule suspensions, emulsifiable granules, soluble liquids, water-soluble concentrates (with water or a water-miscible organic solvent as carrier), impregnated polymer films or in other forms known e.g. from the Manual on Development and Use of FAO and WHO Specifications for Pesticides, United Nations, First Edition, Second Revision (2010). Such formulations can either be used directly or diluted prior to use. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof.

The active ingredients can also be contained in very fine microcapsules. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, *N,N-*dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, n-hexane, n-octylamine, octadecanoic acid, octylamine acetate, oleic acid, oleylamine, o-xylene, phenol, polyethylene glycol, propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, *N*-methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances.

A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di-alkylphosphate esters; and also further substances described e.g. in McCutcheon's Detergents and Emulsifiers Annual, MC Publishing Corp., Ridgewood New Jersey (1981).

Further adjuvants that can be used in pesticidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and liquid and solid fertilisers.

The compositions according to the invention can include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Many oil derivatives are known from the Compendium of Herbicide Adjuvants, 10th Edition, Southern Illinois University, 2010.

The inventive compositions generally comprise from 0.1 to 99 % by weight, especially from 0.1 to 95 % by weight, of compounds of the present invention and from 1 to 99.9 % by weight of a formulation adjuvant which preferably includes from 0 to 25 % by weight of a surface-active substance. Whereas commercial products may preferably be formulated as concentrates, the end user will normally employ dilute formulations.
The rates of application vary within wide limits and depend on the nature of the soil, the method of application, the crop plant, the pest to be controlled, the prevailing climatic conditions, and other factors governed by the method of application, the time of application and the target crop. As a general guideline compounds may be applied at a rate of from 1 to 2000 I/ha, especially from 10 to 1000 I/ha.

Preferred formulations can have the following compositions (weight %):

**Emulsifiable concentrates:**

| | |
|---|---|
| active ingredient: | 1 to 95 %, preferably 60 to 90 % |
| surface-active agent: | 1 to 30 %, preferably 5 to 20 % |
| liquid carrier: | 1 to 80 %, preferably 1 to 35 % |

**Dusts:**

| | |
|---|---|
| active ingredient: | 0.1 to 10 %, preferably 0.1 to 5 % |
| solid carrier: | 99.9 to 90 %, preferably 99.9 to 99 % |

**Suspension concentrates:**

| | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

**Wettable powders:**

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

**Granules:**

| | |
|---|---|
| active ingredient: | 0.1 to 30 %, preferably 0.1 to 15 % |
| solid carrier: | 99.5 to 70 %, preferably 97 to 85 % |

The following Examples further illustrate, but do not limit, the invention.

| Wettable powders | a) | b) | c) |
|---|---|---|---|
| active ingredients | 25% | 50 % | 75 % |
| sodium lignosulfonate | 5% | 5 % | - |
| sodium lauryl sulfate | 3% | - | 5% |
| sodium diisobutylnaphthalenesulfonate | - | 6% | 10 % |
| phenol polyethylene glycol ether (7-8 mol of ethylene oxide) | - | 2 % | - |
| highly dispersed silicic acid | 5% | 10 % | 10 % |
| Kaolin | 62 % | 27 % | - |

The combination is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredients | 25% | 50 % | 75 % |
| light mineral oil | 5% | 5% | 5% |
| highly dispersed silicic acid | 5% | 5 % | - |
| Kaolin | 65 % | 40 % | - |
| Talcum | - | | 20 |

The combination is thoroughly mixed with the adjuvants and the mixture is thoroughly ground in a suitable mill, affording powders that can be used directly for seed treatment.

| Emulsifiable concentrate | |
|---|---|
| active ingredients | 10 % |
| octylphenol polyethylene glycol ether (4-5 mol of ethylene oxide) | 3% |
| calcium dodecylbenzenesulfonate | 3% |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredients | 5% | 6% | 4 % |
| Talcum | 95 % | - | - |
| Kaolin | - | 94 % | - |
| mineral filler | - | - | 96 % |

Ready-for-use dusts are obtained by mixing the combination with the carrier and grinding the mixture in a suitable mill. Such powders can also be used for dry dressings for seed.

| Extruder granules | |
|---|---|
| Active ingredients | 15 % |
| sodium lignosulfonate | 2 % |
| carboxymethylcellulose | 1 % |
| Kaolin | 82 % |

The combination is mixed and ground with the adjuvants, and the mixture is moistened with water. The mixture is extruded and then dried in a stream of air.

| Coated granules | |
|---|---|
| Active ingredients | 8% |
| polyethylene glycol (mol. wt. 200) | 3% |
| Kaolin | 89 % |

The finely ground combination is uniformly applied, in a mixer, to the kaolin moistened with polyethylene glycol. Non-dusty coated granules are obtained in this manner.

**Suspension concentrate**

| | |
|---|---|
| active ingredients | 40 % |
| propylene glycol | 10 % |
| nonylphenol polyethylene glycol ether (15 mol of ethylene oxide) | 6% |
| Sodium lignosulfonate | 10 % |
| carboxymethylcellulose | 1 % |
| silicone oil (in the form of a 75 % emulsion in water) | 1 % |
| Water | 32 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

**Flowable concentrate for seed treatment**

| | |
|---|---|
| active ingredients | 40 % |
| propylene glycol | 5% |
| copolymer butanol PO/EO | 2 % |
| Tristyrenephenole with 10-20 moles EO | 2 % |
| 1,2-benzisothiazolin-3-one (in the form of a 20% solution in water) | 0.5 % |
| monoazo-pigment calcium salt | 5% |
| Silicone oil (in the form of a 75 % emulsion in water) | 0.2 % |
| Water | 45.3 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of the combination are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

Formulation types include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP), a soluble granule (SG) or any technically feasible formulation in combination with agriculturally acceptable adjuvants.

### Preparatory Examples:

"Mp" means melting point in °C. Free radicals represent methyl groups. ¹H NMR measurements were recorded on a Brucker 400MHz spectrometer, chemical shifts are given in ppm relevant to a TMS standard. Spectra measured in deuterated solvents as indicated. Common abbreviations: aq = aqueous, min = minute, h = hour, sat = saturated, Rₜ = retention time, CH₂Cl₂ = dichloromethane, mCPBA = meta-chloroperoxybenzoic acid, EtOAc = ethyl acetate, MeOH = methanol, NaHCO₃ = sodium hydrogen carbonate. Either one of the LCMS or GCMS methods below was used to characterize the compounds. The characteristic LCMS/GCMS values obtained for each compound were the retention time ("Rt", recorded in min) and the measured molecular ion (M+H)⁺ or (M)⁺.

### LCMS or GCMS Methods:

**Method 1:**
   AN BASE, Apparatus: Agilent 1100 Bin. Pump: G1312A, degasser; autosampler, ColCom, DAD: Agilent G1315B, 220-320 nm, MSD: Agilent LC/MSD G6130B ESI, pos/neg 100-800; ELSD PL-ELS2100 gas flow 1.1 ml/min, gas temp: 50°C; column: Waters XSelect™ C18, 50×2.1 mm, 3.5µ, Temp: 25°C, Flow: 0.8 mL/min, Gradient: to = 2% A, t_{3.5min} = 98% A, t₆ₘᵢₙ = 98% A, Posttime: 2 min, Eluent A: 95% acetonitrile + 5% 10mM ammonium bicarbonate in water in acetonitrile, Eluent B: 10mM ammonium bicarbonate in water (pH=9.5).
**Method 2:**
   40S20, Instrument: GC: Agilent 6890N and MS: 5973 MSD, El-positive, Det.temp.: 280°C Mass range: 50-550; Column: RXi-5MS 20m, ID 180µm, df 0.18µm; Average velocity: 50 cm/s; Injection vol: 1 µl; Injector temp: 250°C; Split ratio: 100/1; Carrier gas: He; Initial temp: 40°C; Initial time: 1.5 min; Solvent delay: 1.0 min; Rate 40°C/min; Final temp 250°C; Final time 2.0 min.
**Method 3:**
   Spectra were recorded on a Mass Spectrometer from Waters (SQD or ZQ Single quadrupole mass spectrometer) equipped with an electrospray source (Polarity: positive or negative ions, Capillary: 3.00 kV, Cone range: 30-60 V, Extractor: 2.00 V, Source Temperature: 150°C, Desolvation Temperature: 350°C, Cone Gas Flow: 0 L/Hr, Desolvation Gas Flow: 650 L/Hr, Mass range: 100 to 900 Da) and an Acquity UPLC from Waters: Binary pump, heated column compartment and diode-array detector. Solvent degasser, binary pump, heated column compartment and diode-array detector. Column: Waters UPLC HSS T3, 1.8 µm, 30 x 2.1 mm, Temp: 60°C, DAD Wavelength range (nm): 210 to 500, Solvent Gradient: A = water + 5% MeOH + 0.05 % HCOOH, B= Acetonitrile + 0.05 % HCOOH; gradient: 0 min 0% B, 100% A; 2.7-3.0 min 100% B; Flow (ml/min) 0.85.
**Method 4:**
   Spectra were recorded on a Mass Spectrometer from Waters (SQD, SQDII or ZQ Single quadrupole mass spectrometer) equipped with an electrospray source (Polarity: positive or negative ions, Capillary: 3.00 kV, Cone range: 30-60 V, Extractor: 2.00 V, Source Temperature: 150°C, Desolvation Temperature: 350°C, Cone Gas Flow: 0 L/Hr, Desolvation Gas Flow: 650 L/Hr, Mass range: 100 to 900 Da) and an Acquity UPLC from Waters: Binary pump, heated column compartment and diode-array detector. Solvent degasser, binary pump, heated column compartment and diode-array detector. Column: Waters UPLC HSS T3, 1.8 mm, 30 x 2.1 mm, Temp: 60°C, DAD Wavelength range (nm): 210 to 500, Solvent Gradient: A = water + 5% MeOH + 0.05 % HCOOH, B= Acetonitrile + 0.05 % HCOOH; gradient: 10-100% B in 1.2 min; Flow (ml/min) 0.85.
**Method 5:**
   AN BASE LONG, Apparatus: Agilent 1100 Bin. Pump: G1312A, degasser; autosampler, ColCom, DAD: Agilent G1315B, 220-320 nm, MSD: Agilent LC/MSD G6130B ESI, pos/neg 100-800; ELSD PL-ELS2100 gas flow 1.1 ml/min, gas temp: 50°C; column: Waters XSelect™ C18, 50×2.1 mm, 3.5µ, Temp: 25°C, Flow: 0.8 mL/min, Gradient: to = 2% A, t_{3.5min} = 98% A, t₈ₘᵢₙ = 98% A, Posttime: 2 min, Eluent A: 95% acetonitrile + 5% 10mM ammonium bicarbonate in water in acetonitrile, Eluent B: 10mM ammonium bicarbonate in water (pH=9.5).

### EXAMPLE P1: Preparation of 3-(ethylsulfonyl)-5-(trifluoromethyl)-2-(1-(4-(trifluoromethyl)phenyl)-1H-1,2,3-triazol-4-yl)pyridine (compound P2):

### Step A-1: Preparation of 1-azido-4-(trifluoromethyl)benzene:

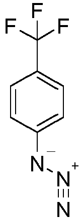

To a suspension of (4-(trifluoromethyl)phenyl)boronic acid (1.899 g, 10.00 mmol) in methanol (50 ml) were added sodium azide (0.975 g, 15.00 mmol) and cupric acetate monohydrate (0.200 g, 1.00 mmol). The mixture was stirred in an open flask at 55°C for 15 hours. The methanol was carefully removed in vacuo and the crude product was filtered over a plug of silica, using ether as the eluent. The filtrate was carefully concentrated in vacuo to afford the crude title compound (0.95 g) as an oil. Used as such for the next step. GCMS (method 2): 187 (M)⁺, retention time 3.63 min.

### Step B-1: Preparation of 3-chloro-5-(trifluoromethyl)-2-((trimethylsilyl)ethynyl)pyridine:

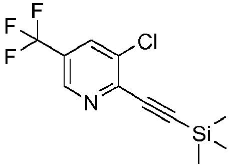

In a microwave vial, a mixture of 2,3-dichloro-5-(trifluoromethyl)pyridine (2.16 g, 10.0 mmol) and triethylamine (2.178 g, 21.52 mmol, 3.0 ml) in acetonitrile (anhydrous, 15 ml) was flushed with argon for 5 min. To this were added ethynyltrimethylsilane (1.050 g, 10.69 mmol, 1.5 ml) and bis(triphenylphosphine)palladium(II) chloride (0.351 g, 0.50 mmol) and copper(I) iodide (0.095 g, 0.50 mmol), the vial was closed and the mixture was heated in the microwave at 120°C for 1 h. The reaction mixture was concentrated and the residue was partitioned between ethyl acetate and water. After washing, the layers were separated, the organic layer was dried over sodium sulfate and concentrated. The crude product was purified over silica by flash column chromatography (0 to 20% gradient of EtOAc in heptane) to afford the title compound (1.90 g) as an oil. GCMS (method 2): 262/264 (M-CH₃)⁺, retention time 5.04 min. ¹H-NMR (CDCl₃, ppm) 0.32 (9H), 7.97 (1H), 8.71 (1H).

### Step B-2: Preparation of 3-chloro-2-ethynyl-5-(trifluoromethyl)pyridine:

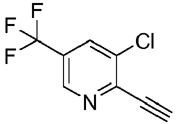

To a solution of 3-chloro-5-(trifluoromethyl)-2-((trimethylsilyl)ethynyl)pyridine (1.9 g, 6.84 mmol) in methanol (20 ml) was added potassium carbonate (catalytic amount) and the mixture was stirred at ambient temperature for 30 min. The reaction mixture was concentrated and the crude product was purified over silica by flash column chromatography (0 to 15% gradient of EtOAc in heptane) to afford the title compound (0.96 g) as an oil. GCMS (method 2): 205/207 (M)⁺, retention time 3.84 min. ¹H-NMR (CDCl₃, ppm) 3.62 (1H), 7.99 (1H), 8.75 (1H).

### Step C-1: Preparation of 3-chloro-5-(trifluoromethyl)-2-(1-(4-(trifluoromethyl)phenyl)-1H-1,2,3-triazol-4-yl)pyridine:

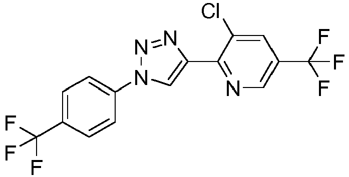

In a reaction vial, 3-chloro-2-ethynyl-5-(trifluoromethyl)pyridine (0.559 g, 2.72 mmol) and 1-azido-4-(trifluoromethyl)benzene (0.509 g, 2.72 mmol) were dissolved in *tert*-butanol/water (1/1, 12 ml) and to this were added L-ascorbic acid sodium salt (0.054 g, 0.272 mmol) and copper(II) sulfate pentahydrate (0.014 g, 0.054 mmol). The vial was closed and the mixture was stirred at ambient temperature for 4 h. The reaction mixture was poured into cold water (50 ml) while stirring and the aqueous suspension was cooled in ice. The solid was flitered off, washed with water and dried. The crude product was purified over silica by flash column chromatography (5 to 40% gradient of EtOAc in heptane). The fractions containing product were combined and concentrated. The product was again purified over silica by flash column chromatography (0 to 25% gradient of EtOAc in heptane) to afford the title compound (0.452 g) as a solid, mp 149-150°C. LCMS (method 1): 393/395 (M+H)⁺, retention time 4.06 min. ¹H-NMR (CDCl₃, ppm) 7.87 (2H), 8.02 (2H), 8.11 (1H), 8.82 (1H), 8.91 (1H).

### Step C-2: Preparation of 3-ethylsulfanyl-5-(trifluoromethyl)-2-(1-(4-(trifluoromethyl)phenyl)-1H-1,2,3-triazol-4-yl)pyridine (compound P1):

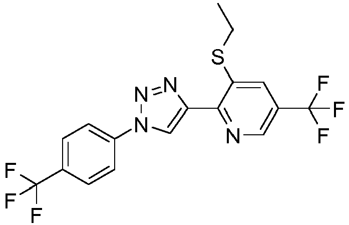

To a solution of 3-chloro-5-(trifluoromethyl)-2-(1-(4-(trifluoromethyl)phenyl)-1H-1,2,3-triazol-4-yl)pyridine (414 mg, 1.054 mmol) in N,N-dimethylformamide (dry) (4 ml) was added sodium ethanethiolate (106 mg, 1.265 mmol) and the mixture was stirred at ambient temperature for 1 h. The reaction mixture was diluted with water, brine was added and the product was extracted with EtOAc. The organic layer was washed with brine, dried over sodium sulfate and concentrated. The crude product was purified over silica by flash column chromatography (0 to 25% gradient of EtOAc in heptane) to afford the title compound (305 mg) P1 as a solid, mp 145-146°C. LCMS (method 1): 419 (M+H)⁺, retention time 4.24 min. ¹H-NMR (CDCl₃, ppm) 1.45 (3H), 3.08 (2H), 7.86 (3H), 8.01 (2H), 8.67 (1H), 8.75 (1H).

### Step C-3: Preparation of 3-(ethylsulfonyl)-5-(trifluoromethyl)-2-(1-(4-(trifluoromethyl)phenyl)-1H-1,2,3-triazol-4-yl)pyridine (title compound P2):

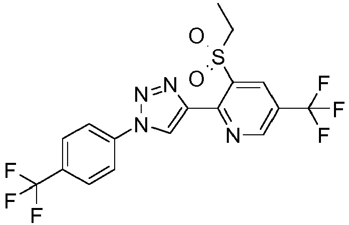

A solution of 3-ethylsulfanyl-5-(trifluoromethyl)-2-(1-(4-(trifluoromethyl)phenyl)-1H-1,2,3-triazol-4-yl)-pyridine (282 mg, 0.674 mmol) in dichloromethane (6.5 ml) was cooled in ice and to this was added mCPBA (70 wt% in water) (349 mg, 1.416 mmol). The cooling bath was removed and the mixture was stirred at ambient temperature for 15 hours. The reaction mixture was diluted with dichloromethane, washed three times with sat. NaHCO₃ (aq), dried over sodium sulfate and concentrated. The crude product was purified over silica by flash column chromatography (5 to 50% gradient of EtOAc in heptane). The fractions containing product were combined and concentrated. The product was was again purified over silica by flash column chromatography (0 to 2.5% gradient of MeOH in CH₂Cl₂) to afford the title compound (228 mg) P2 as a solid, mp 212-213°C. LCMS (method 1): 451 (M+H)⁺, retention time 3.99 min. ¹H-NMR (CDCl₃, ppm) 1.44 (3H), 4.06 (2H), 7.87 (2H), 8.01 (2H), 8.70 (1H), 8.80 (1H), 9.11 (1H).

### EXAMPLE P2: Preparation of 2-[3-ethylsulfonyl-5-(trifluoromethyl)-2-pyridyl]-5-[4-(trifluoromethyl)-2-pyridyl]-1,3,4-oxadiazole (compound P5):

### Step 1: Preparation of 3-chloro-5-(trifluoromethyl)pyridine-2-carbohydrazide:

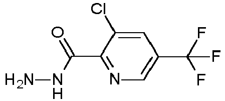

A solution of 3-chloro-5-(trifluoromethyl)pyridine-2-carbonyl chloride (15 g, 61.5 mmol) in dichloro-methane (80 ml) was added dropwise to a cooled (-18°C) solution of hydrazine monohydrate (30.8 g, 615 mmol, 29.9 ml) in dichloromethane (150 ml) under nitrogen. After complete addition, the cooling bath was removed and stirring was continued for 3 hours at ambient temperature. The reaction mixture was diluted with dichloromethane, filtered, the filtrate dried over sodium sulfate and concentrated. The residue was purified over silica by flash column chromatography (gradient of EtOAc in heptane) to afford the title compound as a solid. LCMS (method 3): 240/242 (M+H)⁺, retention time 0.62 min.

### Step 2: Preparation of 3-chloro-5-(trifluoromethyl)-N'-[4-(trifluoromethyl)pyridine-2-carbonyl]pyridine-2-carbohydrazide:

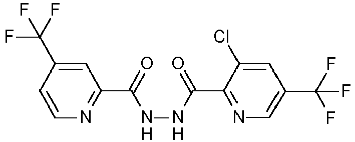

To a solution of 3-chloro-5-(trifluoromethyl)pyridine-2-carbohydrazide (500 mg, 2.09 mmol) in dichloro-methane (15 ml) was added 4-dimethylaminopyridine (70 mg) and pyridine (1.0 ml, 12 mmol). To this mixture at 0-5°C was added a solution of 4-(trifluoromethyl)pyridine-2-carbonyl chloride (481 mg, 2.30 mmol) in dichloromethane (10 ml) dropewise. After complete addition, the cooling bath was removed and stirring continued for 1 hour at ambient temperature. The reaction mixture was diluted with dichloro-methane, washed with an aqueous 1M hydrochloric acid solution (4x), the organic phase washed with water and brine, dried over sodium sulfate and concentrated to afford the title compound as a solid (797 mg). This material was used without further purification. LCMS (method 3): 413/415 (M+H)⁺, retention time 1.39 min.

### Step 3: Preparation of 2-[3-chloro-5-(trifluoromethyl)-2-pyridyl]-5-[4-(trifluoromethyl)-2-pyridyl]-1,3,4-oxadiazole:

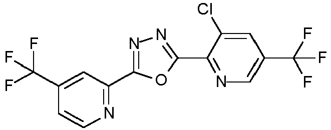

A solution of crude 3-chloro-5-(trifluoromethyl)-N'-[4-(trifluoromethyl)pyridine-2-carbonyl]pyridine-2-carbohydrazide (797 mg, 1.93 mmol) in phosphoryl chloride (12 ml) was heated to reflux for 13.5 hours, then further in the microwave at 130°C for 3 hours. The mixture was carefully poured on iced water, the solution neutralized by addition of sodium hydroxide and the product extracted with ethyl acetate. The organic layer was washed with sat. NaHCO₃ (aq), dried over sodium sulfate and concentrated. The residue was purified over silica by flash column chromatography (0-15% gradient of EtOAc in heptane) to afford the title compound as a solid (255 mg). LCMS (method 3): 395/397 (M+H)⁺, retention time 1.71 min.

### Step 4: Preparation of 2-[3-ethylsulfanyl-5-(trifluoromethyl)-2-pyridyl]-5-[4-(trifluoromethyl)-2-pyridyl]-1,3,4-oxadiazole (compound P4):

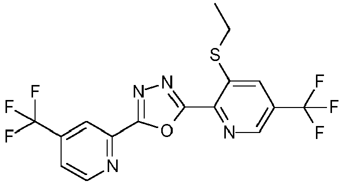

Obtained from 2-[3-chloro-5-(trifluoromethyl)-2-pyridyl]-5-[4-(trifluoromethyl)-2-pyridyl]-1,3,4-oxadiazole (253 mg, 0.64 mmol) and sodium ethanethiolate (92 mg, 0.98 mmol, 90%) in N,N-dimethylformamide (10 ml) according to procedure Example P1, step C-2. The mixture was stirred at ambient temperature for 1.5 hours. Title compound P4 as a solid (175 mg). LCMS (method 3): 421 (M+H)⁺; retention time: 1.87 min.

### Step 5: Preparation of 2-[3-ethylsulfonyl-5-(trifluoromethyl)-2-pyridyl]-5-[4-(trifluoromethyl)-2-pyridyl]-1,3,4-oxadiazole (compound P5):

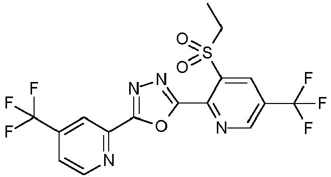

Obtained from 2-[3-ethylsulfanyl-5-(trifluoromethyl)-2-pyridyl]-5-[4-(trifluoromethyl)-2-pyridyl]-1,3,4-oxadiazole (170 mg, 0.404 mmol) and mCPBA (195 mg, 0.849 mmol, 75%) in dichloromethane (15 ml) according to procedure Example P1, step C-3. The mixture was stirred at ambient temperature for 15 hours. Flash chromatography purification (0-15% gradient of EtOAc in heptane) afforded 2-[3-ethylsulfonyl-5-(trifluoromethyl)-2-pyridyl]-5-[4-(trifluoromethyl)-2-pyridyl]-1,3,4-oxadiazole (title compound P5) as a solid (116 mg). LCMS (method 3): 453 (M+H)⁺; retention time: 1.63 min. ¹H-NMR (CDCl₃, ppm) 1.46 (t, *J*=7.34 Hz, 3H), 3.98 (q, *J*=7.34 Hz, 2H), 7.75 (d, *J*=5.14 Hz, 1H), 8.56 (s, 1H), 8.82 (m, 1H), 9.03 (d, *J*=5.14 Hz, 1H), 9.27 (s, 1H).

### EXAMPLE P3: Preparation of 3-(3-ethylsulfonyl-5-(trifluoromethyl)pyridin-2-yl)-5-(4-(trifluoromethyl)phenyl)isoxazole (compound P9):

### Step 1: Preparation of 3-ethylsulfanyl-5-(trifluoromethyl)picolinaldehyde oxime:

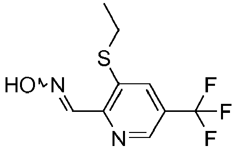

To a solution of 3-ethylsulfanyl-5-(trifluoromethyl)picolinaldehyde (235 mg, 0.999 mmol) in a mixture of ethanol (4.0 ml) and water (2.0 ml) were added hydroxylamine hydrochloride (139 mg, 1.998 mmol) and sodium acetate (246 mg, 3.00 mmol). The mixture was gently heated until complete dissolution was obtained, allowed to cool to ambient temperature and stirred for 15 hours. The mixture was concentrated and the residue was partitioned between ethyl acetate and sat. NaHCO₃ (aq). After washing and separation, the organic layer was washed once more with sat. NaHCO₃ (aq), dried over sodium sulfate and concentrated to afford the title compound (239 mg) is a solid. ¹H-NMR (CDCl₃, ppm) 1.39 (3H), 3.00 (2H), 7.84 (1H), 8.70 (3H).

### Step 2: Preparation of 3-ethylsulfanyl-N-hydroxy-5-(trifluoromethyl)picolinimidoyl chloride:

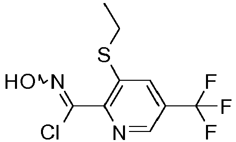

To a solution of 3-ethylsulfanyl-5-(trifluoromethyl)picolinaldehyde oxime (235 mg, 0.939 mmol) in dry N,N-dimethylformamide (2 ml) was added N-chlorosuccinimide (132 mg, 0.986 mmol) and the mixture was stirred at ambient temperature for 2 hours. Ethyl acetate was added, the organic layer was washed three times with brine/water (1/1), dried over sodium sulfate and concentrated. The residue was coevaporated with ether to afford the title compound (265 mg) is a solid, which was immediately used as such for the next step.

### Step 3: Preparation of 3-(3-ethylsulfanyl-5-(trifluoromethyl)pyridin-2-yl)-5-(4-(trifluoromethyl)phenyl)-isoxazole:

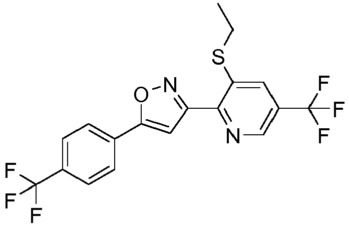

To a solution of 3-ethylsulfanyl-N-hydroxy-5-(trifluoromethyl)picolinimidoyl chloride (267 mg, 0.938 mmol) and 1-ethynyl-4-(trifluoromethyl)benzene (479 mg, 2.81 mmol) in 2-propanol (5 ml) was added sodium hydrogen carbonate (355 mg, 4.22 mmol) and the mixture was stirred at 65°C for 3 hours. The reaction mixture was concentrated and the residue was partitioned between ethyl acetate and water. After washing and separation, the organic layer was washed with brine, dried over sodium sulfate and concentrated. The crude product was purified over silica by flash column chromatography (5 to 40% gradient of EtOAc in heptane). The fractions containing product were combined and concentrated. The product was again purified over silica by flash column chromatography (0 to 25% gradient of diisopropyl ether in heptane) to afford the title compound (136 mg) as a solid, mp 145-146°C. LCMS (method 5): 419 (M+H)⁺, retention time 4.51 min. ¹H-NMR (CDCl₃, ppm) 1.46 (3H), 3.08 (2H), 7.32 (1H), 7.77 (2H), 7.89 (1H), 8.00 (2H), 8.71 (1H).

### Step 4: Preparation of 3-(3-ethylsulfonyl-5-(trifluoromethyl)pyridin-2-yl)-5-(4-(trifluoromethyl)phenyl)-isoxazole (compound P9):

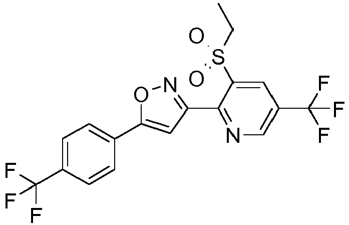

To a solution of 3-(3-ethylsulfanyl-5-(trifluoromethyl)pyridin-2-yl)-5-(4-(trifluoromethyl)phenyl)isoxazole (120 mg, 0.287 mmol) in dichloromethane (3 ml) was added mCPBA (70 wt% in water) (156 mg, 0.631 mmol) and the mixture was stirred at ambient temperature for 15 hours. The reaction mixture was diluted with dichloromethane, washed two times with sat. NaHCO₃ (aq), dried over sodium sulfate and concentrated. The crude product was purified over silica by flash column chromatography (0 to 40% gradient of EtOAc in heptane) to afford the title compound P9 (95 mg) as solid, mp 182-184°C. LCMS (method 5): 451 (M+H)⁺, retention time 4.25 min. ¹H-NMR (CDCl₃, ppm) 1.42 (3H), 3.84 (2H), 7.09 (1H), 7.79 (2H), 7.99 (2H), 8.79 (1H), 9.20 (1H).

### EXAMPLE P4: Preparation of 3-ethylsulfonyl-2-[1-[4-(trifluoromethyl)phenyl]triazol-4-yl]pyridine (compound P17) and 3-ethylsulfonyl-1-oxido-2-[1-[4-(trifluoromethyl)phenyl]triazol-4-yl]pyridin-1-ium (compound P18):

### Step 1: Preparation of 3-fluoro-2-[1-[4-(trifluoromethyl)phenyl]triazol-4-yl]pyridine

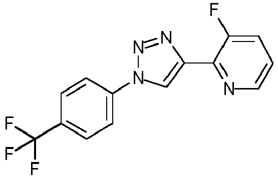

Iodine (1.56 g, 6.15 mmol) was added to a mixture of 4-(trifluoromethyl)aniline (0.62 ml, 4.92 mmol),1-(3-fluoro-2-pyridyl)ethanone (600 mg, 4.10 mmol) and p-toluenesulfonyl hydrazide (1.14 g, 6.15 mmol) in dry dimethylsulfoxide (20 ml) and the mixture was stirred at 100°C under air for one hour. After completion of the reaction, water was added to the reaction mixture and the product extracted with ethyl acetate (3x). The combined organic layers were washed with a 10% aqueous sodium thiosulfate solution, dried over anhydrous sodium sulfate and concentrated under vacuum. The residue was purified over silica by flash column chromatography (0 to 30% gradient of ethyl acetate in cyclohexane) to afford 3-fluoro-2-[1-[4-(trifluoromethyl)phenyl]triazol-4-yl]pyridine (820 mg) as solid, mp 163-164°C. LCMS (method 4): 309 (M+H)⁺, retention time 0.95 min. ¹H-NMR (CDCl₃, ppm) 7.36 (m, 1H), 7.58 (m, 1H), 7.85 (d, *J*=8.44 Hz, 2H), 8.01 (d, *J*=8.44 Hz, 2H), 8.60 (m, 2H).

### Step 2: Preparation of 3-ethylsulfanyl-2-[1-[4-(trifluoromethyl)phenyl]triazol-4-yl]pyridine (compound P16)

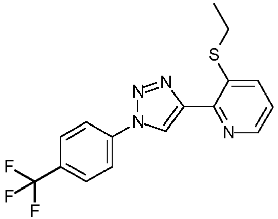

To a solution of sodium hydride (112 mg, ∼60%, 2.79 mmol) in tetrahydrofuran (10 ml) at 0-5°C was added a solution of 3-fluoro-2-[1-[4-(trifluoromethyl)phenyl]triazol-4-yl]pyridine (820 mg, 2.66 mmol) in tetrahydrofuran (10 ml) dropewise, followed after 30 minutes by a solution of ethanethiol (0.21 ml, 2.79 mmol) in tetrahydrofuran (5 ml). The reaction mixture was warmed to room temperature and stirred for 30 minutes, then quenched by addition of water. The resulting precipitate was filtered, solubilized in dichloromethane, the organic phase dried over anhydrous sodium sulfate and concentrated *in vacuo* to afford 3-ethylsulfanyl-2-[1-[4-(trifluoromethyl) phenyl]triazol-4-yl]pyridine (compound P16) as a solid (631 mg), mp 116-118°C. This material was used in the next step without further purification. LCMS (method 4): 351 (M+H)⁺, retention time 1.04 min. ¹H-NMR (CDCl₃, ppm) 1.39 (t, *J*=7.34 Hz, 3H), 3.02 (q, *J*=7.34 Hz, 2H), 7.26 (dd, *J*=8.07, 4.77 Hz, 1H), 7.74 (dd, *J*=8.07, 1.47 Hz, 1H), 7.83 (d, *J*=8.44 Hz, 2H), 8.00 (d, *J*=8.44 Hz, 2H), 8.48 (dd, *J*=4.77, 1.47 Hz, 1H), 8.68 (s, 1H).

### Step 3: Preparation of 3-ethylsulfonyl-2-[1-[4-(trifluoromethyl)phenyl]triazol-4-yl]pyridine (compound P17) and 3-ethylsulfonyl-1-oxido-2-[1-[4-(trifluoromethyl)phenyl]triazol-4-yl]pyridin-1-ium (compound P18)

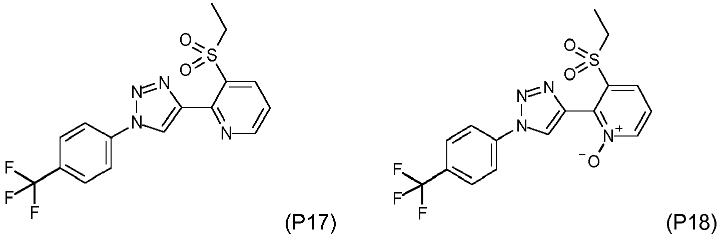

Obtained from 3-ethylsulfanyl-2-[1-[4-(trifluoromethyl)phenyl]triazol-4-yl]pyridine (500 mg, 1.43 mmol) and mCPBA (660 mg, 2.87 mmol, 75%) in dichloromethane (20 ml) according to procedure Example P1, step C-3. The mixture was stirred at ambient temperature overnight. Flash chromatography purification (0-100% gradient of ethyl acetate in cyclohexane) afforded first 3-ethylsulfonyl-2-[1-[4-(trifluoromethyl)phenyl]triazol-4-yl]pyridine (title compound P17) as a solid (400 mg), mp 182-183°C. LCMS (method 4): 383 (M+H)⁺; retention time: 0.96 min. ¹H-NMR (CDCl₃, ppm) 1.38 (t, *J*=7.34 Hz, 3H), 3.90 (q, *J*=7.34 Hz, 2H), 7.57 (dd, *J*=8.07, 4.77 Hz, 1H), 7.85 (d, *J*=8.44 Hz, 2H), 8.00 (d, *J*=8.44 Hz, 2H), 8.56 (dd, *J*=8.07, 1.47 Hz, 1H), 8.61 (s, 1H), 8.90 (dd, *J*=4.77, 1.47 Hz, 1H).

Further elution delivered 3-ethylsulfonyl-1-oxido-2-[1-[4-(trifluoromethyl)phenyl]triazol-4-yl]pyridin-1-ium (title compound P18) as a solid (80 mg), mp 236-238°C. LCMS (method 4): 399 (M+H)⁺; retention time: 0.85 min. ¹H-NMR (CDCl₃, ppm) 1.46 (t, *J*=7.34 Hz, 3H), 4.04 (q, *J*=7.34 Hz, 2H), 7.49 (dd, *J*=8.07, 6.60 Hz, 1H), 7.86 (m, *J*=8.80 Hz, 2H), 8.00 (m, *J*=8.80 Hz, 2H), 8.10 (dd, *J*=8.07, 1.10 Hz, 1H), 8.53 (dd, *J*=6.60, 1.10 Hz, 1H), 9.04 (s, 1H).

### EXAMPLE P5: Preparation of 5-(4-chlorophenyl)-3-ethylsulfonyl-2-[1-[3-(trifluoromethyl)phenyl]triazol-4-yl]pyridine (compound P13):

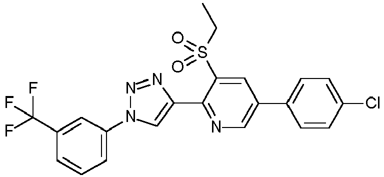

A solution of 5-bromo-3-ethylsulfonyl-2-[1-[3-(trifluoromethyl)phenyl]triazol-4-yl]pyridine (compound P12, obtained by analogy according to procedure Example P4) (30 mg, 0.065 mmol), (4-chlorophenyl)boronic acid (20.3 mg, 0.13 mmol), aqueous 2M sodium carbonate (0.098 ml, 0.195 mmol) in 1,2-dimethoxyethane (1.5 ml) was flushed with argon. Bis(triphenylphosphine) palladium(II) dichloride (0.01 eq.) was added, and the mixture was stirred in the microwave at 110°C for 30 minutes. The reaction mixture was diluted with ethyl acetate and water, the layers separated, the organic phase washed with water and brine, dried over anhydrous sodium sulfate and concentrated *in vacuo.* The residue was purified over silica by flash column chromatography (0 to 40% gradient of ethyl acetate in cyclohexane) to afford 5-(4-chlorophenyl)-3-ethylsulfonyl-2-[1-[3-(trifluoromethyl)phenyl]triazol-4-yl]pyridine (compound P13) as a solid (9 mg), mp 196-198°C. LCMS (method 4): 493/495 (M+H)⁺; retention time: 1.18 min.

### EXAMPLE P6: Preparation of 3-ethylsulfonyl-2-[1-methyl-5-[6-(trifluoromethyl)-3-pyridyl]pyrazol-3-yl]pyridine (compound P34)

### Step 1: Preparation of 1-(6-chloro-3-ethylsulfanyl-2-pyridyl)-3-[6-(trifluoromethyl)-3-pyridyl]propane-1,3-dione (compound W2)

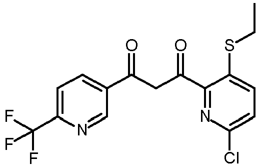

To a solution of 1-[6-(trifluoromethyl)-3-pyridyl]ethanone (4.03 g, 21.3 mmol ) at 0-5°C in tetrahydrofuran (110 ml) under argon was added a 1M [bis(trimethylsilyl)amino]lithium solution in tetrahydrofuran (LiHMDS, 42.6 ml, 42.6 mmol) dropwise. The mixture was stirred one hour at 5-10°C, then warmed to room temperature for one hour. Methyl 6-chloro-3-ethylsulfanyl-pyridine-2-carboxylate (4.94 g, 21.3 mmol) was added as a solid in 5 portions to the previous solution at 0-5°C, and stirring continued for one hour whilst warming to room temperature. Upon completion of the reaction, the mixture was concentrated *in vacuo* and the solid residue added to a vigorously stirred ice-cooled solution of acetic acid and water (100 ml, 1:1, v/v). The yellow suspension was stirred for 15 minutes, filtered and washed with cold water. The solid was dissolved into dichloromethane, the solution dried over sodium sulfate, filtered and concentrated *in vacuo* to dryness to afford 1-(6-chloro-3-ethylsulfanyl-2-pyridyl)-3-[6-(trifluoromethyl)-3-pyridyl]propane-1,3-dione (compound W2) as a solid (4.3 g), mp 123-125°C. This material was used in the next step without further purification. LCMS (method 4): 389/391 (M+H)⁺; retention time: 1.25 min. ¹H-NMR (CDCl₃, ppm) 1.43 (t, *J*=7.34 Hz, 3H), 2.98 (q, *J*=7.34 Hz, 2H), 7.42 (d, *J*=8.80 Hz, 1H), 7.54 (s, 1H), 7.70 (d, *J*=8.80 Hz, 1H), 7.80 (d, *J*=8.25 Hz, 1H), 8.42 (dd, *J*=8.25, 1.8 Hz, 1H), 9.28 (d, *J*=1.8 Hz, 1H), 15.25 (br s, 1H). [major tautomer, keto-enol form]

### Step 2: Preparation of 6-chloro-3-ethylsulfanyl-2-[1-methyl-5-[6-(trifluoromethyl)-3-pyridyl]pyrazol-3-yl]pyridine (compound P32)

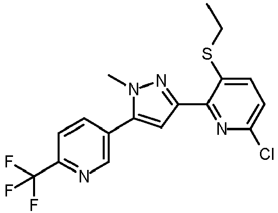

Two separate reaction vials were each charged with 1-(6-chloro-3-ethylsulfanyl-2-pyridyl)-3-[6-(trifluoromethyl)-3-pyridyl]propane-1,3-dione (2.1 g, 5.40 mmol), ethanol (15 ml) and methylhydrazine (0.293 ml, 5.40 mmol). Each mixture was flushed with argon, the vials were capped, and heated at 100°C for one hour. The mixture solutions were combined, cooled to room temperature under stirring, the resulting suspension filtered, the solid residue washed with cold ethanol and dried *in vacuo* at 40°C to afford a first crop of 6-chloro-3-ethylsulfanyl-2-[1-methyl-5-[6-(trifluoromethyl)-3-pyridyl] pyrazol-3-yl]pyridine (compound P32) as a pure solid (2.3 g). The mother liquor was concentrated *in vacuo* and the residue purified via Combiflash (RediSep gold-column; gradient 0-10% ethylacetate in cylohexane) to further deliver 6-chloro-3-ethylsulfanyl-2-[1-methyl-5-[6-(trifluoromethyl)-3-pyridyl] pyrazol-3-yl]pyridine (compound P32) as a solid (502 mg), mp 136-138°C. LCMS (method 3): 399/401 (M+H)⁺; retention time: 1.89 min. ¹H-NMR (CDCl₃, ppm) 1.39 (t, *J*=7.3 Hz, 3H), 2.98 (q, *J*=7.3 Hz, 2H), 4.05 (s, 3H), 7.12 (s, 1H), 7.24 (d, *J*=8.4 Hz, 1H), 7.62 (d, *J*=8.4 Hz, 1H), 7.83 (d, *J*=8.1 Hz, 1H), 8.01 (dd, *J*=8.1, 1.8 Hz, 1H), 8.89 (d, *J*=1.8 Hz, 1H).

The chromatographic purification also afforded the regioisomeric 6-chloro-3-ethylsulfanyl-2-[2-methyl-5-[6-(trifluoromethyl)-3-pyridyl]pyrazol-3-yl]pyridine as a solid (220 mg). LCMS (method 3): 399/401 (M+H)⁺; retention time: 1.96 min. ¹H-NMR (CDCl₃, ppm) 1.34 (t, *J*=7.3 Hz, 3H), 2.93 (q, *J*=7.3 Hz, 2H), 3.99 (s, 3H), 7.03 (s, 1H), 7.35 (d, *J*=8.5 Hz, 1H), 7.66 (d, *J*=8.5 Hz, 1H), 7.72 (d, *J*=8.1 Hz, 1H), 8.30 (dd, *J*=8.1, 1.8 Hz, 1H), 9.14 (d, *J*=1.8 Hz, 1H).

### Step 3: Preparation of 6-chloro-3-ethylsulfonyl-2-[1-methyl-5-[6-(trifluoromethyl)-3-pyridyl]pyrazol-3-yl]pyridine (compound P33)

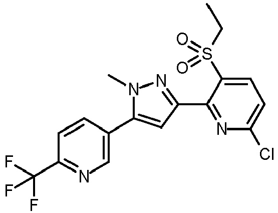

Obtained from 6-chloro-3-ethylsulfanyl-2-[1-methyl-5-[6-(trifluoromethyl)-3-pyridyl]pyrazol-3-yl]pyridine (2.7 g, 6.77 mmol) and mCPBA (3.3 g, 13.77 mmol, 72%) in dichloromethane (50 ml) according to procedure Example P1, step C-3. The mixture was stirred at 0-5°C for one hour, then warmed to ambient temperature. Aqueous workup afforded 6-chloro-3-ethylsulfonyl-2-[1-methyl-5-[6-(trifluoromethyl)-3-pyridyl]pyrazol-3-yl]pyridine (compound P33) as a solid (2.35 g), mp 198-200°C. This material was used in the next step without further purification. LCMS (method 4): 431/433 (M+H)⁺; retention time: 1.03 min. ¹H-NMR (CDCl₃, ppm) 1.36 (t, *J*=7.3 Hz, 3H), 3.87 (q, *J*=7.3 Hz, 2H), 4.01 (s, 3H), 7.05 (s, 1H), 7.50 (d, *J*=8.4 Hz, 1H), 7.83 (d, *J*=8.1 Hz, 1H), 8.01 (dd, *J*=8.1, 1.8 Hz, 1H), 8.45 (d, *J*=8.4 Hz, 1H), 8.90 (d, *J*=1.8 Hz, 1H).

### Step 4: Preparation of 3-ethylsulfonyl-2-[1-methyl-5-[6-(trifluoromethyl)-3-pyridyl]pyrazol-3-yl]pyridine (compound P34)

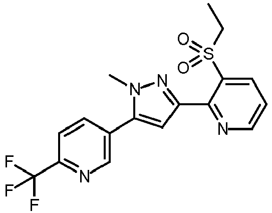

To a solution of 6-chloro-3-ethylsulfonyl-2-[1-methyl-5-[6-(trifluoromethyl)-3-pyridyl]pyrazol-3-yl] pyridine (400 mg, 0.93 mmol) in N,N-dimethylformamide (20 ml) were added triethylamine (0.51 ml, 3.68 mmol), formic acid (0.14 ml, 3.70 mmol), triphenylphosphine (4.87 mg, 0.0186 mmol) and palladium(II) acetate (10.42 mg, 0.0464 mmol). The reaction mixture was stirred at 60°C for 3 hours. More triethylamine (0.26 ml) and formic acid (0.07 ml) were added, and stirring continued at 60°C for another hour. After dilution with dichloromethane, the organic phase was washed water and brine, dried over sodium sulfate and evaporated. The residue was purified by chromatography on silica gel (0 to 80% gradient of ethyl acetate in cyclohexane) to afford 3-ethylsulfonyl-2-[1-methyl-5-[6-(trifluoromethyl)-3-pyridyl]pyrazol-3-yl]pyridine (compound P34) as a solid (200 mg), mp 160-162°C. LCMS (method 4): 397 (M+H)⁺; retention time: 0.90 min. ¹H-NMR (CDCl₃, ppm) 1.35 (t, *J*=7.3 Hz, 3H), 3.80 (q, *J*=7.3 Hz, 2H), 4.02 (s, 3H), 7.03 (s, 1H), 7.52 (dd, *J*=8.1, 4.8 Hz, 1H), 7.83 (d, *J*=8.1 Hz, 1H), 8.02 (dd, *J*=8.1, 1.8 Hz, 1H), 8.53 (dd, *J*=8.1, 1.6 Hz, 1H), 8.89 (dd, *J*=4.8, 1.6 Hz, 1H), 8.91 (d, *J*=1.8 Hz, 1H).

### EXAMPLE P7: Preparation of 3-ethylsulfonyl-2-[1-methyl-5-[6-(trifluoromethyl)-3-pyridyl]pyrazol-3-yl] 6-(1,2,4-triazol-1-yl)pyridine (compound P35)

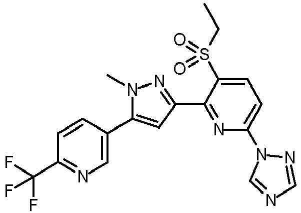

A solution of 6-chloro-3-ethylsulfonyl-2-[1-methyl-5-[6-(trifluoromethyl)-3-pyridyl]pyrazol-3-yl]pyridine (400 mg, 0.93 mmol) and 1H-1,2,4-triazole (96.2 mg, 1.39 mmol) in pyridine (3 ml) unter argon was heated in the microwave at 150°C for 60 minutes. After cooling, the reaction mixture was poured onto iced water, the product extracted with ethyl acetate, the combined organic layers dried over sodium sulfate, filtered and concentrated *in vacuo* to dryness. The residue was purified by chromatography on silica gel (0 to 80% gradient of ethyl acetate in cyclohexane) to afford 3-ethylsulfonyl-2-[1-methyl-5-[6-(trifluoromethyl)-3-pyridyl]pyrazol-3-yl]-6-(1,2,4-triazol-1-yl)pyridine (compound P35) as a solid (145 mg), mp 175-177°C. LCMS (method 4): 464 (M+H)⁺; retention time: 0.94 min. ¹H-NMR (CDCl₃, ppm) 1.40 (t, *J*=7.3 Hz, 3H), 3.92 (q, *J*=7.3 Hz, 2H), 4.05 (s, 3H), 7.06 (s, 1H), 7.87 (d, *J*=8.1 Hz, 1H), 8.05 (d, *J*=8.4 Hz, 1H), 8.05 (dd, *J*=8.1, 1.8 Hz, 1H), 8.16 (s, 1H), 8.70 (d, *J*=8.4 Hz, 1H), 8.93 (d, *J*=1.8 Hz, 1H), 9.27 (s, 1H).

**Table P: Examples of compounds of formula (I)**

| P2, P12, P13, P17, P18, P20, P22, P23, P25 and P27 are examples of compounds according to the present invention; other reference compounds are also provided: | | | | | |
|---|---|---|---|---|---|
| Compound No. | Structures | LCMS | | | Mp (°C) |
| | | Rₜ | [M+H]⁺ | Method | |
| | | (min) | (measured) | | |
| P1 | 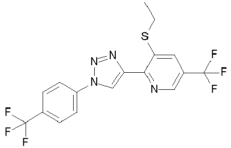 | 4.24 | 419 | 1 | 145-146 |
| P2 | 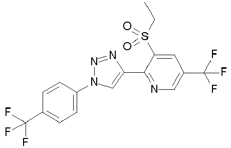 | 3.99 | 451 | 1 | 212-213 |
| P3 | 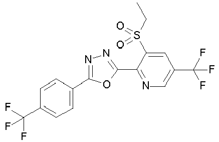 | 1.81 | 452 | 3 | 183-184 |
| P4 | 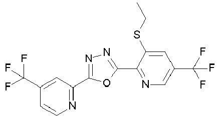 | 1.87 | 421 | 3 | 199-200 |
| P5 | 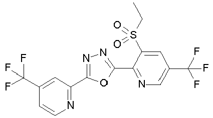 | 1.63 | 453 | 3 | solid |

| Compound No. | Structures | LCMS | | | Mp (°C) |
|---|---|---|---|---|---|
| | | Rₜ | [M+H]⁺ | Method | |
| | | (min) | (measured) | | |
| P6 | | 1.01 | 453 | 4 | 175-177 |
| P7 | | 1.03 | 452 | 4 | 169-171 |
| P8 | | 4.20 | 465 | 5 | 156-157 |
| P9 | | 4.25 | 451 | 5 | 182-184 |
| P10 | 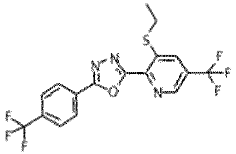 | 2.06 | 420 | 3 | solid |
| P11 | 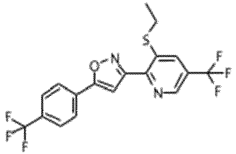 | 4.51 | 419 | 5 | 145-146 |
| P12 | 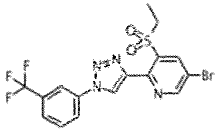 | 1.08 | 461/463 | 4 | 185-187 |
| P13 | 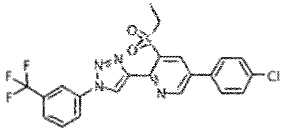 | 1.18 | 493/495 | 4 | 196-198 |
| P14 | 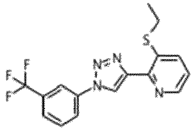 | 1.04 | 351 | 4 | 70-71 |
| P15 | 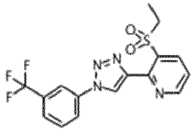 | 0.97 | 383 | 4 | 146-148 |
| P16 | 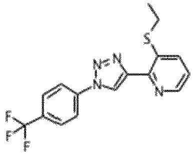 | 1.04 | 351 | 4 | 116-118 |
| P17 | 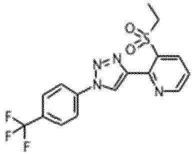 | 0.96 | 383 | 4 | 182-183 |
| P18 | 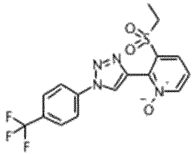 | 0.85 | 399 | 4 | 236-238 |
| P19 | 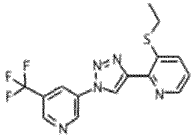 | 0.94 | 352 | 4 | 113-114 |
| P20 | 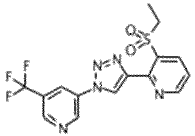 | 0.85 | 384 | 4 | 168-169 |
| P21 | 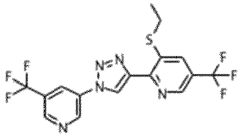 | 1.12 | 420 | 4 | 114-116 |
| P22 | 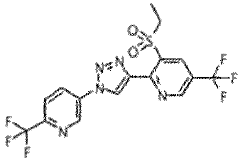 | 0.96 | 452 | 4 | 177-178 |
| P23 | 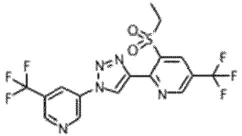 | 1.02 | 452 | 4 | 119-120 |
| P24 | 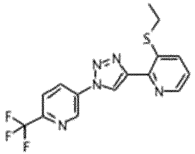 | 0.96 | 352 | 4 | 140-141 |
| P25 | 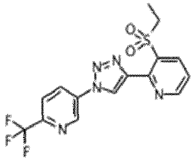 | 0.88 | 384 | 4 | 188-189 |
| P26 | | 1.04 | 352 | 4 | solid |
| P27 | | 0.94 | 384 | 4 | solid |
| P28 | | 2.05 | 399/401 | 3 | 138-140 |
| P29 | | 1.10 | 431/433 | 4 | 255-257 |
| P30 | 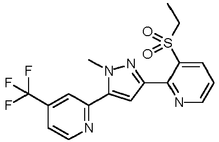 | 0.96 | 397 | 4 | 171-173 |
| P31 | 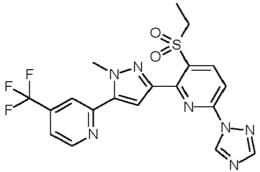 | 1.01 | 464 | 4 | 238-240 |
| P32 | 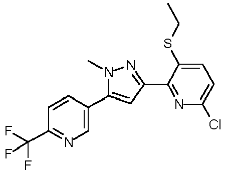 | 1.89 | 399/401 | 3 | 136-138 |
| P33 | 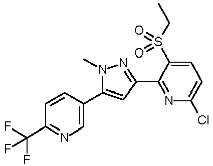 | 1.03 | 431/433 | 4 | 198-200 |
| P34 | 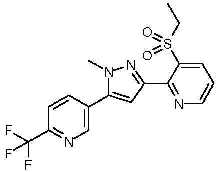 | 0.90 | 397 | 4 | 160-162 |
| P35 | 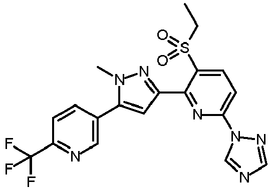 | 0.94 | 464 | 4 | 175-177 |
| P36 | 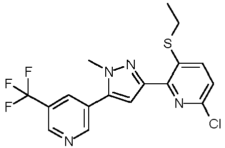 | 1.86 | 399/401 | 3 | 125-126 |
| P37 | 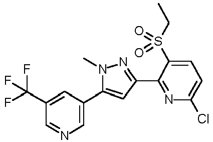 | 1.01 | 431/433 | 4 | 152-153 |
| P38 | 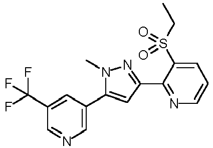 | 0.89 | 397 | 4 | solid |
| P39 | 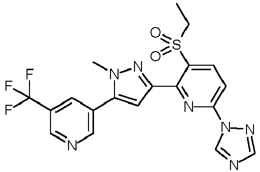 | 0.93 | 464 | 4 | 235-237 |

**Table W: Examples of intermediate compounds of formula (XXXb)**

| Compound No. | Structures | LCMS | | | Mp (°C) |
|---|---|---|---|---|---|
| | | Rₜ | [M+H]⁺ | Method | |
| | | (min) | (measured) | | |
| W1 | 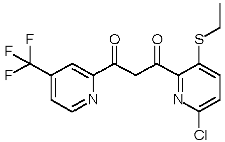 | 1.31 | 389/391 | 4 | 150-152 |
| W2 | 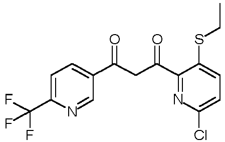 | 1.25 | 389/391 | 4 | 123-125 |
| W3 | 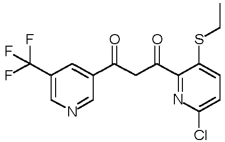 | 1.24 | 389/391 | 4 | 138-139 |

The activity of the compositions according to the invention can be broadened considerably, and adapted to prevailing circumstances, by adding other insecticidally, acaricidally and/or fungicidally active ingredients. The mixtures of the compounds of formula I with other insecticidally, acaricidally and/or fungicidally active ingredients may also have further surprising advantages which can also be described, in a wider sense, as synergistic activity. For example, better tolerance by plants, reduced phytotoxicity, insects can be controlled in their different development stages or better behaviour during their production, for example during grinding or mixing, during their storage or during their use. Suitable additions to active ingredients here are, for example, representatives of the following classes of active ingredients: organophosphorus compounds, nitrophenol derivatives, thioureas, juvenile hormones, formamidines, benzophenone derivatives, ureas, pyrrole derivatives, carbamates, pyrethroids, chlorinated hydrocarbons, acylureas, pyridylmethyleneamino derivatives, macrolides, neonicotinoids and Bacillus thuringiensis preparations.

The following mixtures of the compounds of formula I with active ingredients are preferred (the abbreviation "TX" means "one compound selected from the group consisting of the compounds described in Tables 1 to 27 and Table P"):
an adjuvant selected from the group of substances consisting of petroleum oils (628) + TX, an acaricide selected from the group of substances consisting of 1,1-bis(4-chlorophenyl)-2-ethoxyethanol (IUPAC name) (910) + TX, 2,4-dichlorophenyl benzenesulfonate (IUPAC/Chemical Abstracts name) (1059) + TX, 2-fluoro-*N*-methyl-*N*-1-naphthylacetamide (IUPAC name) (1295) + TX, 4-chlorophenyl phenyl sulfone (IUPAC name) (981) + TX, abamectin (1) + TX, acequinocyl (3) + TX, acetoprole [CCN] + TX, acrinathrin (9) + TX, aldicarb (16) + TX, aldoxycarb (863) + TX, alpha-cypermethrin (202) + TX, amidithion (870) + TX, amidoflumet [CCN] + TX, amidothioate (872) + TX, amiton (875) + TX, amiton hydrogen oxalate (875) + TX, amitraz (24) + TX, aramite (881) + TX, arsenous oxide (882) + TX, AVI 382 (compound code) + TX, AZ 60541 (compound code) + TX, azinphos-ethyl (44) + TX, azinphos-methyl (45) + TX, azobenzene (IUPAC name) (888) + TX, azocyclotin (46) + TX, azothoate (889) + TX, benomyl (62) + TX, benoxafos [CCN] + TX, benzoximate (71) + TX, benzyl benzoate (IUPAC name) [CCN] + TX, bifenazate (74) + TX, bifenthrin (76) + TX, binapacryl (907) + TX, brofenvalerate + TX, bromocyclen (918) + TX, bromophos (920) + TX, bromophos-ethyl (921) + TX, bromopropylate (94) + TX, buprofezin (99) + TX, butocarboxim (103) + TX, butoxycarboxim (104) + TX, butylpyridaben + TX, calcium polysulfide (IUPAC name) (111) + TX, camphechlor (941) + TX, carbanolate (943) + TX, carbaryl (115) + TX, carbofuran (118) + TX, carbophenothion (947) + TX, CGA 50'439 (development code) (125) + TX, chinomethionat (126) + TX, chlorbenside (959) + TX, chlordimeform (964) + TX, chlordimeform hydrochloride (964) + TX, chlorfenapyr (130) + TX, chlorfenethol (968) + TX, chlorfenson (970) + TX, chlorfensulfide (971) + TX, chlorfenvinphos (131) + TX, chlorobenzilate (975) + TX, chloromebuform (977) + TX, chloromethiuron (978) + TX, chloropropylate (983) + TX, chlorpyrifos (145) + TX, chlorpyrifos-methyl (146) + TX, chlorthiophos (994) + TX, cinerin I (696) + TX, cinerin II (696) + TX, cinerins (696) + TX, clofentezine (158) + TX, closantel [CCN] + TX, coumaphos (174) + TX, crotamiton [CCN] + TX, crotoxyphos (1010) + TX, cufraneb (1013) + TX, cyanthoate (1020) + TX, cyflumetofen (CAS Reg. No.: 400882-07-7) + TX, cyhalothrin (196) + TX, cyhexatin (199) + TX, cypermethrin (201) + TX, DCPM (1032) + TX, DDT (219) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulfon (1039) + TX, diafenthiuron (226) + TX, dialifos (1042) + TX, diazinon (227) + TX, dichlofluanid (230) + TX, dichlorvos (236) + TX, dicliphos + TX, dicofol (242) + TX, dicrotophos (243) + TX, dienochlor (1071) + TX, dimefox (1081) + TX, dimethoate (262) + TX, dinactin (653) + TX, dinex (1089) + TX, dinex-diclexine (1089) + TX, dinobuton (269) + TX, dinocap (270) + TX, dinocap-4 [CCN] + TX, dinocap-6 [CCN] + TX, dinocton (1090) + TX, dinopenton (1092) + TX, dinosulfon (1097) + TX, dinoterbon (1098) + TX, dioxathion (1102) + TX, diphenyl sulfone (IUPAC name) (1103) + TX, disulfiram [CCN] + TX, disulfoton (278) + TX, DNOC (282) + TX, dofenapyn (1113) + TX, doramectin [CCN] + TX, endosulfan (294) + TX, endothion (1121) + TX, EPN (297) + TX, eprinomectin [CCN] + TX, ethion (309) + TX, ethoate-methyl (1134) + TX, etoxazole (320) + TX, etrimfos (1142) + TX, fenazaflor (1147) + TX, fenazaquin (328) + TX, fenbutatin oxide (330) + TX, fenothiocarb (337) + TX, fenpropathrin (342) + TX, fenpyrad + TX, fenpyroximate (345) + TX, fenson (1157) + TX, fentrifanil (1161) + TX, fenvalerate (349) + TX, fipronil (354) + TX, fluacrypyrim (360) + TX, fluazuron (1166) + TX, flubenzimine (1167) + TX, flucycloxuron (366) + TX, flucythrinate (367) + TX, fluenetil (1169) + TX, flufenoxuron (370) + TX, flumethrin (372) + TX, fluorbenside (1174) + TX, fluvalinate (1184) + TX, FMC 1137 (development code) (1185) + TX, formetanate (405) + TX, formetanate hydrochloride (405) + TX, formothion (1192) + TX, formparanate (1193) + TX, gamma-HCH (430) + TX, glyodin (1205) + TX, halfenprox (424) + TX, heptenophos (432) + TX, hexadecyl cyclopropanecarboxylate (IUPAC/Chemical Abstracts name) (1216) + TX, hexythiazox (441) + TX, iodomethane (IUPAC name) (542) + TX, isocarbophos (473) + TX, isopropyl 0-(methoxyaminothiophosphoryl)salicylate (IUPAC name) (473) + TX, ivermectin [CCN] + TX, jasmolin I (696) + TX, jasmolin II (696) + TX, jodfenphos (1248) + TX, lindane (430) + TX, lufenuron (490) + TX, malathion (492) + TX, malonoben (1254) + TX, mecarbam (502) + TX, mephosfolan (1261) + TX, mesulfen [CCN] + TX, methacrifos (1266) + TX, methamidophos (527) + TX, methidathion (529) + TX, methiocarb (530) + TX, methomyl (531) + TX, methyl bromide (537) + TX, metolcarb (550) + TX, mevinphos (556) + TX, mexacarbate (1290) + TX, milbemectin (557) + TX, milbemycin oxime [CCN] + TX, mipafox (1293) + TX, monocrotophos (561) + TX, morphothion (1300) + TX, moxidectin [CCN] + TX, naled (567) + TX, NC-184 (compound code) + TX, NC-512 (compound code) + TX, nifluridide (1309) + TX, nikkomycins [CCN] + TX, nitrilacarb (1313) + TX, nitrilacarb 1:1 zinc chloride complex (1313) + TX, NNI-0101 (compound code) + TX, NNI-0250 (compound code) + TX, omethoate (594) + TX, oxamyl (602) + TX, oxydeprofos (1324) + TX, oxydisulfoton (1325) + TX, pp'-DDT (219) + TX, parathion (615) + TX, permethrin (626) + TX, petroleum oils (628) + TX, phenkapton (1330) + TX, phenthoate (631) + TX, phorate (636) + TX, phosalone (637) + TX, phosfolan (1338) + TX, phosmet (638) + TX, phosphamidon (639) + TX, phoxim (642) + TX, pirimiphos-methyl (652) + TX, polychloroterpenes (traditional name) (1347) + TX, polynactins (653) + TX, proclonol (1350) + TX, profenofos (662) + TX, promacyl (1354) + TX, propargite (671) + TX, propetamphos (673) + TX, propoxur (678) + TX, prothidathion (1360) + TX, prothoate (1362) + TX, pyrethrin I (696) + TX, pyrethrin II (696) + TX, pyrethrins (696) + TX, pyridaben (699) + TX, pyridaphenthion (701) + TX, pyrimidifen (706) + TX, pyrimitate (1370) + TX, quinalphos (711) + TX, quintiofos (1381) + TX, R-1492 (development code) (1382) + TX, RA-17 (development code) (1383) + TX, rotenone (722) + TX, schradan (1389) + TX, sebufos + TX, selamectin [CCN] + TX, SI-0009 (compound code) + TX, sophamide (1402) + TX, spirodiclofen (738) + TX, spiromesifen (739) + TX, SSI-121 (development code) (1404) + TX, sulfiram [CCN] + TX, sulfluramid (750) + TX, sulfotep (753) + TX, sulfur (754) + TX, SZI-121 (development code) (757) + TX, tau-fluvalinate (398) + TX, tebufenpyrad (763) + TX, TEPP (1417) + TX, terbam + TX, tetrachlorvinphos (777) + TX, tetradifon (786) + TX, tetranactin (653) + TX, tetrasul (1425) + TX, thiafenox + TX, thiocarboxime (1431) + TX, thiofanox (800) + TX, thiometon (801) + TX, thioquinox (1436) + TX, thuringiensin [CCN] + TX, triamiphos (1441) + TX, triarathene (1443) + TX, triazophos (820) + TX, triazuron + TX, trichlorfon (824) + TX, trifenofos (1455) + TX, trinactin (653) + TX, vamidothion (847) + TX, vaniliprole [CCN] and YI-5302 (compound code) + TX,
an algicide selected from the group of substances consisting of bethoxazin [CCN] + TX, copper dioctanoate (IUPAC name) (170) + TX, copper sulfate (172) + TX, cybutryne [CCN] + TX, dichlone (1052) + TX, dichlorophen (232) + TX, endothal (295) + TX, fentin (347) + TX, hydrated lime [CCN] + TX, nabam (566) + TX, quinoclamine (714) + TX, quinonamid (1379) + TX, simazine (730) + TX, triphenyltin acetate (IUPAC name) (347) and triphenyltin hydroxide (IUPAC name) (347) + TX,
an anthelmintic selected from the group of substances consisting of abamectin (1) + TX, crufomate (1011) + TX, doramectin [CCN] + TX, emamectin (291) + TX, emamectin benzoate (291) + TX, eprinomectin [CCN] + TX, ivermectin [CCN] + TX, milbemycin oxime [CCN] + TX, moxidectin [CCN] + TX, piperazine [CCN] + TX, selamectin [CCN] + TX, spinosad (737) and thiophanate (1435) + TX,
an avicide selected from the group of substances consisting of chloralose (127) + TX, endrin (1122) + TX, fenthion (346) + TX, pyridin-4-amine (IUPAC name) (23) and strychnine (745) + TX,
a bactericide selected from the group of substances consisting of 1-hydroxy-1*H*-pyridine-2-thione (IUPAC name) (1222) + TX, 4-(quinoxalin-2-ylamino)benzenesulfonamide (IUPAC name) (748) + TX, 8-hydroxyquinoline sulfate (446) + TX, bronopol (97) + TX, copper dioctanoate (IUPAC name) (170) + TX, copper hydroxide (IUPAC name) (169) + TX, cresol [CCN] + TX, dichlorophen (232) + TX, dipyrithione (1105) + TX, dodicin (1112) + TX, fenaminosulf (1144) + TX, formaldehyde (404) + TX, hydrargaphen [CCN] + TX, kasugamycin (483) + TX, kasugamycin hydrochloride hydrate (483) + TX, nickel bis(dimethyldithiocarbamate) (IUPAC name) (1308) + TX, nitrapyrin (580) + TX, octhilinone (590) + TX, oxolinic acid (606) + TX, oxytetracycline (611) + TX, potassium hydroxyquinoline sulfate (446) + TX, probenazole (658) + TX, streptomycin (744) + TX, streptomycin sesquisulfate (744) + TX, tecloftalam (766) + TX, and thiomersal [CCN] + TX, a biological agent selected from the group of substances consisting of *Adoxophyes orana* GV (12) + TX, *Agrobacterium radiobacter* (13) + TX, *Amblyseius* spp. (19) + TX, *Anagrapha falcifera* NPV (28) + TX, *Anagrus atomus* (29) + TX, *Aphelinus abdominalis* (33) + TX, *Aphidius colemani* (34) + TX, *Aphidoletes aphidimyza* (35) + TX, *Autographa californica* NPV (38) + TX, *Bacillus firmus* (48) + TX, *Bacillus sphaericus* Neide (scientific name) (49) + TX, *Bacillus thuringiensis* Berliner (scientific name) (51) + TX, *Bacillus thuringiensis* subsp. *aizawai* (scientific name) (51) + TX, *Bacillus thuringiensis* subsp. *israelensis* (scientific name) (51) + TX, *Bacillus thuringiensis* subsp. *japonensis* (scientific name) (51) + TX, *Bacillus thuringiensis* subsp. *kurstaki* (scientific name) (51) + TX, *Bacillus thuringiensis* subsp. *tenebrionis* (scientific name) (51) + TX, *Beauveria bassiana* (53) + TX, *Beauveria brongniartii* (54) + TX, *Chrysoperla carnea* (151) + TX, *Cryptolaemus montrouzieri* (178) + TX, *Cydia pomonella* GV (191) + TX, *Dacnusa sibirica* (212) + TX, *Diglyphus isaea* (254) + TX, *Encarsia formosa* (scientific name) (293) + TX, *Eretmocerus eremicus* (300) + TX, *Helicoverpa* zea NPV (431) + TX, *Heterorhabditis bacteriophora* and *H. megidis* (433) + TX, *Hippodamia convergens* (442) + TX, *Leptomastix dactylopii* (488) + TX, *Macrolophus caliginosus* (491) + TX, *Mamestra brassicae* NPV (494) + TX, *Metaphycus helvolus* (522) + TX, *Metarhizium anisopliae* var. *acridum* (scientific name) (523) + TX, *Metarhizium anisopliae* var. *anisopliae* (scientific name) (523) + TX, *Neodiprion sertifer* NPV and *N. lecontei* NPV (575) + TX, *Orius* spp. (596) + TX, *Paecilomyces fumosoroseus* (613) + TX, *Phytoseiulus persimilis* (644) + TX, *Spodoptera exigua* multicapsid nuclear polyhedrosis virus (scientific name) (741) + TX, *Steinernema bibionis* (742) + TX, *Steinernema carpocapsae* (742) + TX, *Steinernema feltiae* (742) + TX, *Steinernema glaseri* (742) + TX, *Steinernema riobrave* (742) + TX, *Steinernema riobravis* (742) + TX, *Steinernema scapterisci* (742) + TX, *Steinernema* spp. (742) + TX, *Trichogramma* spp. (826) + TX, *Typhlodromus occidentalis* (844) and *Verticillium lecanii* (848) + TX,
a soil sterilant selected from the group of substances consisting of iodomethane (IUPAC name) (542) and methyl bromide (537) + TX,
a chemosterilant selected from the group of substances consisting of apholate [CCN] + TX, bisazir [CCN] + TX, busulfan [CCN] + TX, diflubenzuron (250) + TX, dimatif [CCN] + TX, hemel [CCN] + TX, hempa [CCN] + TX, metepa [CCN] + TX, methiotepa [CCN] + TX, methyl apholate [CCN] + TX, morzid [CCN] + TX, penfluron [CCN] + TX, tepa [CCN] + TX, thiohempa [CCN] + TX, thiotepa [CCN] + TX, tretamine [CCN] and uredepa [CCN] + TX,
an insect pheromone selected from the group of substances consisting of (*E*)-dec-5-en-1-yl acetate with (*E*)-dec-5-en-1-ol (IUPAC name) (222) + TX, (*E*)-tridec-4-en-1-yl acetate (IUPAC name) (829) + TX, (*E*)-6-methylhept-2-en-4-ol (IUPAC name) (541) + TX, (E,Z)-tetradeca-4,10-dien-1-yl acetate (IUPAC name) (779) + TX, (Z)-dodec-7-en-1-yl acetate (IUPAC name) (285) + TX, (*Z*)-hexadec-11-enal (IUPAC name) (436) + TX, (*Z*)-hexadec-11-en-1-yl acetate (IUPAC name) (437) + TX, (Z)-hexadec-13-en-11-yn-1-yl acetate (IUPAC name) (438) + TX, (Z)-icos-13-en-10-one (IUPAC name) (448) + TX, (Z)-tetradec-7-en-1-al (IUPAC name) (782) + TX, (*Z*)-tetradec-9-en-1-ol (IUPAC name) (783) + TX, (Z)-tetradec-9-en-1-yl acetate (IUPAC name) (784) + TX, (7*E*,9Z)-dodeca-7,9-dien-1-yl acetate (IUPAC name) (283) + TX, (9Z,11*E*)-tetradeca-9,11-dien-1-yl acetate (IUPAC name) (780) + TX, (9*Z*,12*E*)-tetradeca-9,12-dien-1-yl acetate (IUPAC name) (781) + TX, 14-methyloctadec-1-ene (IUPAC name) (545) + TX, 4-methylnonan-5-ol with 4-methylnonan-5-one (IUPAC name) (544) + TX, alpha-multistriatin [CCN] + TX, brevicomin [CCN] + TX, codlelure [CCN] + TX, codlemone (167) + TX, cuelure (179) + TX, disparlure (277) + TX, dodec-8-en-1-yl acetate (IUPAC name) (286) + TX, dodec-9-en-1-yl acetate (IUPAC name) (287) + TX, dodeca-8 + TX, 10-dien-1-yl acetate (IUPAC name) (284) + TX, dominicalure [CCN] + TX, ethyl 4-methyloctanoate (IUPAC name) (317) + TX, eugenol [CCN] + TX, frontalin [CCN] + TX, gossyplure (420) + TX, grandlure (421) + TX, grandlure I (421) + TX, grandlure II (421) + TX, grandlure III (421) + TX, grandlure IV (421) + TX, hexalure [CCN] + TX, ipsdienol [CCN] + TX, ipsenol [CCN] + TX, japonilure (481) + TX, lineatin [CCN] + TX, litlure [CCN] + TX, looplure [CCN] + TX, medlure [CCN] + TX, megatomoic acid [CCN] + TX, methyl eugenol (540) + TX, muscalure (563) + TX, octadeca-2,13-dien-1-yl acetate (IUPAC name) (588) + TX, octadeca-3,13-dien-1-yl acetate (IUPAC name) (589) + TX, orfralure [CCN] + TX, oryctalure (317) + TX, ostramone [CCN] + TX, siglure [CCN] + TX, sordidin (736) + TX, sulcatol [CCN] + TX, tetradec-11-en-1-yl acetate (IUPAC name) (785) + TX, trimedlure (839) + TX, trimedlure A (839) + TX, trimedlure B₁ (839) + TX, trimedlure B₂ (839) + TX, trimedlure C (839) and trunc-call [CCN] + TX,
an insect repellent selected from the group of substances consisting of 2-(octylthio)ethanol (IUPAC name) (591) + TX, butopyronoxyl (933) + TX, butoxy(polypropylene glycol) (936) + TX, dibutyl adipate (IUPAC name) (1046) + TX, dibutyl phthalate (1047) + TX, dibutyl succinate (IUPAC name) (1048) + TX, diethyltoluamide [CCN] + TX, dimethyl carbate [CCN] + TX, dimethyl phthalate [CCN] + TX, ethyl hexanediol (1137) + TX, hexamide [CCN] + TX, methoquin-butyl (1276) + TX, methylneodecanamide [CCN] + TX, oxamate [CCN] and picaridin [CCN] + TX,
an insecticide selected from the group of substances consisting of 1-dichloro-1-nitroethane (IUPAC/Chemical Abstracts name) (1058) + TX, 1,1-dichloro-2,2-bis(4-ethylphenyl)ethane (IUPAC name) (1056), + TX, 1,2-dichloropropane (IUPAC/Chemical Abstracts name) (1062) + TX, 1,2-dichloropropane with 1,3-dichloropropene (IUPAC name) (1063) + TX, 1-bromo-2-chloroethane (IUPAC/Chemical Abstracts name) (916) + TX, 2,2,2-trichloro-1-(3,4-dichlorophenyl)ethyl acetate (IUPAC name) (1451) + TX, 2,2-dichlorovinyl 2-ethylsulfinylethyl methyl phosphate (IUPAC name) (1066) + TX, 2-(1,3-dithiolan-2-yl)phenyl dimethylcarbamate (IUPAC/ Chemical Abstracts name) (1109) + TX, 2-(2-butoxyethoxy)ethyl thiocyanate (IUPAC/Chemical Abstracts name) (935) + TX, 2-(4,5-dimethyl-1,3-dioxolan-2-yl)phenyl methylcarbamate (IUPAC/ Chemical Abstracts name) (1084) + TX, 2-(4-chloro-3,5-xylyloxy)ethanol (IUPAC name) (986) + TX, 2-chlorovinyl diethyl phosphate (IUPAC name) (984) + TX, 2-imidazolidone (IUPAC name) (1225) + TX, 2-isovalerylindan-1,3-dione (IUPAC name) (1246) + TX, 2-methyl(prop-2-ynyl)aminophenyl methylcarbamate (IUPAC name) (1284) + TX, 2-thiocyanatoethyl laurate (IUPAC name) (1433) + TX, 3-bromo-1-chloroprop-1-ene (IUPAC name) (917) + TX, 3-methyl-1-phenylpyrazol-5-yl dimethylcarbamate (IUPAC name) (1283) + TX, 4-methyl(prop-2-ynyl)amino-3,5-xylyl methylcarbamate (IUPAC name) (1285) + TX, 5,5-dimethyl-3-oxocyclohex-1-enyl dimethylcarbamate (IUPAC name) (1085) + TX, abamectin (1) + TX, acephate (2) + TX, acetamiprid (4) + TX, acethion [CCN] + TX, acetoprole [CCN] + TX, acrinathrin (9) + TX, acrylonitrile (IUPAC name) (861) + TX, alanycarb (15) + TX, aldicarb (16) + TX, aldoxycarb (863) + TX, aldrin (864) + TX, allethrin (17) + TX, allosamidin [CCN] + TX, allyxycarb (866) + TX, alpha-cypermethrin (202) + TX, alpha-ecdysone [CCN] + TX, aluminium phosphide (640) + TX, amidithion (870) + TX, amidothioate (872) + TX, aminocarb (873) + TX, amiton (875) + TX, amiton hydrogen oxalate (875) + TX, amitraz (24) + TX, anabasine (877) + TX, athidathion (883) + TX, AVI 382 (compound code) + TX, AZ 60541 (compound code) + TX, azadirachtin (41) + TX, azamethiphos (42) + TX, azinphos-ethyl (44) + TX, azinphos-methyl (45) + TX, azothoate (889) + TX, *Bacillus thuringiensis* delta endotoxins (52) + TX, barium hexafluorosilicate [CCN] + TX, barium polysulfide (IUPAC/Chemical Abstracts name) (892) + TX, barthrin [CCN] + TX, Bayer 22/190 (development code) (893) + TX, Bayer 22408 (development code) (894) + TX, bendiocarb (58) + TX, benfuracarb (60) + TX, bensultap (66) + TX, beta-cyfluthrin (194) + TX, beta-cypermethrin (203) + TX, bifenthrin (76) + TX, bioallethrin (78) + TX, bioallethrin S-cyclopentenyl isomer (79) + TX, bioethanomethrin [CCN] + TX, biopermethrin (908) + TX, bioresmethrin (80) + TX, bis(2-chloroethyl) ether (IUPAC name) (909) + TX, bistrifluron (83) + TX, borax (86) + TX, brofenvalerate + TX, bromfenvinfos (914) + TX, bromocyclen (918) + TX, bromo-DDT [CCN] + TX, bromophos (920) + TX, bromophos-ethyl (921) + TX, bufencarb (924) + TX, buprofezin (99) + TX, butacarb (926) + TX, butathiofos (927) + TX, butocarboxim (103) + TX, butonate (932) + TX, butoxycarboxim (104) + TX, butylpyridaben + TX, cadusafos (109) + TX, calcium arsenate [CCN] + TX, calcium cyanide (444) + TX, calcium polysulfide (IUPAC name) (111) + TX, camphechlor (941) + TX, carbanolate (943) + TX, carbaryl (115) + TX, carbofuran (118) + TX, carbon disulfide (IUPAC/Chemical Abstracts name) (945) + TX, carbon tetrachloride (IUPAC name) (946) + TX, carbophenothion (947) + TX, carbosulfan (119) + TX, cartap (123) + TX, cartap hydrochloride (123) + TX, cevadine (725) + TX, chlorbicyclen (960) + TX, chlordane (128) + TX, chlordecone (963) + TX, chlordimeform (964) + TX, chlordimeform hydrochloride (964) + TX, chlorethoxyfos (129) + TX, chlorfenapyr (130) + TX, chlorfenvinphos (131) + TX, chlorfluazuron (132) + TX, chlormephos (136) + TX, chloroform [CCN] + TX, chloropicrin (141) + TX, chlorphoxim (989) + TX, chlorprazophos (990) + TX, chlorpyrifos (145) + TX, chlorpyrifos-methyl (146) + TX, chlorthiophos (994) + TX, chromafenozide (150) + TX, cinerin I (696) + TX, cinerin II (696) + TX, cinerins (696) + TX, cis-resmethrin + TX, cismethrin (80) + TX, clocythrin + TX, cloethocarb (999) + TX, closantel [CCN] + TX, clothianidin (165) + TX, copper acetoarsenite [CCN] + TX, copper arsenate [CCN] + TX, copper oleate [CCN] + TX, coumaphos (174) + TX, coumithoate (1006) + TX, crotamiton [CCN] + TX, crotoxyphos (1010) + TX, crufomate (1011) + TX, cryolite (177) + TX, CS 708 (development code) (1012) + TX, cyanofenphos (1019) + TX, cyanophos (184) + TX, cyanthoate (1020) + TX, cyclethrin [CCN] + TX, cycloprothrin (188) + TX, cyfluthrin (193) + TX, cyhalothrin (196) + TX, cypermethrin (201) + TX, cyphenothrin (206) + TX, cyromazine (209) + TX, cythioate [CCN] + TX, *d*-limonene [CCN] + TX, *d*-tetramethrin (788) + TX, DAEP (1031) + TX, dazomet (216) + TX, DDT (219) + TX, decarbofuran (1034) + TX, deltamethrin (223) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulphon (1039) + TX, diafenthiuron (226) + TX, dialifos (1042) + TX, diamidafos (1044) + TX, diazinon (227) + TX, dicapthon (1050) + TX, dichlofenthion (1051) + TX, dichlorvos (236) + TX, dicliphos + TX, dicresyl [CCN] + TX, dicrotophos (243) + TX, dicyclanil (244) + TX, dieldrin (1070) + TX, diethyl 5-methylpyrazol-3-yl phosphate (IUPAC name) (1076) + TX, diflubenzuron (250) + TX, dilor [CCN] + TX, dimefluthrin [CCN] + TX, dimefox (1081) + TX, dimetan (1085) + TX, dimethoate (262) + TX, dimethrin (1083) + TX, dimethylvinphos (265) + TX, dimetilan (1086) + TX, dinex (1089) + TX, dinex-diclexine (1089) + TX, dinoprop (1093) + TX, dinosam (1094) + TX, dinoseb (1095) + TX, dinotefuran (271) + TX, diofenolan (1099) + TX, dioxabenzofos (1100) + TX, dioxacarb (1101) + TX, dioxathion (1102) + TX, disulfoton (278) + TX, dithicrofos (1108) + TX, DNOC (282) + TX, doramectin [CCN] + TX, DSP (1115) + TX, ecdysterone [CCN] + TX, EI 1642 (development code) (1118) + TX, emamectin (291) + TX, emamectin benzoate (291) + TX, EMPC (1120) + TX, empenthrin (292) + TX, endosulfan (294) + TX, endothion (1121) + TX, endrin (1122) + TX, EPBP (1123) + TX, EPN (297) + TX, epofenonane (1124) + TX, eprinomectin [CCN] + TX, esfenvalerate (302) + TX, etaphos [CCN] + TX, ethiofencarb (308) + TX, ethion (309) + TX, ethiprole (310) + TX, ethoate-methyl (1134) + TX, ethoprophos (312) + TX, ethyl formate (IUPAC name) [CCN] + TX, ethyl-DDD (1056) + TX, ethylene dibromide (316) + TX, ethylene dichloride (chemical name) (1136) + TX, ethylene oxide [CCN] + TX, etofenprox (319) + TX, etrimfos (1142) + TX, EXD (1143) + TX, famphur (323) + TX, fenamiphos (326) + TX, fenazaflor (1147) + TX, fenchlorphos (1148) + TX, fenethacarb (1149) + TX, fenfluthrin (1150) + TX, fenitrothion (335) + TX, fenobucarb (336) + TX, fenoxacrim (1153) + TX, fenoxycarb (340) + TX, fenpirithrin (1155) + TX, fenpropathrin (342) + TX, fenpyrad + TX, fensulfothion (1158) + TX, fenthion (346) + TX, fenthion-ethyl [CCN] + TX, fenvalerate (349) + TX, fipronil (354) + TX, flonicamid (358) + TX, flubendiamide (CAS. Reg. No.: 272451-65-7) + TX, flucofuron (1168) + TX, flucycloxuron (366) + TX, flucythrinate (367) + TX, fluenetil (1169) + TX, flufenerim [CCN] + TX, flufenoxuron (370) + TX, flufenprox (1171) + TX, flumethrin (372) + TX, fluvalinate (1184) + TX, FMC 1137 (development code) (1185) + TX, fonofos (1191) + TX, formetanate (405) + TX, formetanate hydrochloride (405) + TX, formothion (1192) + TX, formparanate (1193) + TX, fosmethilan (1194) + TX, fospirate (1195) + TX, fosthiazate (408) + TX, fosthietan (1196) + TX, furathiocarb (412) + TX, furethrin (1200) + TX, gamma-cyhalothrin (197) + TX, gamma-HCH (430) + TX, guazatine (422) + TX, guazatine acetates (422) + TX, GY-81 (development code) (423) + TX, halfenprox (424) + TX, halofenozide (425) + TX, HCH (430) + TX, HEOD (1070) + TX, heptachlor (1211) + TX, heptenophos (432) + TX, heterophos [CCN] + TX, hexaflumuron (439) + TX, HHDN (864) + TX, hydramethylnon (443) + TX, hydrogen cyanide (444) + TX, hydroprene (445) + TX, hyquincarb (1223) + TX, imidacloprid (458) + TX, imiprothrin (460) + TX, indoxacarb (465) + TX, iodomethane (IUPAC name) (542) + TX, IPSP (1229) + TX, isazofos (1231) + TX, isobenzan (1232) + TX, isocarbophos (473) + TX, isodrin (1235) + TX, isofenphos (1236) + TX, isolane (1237) + TX, isoprocarb (472) + TX, isopropyl *O*-(methoxyaminothiophosphoryl)salicylate (IUPAC name) (473) + TX, isoprothiolane (474) + TX, isothioate (1244) + TX, isoxathion (480) + TX, ivermectin [CCN] + TX, jasmolin I (696) + TX, jasmolin II (696) + TX, jodfenphos (1248) + TX, juvenile hormone I [CCN] + TX, juvenile hormone II [CCN] + TX, juvenile hormone III [CCN] + TX, kelevan (1249) + TX, kinoprene (484) + TX, lambda-cyhalothrin (198) + TX, lead arsenate [CCN] + TX, lepimectin (CCN) + TX, leptophos (1250) + TX, lindane (430) + TX, lirimfos (1251) + TX, lufenuron (490) + TX, lythidathion (1253) + TX, m-cumenyl methylcarbamate (IUPAC name) (1014) + TX, magnesium phosphide (IUPAC name) (640) + TX, malathion (492) + TX, malonoben (1254) + TX, mazidox (1255) + TX, mecarbam (502) + TX, mecarphon (1258) + TX, menazon (1260) + TX, mephosfolan (1261) + TX, mercurous chloride (513) + TX, mesulfenfos (1263) + TX, metaflumizone (CCN) + TX, metam (519) + TX, metam-potassium (519) + TX, metam-sodium (519) + TX, methacrifos (1266) + TX, methamidophos (527) + TX, methanesulfonyl fluoride (IUPAC/Chemical Abstracts name) (1268) + TX, methidathion (529) + TX, methiocarb (530) + TX, methocrotophos (1273) + TX, methomyl (531) + TX, methoprene (532) + TX, methoquin-butyl (1276) + TX, methothrin (533) + TX, methoxychlor (534) + TX, methoxyfenozide (535) + TX, methyl bromide (537) + TX, methyl isothiocyanate (543) + TX, methylchloroform [CCN] + TX, methylene chloride [CCN] + TX, metofluthrin [CCN] + TX, metolcarb (550) + TX, metoxadiazone (1288) + TX, mevinphos (556) + TX, mexacarbate (1290) + TX, milbemectin (557) + TX, milbemycin oxime [CCN] + TX, mipafox (1293) + TX, mirex (1294) + TX, monocrotophos (561) + TX, morphothion (1300) + TX, moxidectin [CCN] + TX, naftalofos [CCN] + TX, naled (567) + TX, naphthalene (IUPAC/Chemical Abstracts name) (1303) + TX, NC-170 (development code) (1306) + TX, NC-184 (compound code) + TX, nicotine (578) + TX, nicotine sulfate (578) + TX, nifluridide (1309) + TX, nitenpyram (579) + TX, nithiazine (1311) + TX, nitrilacarb (1313) + TX, nitrilacarb 1:1 zinc chloride complex (1313) + TX, NNI-0101 (compound code) + TX, NNI-0250 (compound code) + TX, nornicotine (traditional name) (1319) + TX, novaluron (585) + TX, noviflumuron (586) + TX, *O*-5-dichloro-4-iodophenyl *O*-ethyl ethylphosphonothioate (IUPAC name) (1057) + TX, *O,O-*diethyl *O*-4-methyl-2-oxo-2*H*-chromen-7-yl phosphorothioate (IUPAC name) (1074) + TX, *O,O*-diethyl *O*-6-methyl-2-propylpyrimidin-4-yl phosphorothioate (IUPAC name) (1075) + TX, *O,O,O*'*,O*'-tetrapropyl dithiopyrophosphate (IUPAC name) (1424) + TX, oleic acid (IUPAC name) (593) + TX, omethoate (594) + TX, oxamyl (602) + TX, oxydemeton-methyl (609) + TX, oxydeprofos (1324) + TX, oxydisulfoton (1325) + TX, pp'-DDT (219) + TX, para-dichlorobenzene [CCN] + TX, parathion (615) + TX, parathion-methyl (616) + TX, penfluron [CCN] + TX, pentachlorophenol (623) + TX, pentachlorophenyl laurate (IUPAC name) (623) + TX, permethrin (626) + TX, petroleum oils (628) + TX, PH 60-38 (development code) (1328) + TX, phenkapton (1330) + TX, phenothrin (630) + TX, phenthoate (631) + TX, phorate (636) + TX, phosalone (637) + TX, phosfolan (1338) + TX, phosmet (638) + TX, phosnichlor (1339) + TX, phosphamidon (639) + TX, phosphine (IUPAC name) (640) + TX, phoxim (642) + TX, phoxim-methyl (1340) + TX, pirimetaphos (1344) + TX, pirimicarb (651) + TX, pirimiphos-ethyl (1345) + TX, pirimiphos-methyl (652) + TX, polychlorodicyclopentadiene isomers (IUPAC name) (1346) + TX, polychloroterpenes (traditional name) (1347) + TX, potassium arsenite [CCN] + TX, potassium thiocyanate [CCN] + TX, prallethrin (655) + TX, precocene I [CCN] + TX, precocene II [CCN] + TX, precocene III [CCN] + TX, primidophos (1349) + TX, profenofos (662) + TX, profluthrin [CCN] + TX, promacyl (1354) + TX, promecarb (1355) + TX, propaphos (1356) + TX, propetamphos (673) + TX, propoxur (678) + TX, prothidathion (1360) + TX, prothiofos (686) + TX, prothoate (1362) + TX, protrifenbute [CCN] + TX, pymetrozine (688) + TX, pyraclofos (689) + TX, pyrazophos (693) + TX, pyresmethrin (1367) + TX, pyrethrin I (696) + TX, pyrethrin II (696) + TX, pyrethrins (696) + TX, pyridaben (699) + TX, pyridalyl (700) + TX, pyridaphenthion (701) + TX, pyrimidifen (706) + TX, pyrimitate (1370) + TX, pyriproxyfen (708) + TX, quassia [CCN] + TX, quinalphos (711) + TX, quinalphos-methyl (1376) + TX, quinothion (1380) + TX, quintiofos (1381) + TX, R-1492 (development code) (1382) + TX, rafoxanide [CCN] + TX, resmethrin (719) + TX, rotenone (722) + TX, RU 15525 (development code) (723) + TX, RU 25475 (development code) (1386) + TX, ryania (1387) + TX, ryanodine (traditional name) (1387) + TX, sabadilla (725) + TX, schradan (1389) + TX, sebufos + TX, selamectin [CCN] + TX, SI-0009 (compound code) + TX, SI-0205 (compound code) + TX, SI-0404 (compound code) + TX, SI-0405 (compound code) + TX, silafluofen (728) + TX, SN 72129 (development code) (1397) + TX, sodium arsenite [CCN] + TX, sodium cyanide (444) + TX, sodium fluoride (IUPAC/Chemical Abstracts name) (1399) + TX, sodium hexafluorosilicate (1400) + TX, sodium pentachlorophenoxide (623) + TX, sodium selenate (IUPAC name) (1401) + TX, sodium thiocyanate [CCN] + TX, sophamide (1402) + TX, spinosad (737) + TX, spiromesifen (739) + TX, spirotetrmat (CCN) + TX, sulcofuron (746) + TX, sulcofuron-sodium (746) + TX, sulfluramid (750) + TX, sulfotep (753) + TX, sulfuryl fluoride (756) + TX, sulprofos (1408) + TX, tar oils (758) + TX, tau-fluvalinate (398) + TX, tazimcarb (1412) + TX, TDE (1414) + TX, tebufenozide (762) + TX, tebufenpyrad (763) + TX, tebupirimfos (764) + TX, teflubenzuron (768) + TX, tefluthrin (769) + TX, temephos (770) + TX, TEPP (1417) + TX, terallethrin (1418) + TX, terbam + TX, terbufos (773) + TX, tetrachloroethane [CCN] + TX, tetrachlorvinphos (777) + TX, tetramethrin (787) + TX, theta-cypermethrin (204) + TX, thiacloprid (791) + TX, thiafenox + TX, thiamethoxam (792) + TX, thicrofos (1428) + TX, thiocarboxime (1431) + TX, thiocyclam (798) + TX, thiocyclam hydrogen oxalate (798) + TX, thiodicarb (799) + TX, thiofanox (800) + TX, thiometon (801) + TX, thionazin (1434) + TX, thiosultap (803) + TX, thiosultap-sodium (803) + TX, thuringiensin [CCN] + TX, tolfenpyrad (809) + TX, tralomethrin (812) + TX, transfluthrin (813) + TX, transpermethrin (1440) + TX, triamiphos (1441) + TX, triazamate (818) + TX, triazophos (820) + TX, triazuron + TX, trichlorfon (824) + TX, trichlormetaphos-3 [CCN] + TX, trichloronat (1452) + TX, trifenofos (1455) + TX, triflumuron (835) + TX, trimethacarb (840) + TX, triprene (1459) + TX, vamidothion (847) + TX, vaniliprole [CCN] + TX, veratridine (725) + TX, veratrine (725) + TX, XMC (853) + TX, xylylcarb (854) + TX, YI-5302 (compound code) + TX, zeta-cypermethrin (205) + TX, zetamethrin + TX, zinc phosphide (640) + TX, zolaprofos (1469) and ZXI 8901 (development code) (858) + TX, cyantraniliprole [736994-63-19 + TX, chlorantraniliprole [500008-45-7] + TX, cyenopyrafen [560121-52-0] + TX, cyflumetofen [400882-07-7] + TX, pyrifluquinazon [337458-27-2] + TX, spinetoram [187166-40-1 + 187166-15-0] + TX, spirotetramat [203313-25-1] + TX, sulfoxaflor [946578-00-3] + TX, flufiprole [704886-18-0] + TX, meperfluthrin [915288-13-0] + TX, tetramethylfluthrin [84937-88-2] + TX, triflumezopyrim (disclosed in WO 2012/092115) + TX, fluxametamide (WO 2007/026965) + TX, epsilon-metofluthrin [240494-71-7] + TX, epsilon-momfluorothrin [1065124-65-3] + TX, fluazaindolizine [1254304-22-7] + TX, chloroprallethrin [399572-87-3] + TX, fluxametamide [928783-29-3] + TX, cyhalodiamide [1262605-53-7] + TX, tioxazafen [330459-31-9] + TX, broflanilide [1207727-04-5] + TX, flufiprole [704886-18-0] + TX, cyclaniliprole [1031756-98-5] + TX, tetraniliprole [1229654-66-3] + TX, guadipyr (described in WO2010/060231) + TX, cycloxaprid (described in WO2005/077934) + TX,
a molluscicide selected from the group of substances consisting of bis(tributyltin) oxide (IUPAC name) (913) + TX, bromoacetamide [CCN] + TX, calcium arsenate [CCN] + TX, cloethocarb (999) + TX, copper acetoarsenite [CCN] + TX, copper sulfate (172) + TX, fentin (347) + TX, ferric phosphate (IUPAC name) (352) + TX, metaldehyde (518) + TX, methiocarb (530) + TX, niclosamide (576) + TX, niclosamide-olamine (576) + TX, pentachlorophenol (623) + TX, sodium pentachlorophenoxide (623) + TX, tazimcarb (1412) + TX, thiodicarb (799) + TX, tributyltin oxide (913) + TX, trifenmorph (1454) + TX, trimethacarb (840) + TX, triphenyltin acetate (IUPAC name) (347) and triphenyltin hydroxide (IUPAC name) (347) + TX, pyriprole [394730-71-3] + TX,
a nematicide selected from the group of substances consisting of AKD-3088 (compound code) + TX, 1,2-dibromo-3-chloropropane (IUPAC/Chemical Abstracts name) (1045) + TX, 1,2-dichloropropane (IUPAC/ Chemical Abstracts name) (1062) + TX, 1,2-dichloropropane with 1,3-dichloropropene (IUPAC name) (1063) + TX, 1,3-dichloropropene (233) + TX, 3,4-dichlorotetrahydrothiophene 1,1-dioxide (IUPAC/Chemical Abstracts name) (1065) + TX, 3-(4-chlorophenyl)-5-methylrhodanine (IUPAC name) (980) + TX, 5-methyl-6-thioxo-1,3,5-thiadiazinan-3-ylacetic acid (IUPAC name) (1286) + TX, 6-isopentenylaminopurine (210) + TX, abamectin (1) + TX, acetoprole [CCN] + TX, alanycarb (15) + TX, aldicarb (16) + TX, aldoxycarb (863) + TX, AZ 60541 (compound code) + TX, benclothiaz [CCN] + TX, benomyl (62) + TX, butylpyridaben + TX, cadusafos (109) + TX, carbofuran (118) + TX, carbon disulfide (945) + TX, carbosulfan (119) + TX, chloropicrin (141) + TX, chlorpyrifos (145) + TX, cloethocarb (999) + TX, cytokinins (210) + TX, dazomet (216) + TX, DBCP (1045) + TX, DCIP (218) + TX, diamidafos (1044) + TX, dichlofenthion (1051) + TX, dicliphos + TX, dimethoate (262) + TX, doramectin [CCN] + TX, emamectin (291) + TX, emamectin benzoate (291) + TX, eprinomectin [CCN] + TX, ethoprophos (312) + TX, ethylene dibromide (316) + TX, fenamiphos (326) + TX, fenpyrad + TX, fensulfothion (1158) + TX, fosthiazate (408) + TX, fosthietan (1196) + TX, furfural [CCN] + TX, GY-81 (development code) (423) + TX, heterophos [CCN] + TX, iodomethane (IUPAC name) (542) + TX, isamidofos (1230) + TX, isazofos (1231) + TX, ivermectin [CCN] + TX, kinetin (210) + TX, mecarphon (1258) + TX, metam (519) + TX, metam-potassium (519) + TX, metam-sodium (519) + TX, methyl bromide (537) + TX, methyl isothiocyanate (543) + TX, milbemycin oxime [CCN] + TX, moxidectin [CCN] + TX, *Myrothecium verrucaria* composition (565) + TX, NC-184 (compound code) + TX, oxamyl (602) + TX, phorate (636) + TX, phosphamidon (639) + TX, phosphocarb [CCN] + TX, sebufos + TX, selamectin [CCN] + TX, spinosad (737) + TX, terbam + TX, terbufos (773) + TX, tetrachlorothiophene (IUPAC/ Chemical Abstracts name) (1422) + TX, thiafenox + TX, thionazin (1434) + TX, triazophos (820) + TX, triazuron + TX, xylenols [CCN] + TX, YI-5302 (compound code) and zeatin (210) + TX, fluensulfone [318290-98-1] + TX,
a nitrification inhibitor selected from the group of substances consisting of potassium ethylxanthate [CCN] and nitrapyrin (580) + TX,
a plant activator selected from the group of substances consisting of acibenzolar (6) + TX, acibenzolar-S-methyl (6) + TX, probenazole (658) and *Reynoutria sachalinensis* extract (720) + TX, a rodenticide selected from the group of substances consisting of 2-isovalerylindan-1,3-dione (IUPAC name) (1246) + TX, 4-(quinoxalin-2-ylamino)benzenesulfonamide (IUPAC name) (748) + TX, alpha-chlorohydrin [CCN] + TX, aluminium phosphide (640) + TX, antu (880) + TX, arsenous oxide (882) + TX, barium carbonate (891) + TX, bisthiosemi (912) + TX, brodifacoum (89) + TX, bromadiolone (91) + TX, bromethalin (92) + TX, calcium cyanide (444) + TX, chloralose (127) + TX, chlorophacinone (140) + TX, cholecalciferol (850) + TX, coumachlor (1004) + TX, coumafuryl (1005) + TX, coumatetralyl (175) + TX, crimidine (1009) + TX, difenacoum (246) + TX, difethialone (249) + TX, diphacinone (273) + TX, ergocalciferol (301) + TX, flocoumafen (357) + TX, fluoroacetamide (379) + TX, flupropadine (1183) + TX, flupropadine hydrochloride (1183) + TX, gamma-HCH (430) + TX, HCH (430) + TX, hydrogen cyanide (444) + TX, iodomethane (IUPAC name) (542) + TX, lindane (430) + TX, magnesium phosphide (IUPAC name) (640) + TX, methyl bromide (537) + TX, norbormide (1318) + TX, phosacetim (1336) + TX, phosphine (IUPAC name) (640) + TX, phosphorus [CCN] + TX, pindone (1341) + TX, potassium arsenite [CCN] + TX, pyrinuron (1371) + TX, scilliroside (1390) + TX, sodium arsenite [CCN] + TX, sodium cyanide (444) + TX, sodium fluoroacetate (735) + TX, strychnine (745) + TX, thallium sulfate [CCN] + TX, warfarin (851) and zinc phosphide (640) + TX,
a synergist selected from the group of substances consisting of 2-(2-butoxyethoxy)ethyl piperonylate (IUPAC name) (934) + TX, 5-(1,3-benzodioxol-5-yl)-3-hexylcyclohex-2-enone (IUPAC name) (903) + TX, farnesol with nerolidol (324) + TX, MB-599 (development code) (498) + TX, MGK 264 (development code) (296) + TX, piperonyl butoxide (649) + TX, piprotal (1343) + TX, propyl isomer (1358) + TX, S421 (development code) (724) + TX, sesamex (1393) + TX, sesasmolin (1394) and sulfoxide (1406) + TX,
an animal repellent selected from the group of substances consisting of anthraquinone (32) + TX, chloralose (127) + TX, copper naphthenate [CCN] + TX, copper oxychloride (171) + TX, diazinon (227) + TX, dicyclopentadiene (chemical name) (1069) + TX, guazatine (422) + TX, guazatine acetates (422) + TX, methiocarb (530) + TX, pyridin-4-amine (IUPAC name) (23) + TX, thiram (804) + TX, trimethacarb (840) + TX, zinc naphthenate [CCN] and ziram (856) + TX,
a virucide selected from the group of substances consisting of imanin [CCN] and ribavirin [CCN] + TX,
a wound protectant selected from the group of substances consisting of mercuric oxide (512) + TX, octhilinone (590) and thiophanate-methyl (802) + TX,
and biologically active compounds selected from the group consisting of azaconazole (60207-31-0] + TX, bitertanol [70585-36-3] + TX, bromuconazole [116255-48-2] + TX, cyproconazole [94361-06-5] + TX, difenoconazole [119446-68-3] + TX, diniconazole [83657-24-3] + TX, epoxiconazole [106325-08-0] + TX, fenbuconazole [114369-43-6] + TX, fluquinconazole [136426-54-5] + TX, flusilazole [85509-19-9] + TX, flutriafol [76674-21-0] + TX, hexaconazole [79983-71-4] + TX, imazalil [35554-44-0] + TX, imibenconazole [86598-92-7] + TX, ipconazole [125225-28-7] + TX, metconazole [125116-23-6] + TX, myclobutanil [88671-89-0] + TX, pefurazoate [101903-30-4] + TX, penconazole [66246-88-6] + TX, prothioconazole [178928-70-6] + TX, pyrifenox [88283-41-4] + TX, prochloraz [67747-09-5] + TX, propiconazole [60207-90-1] + TX, simeconazole [149508-90-7] + TX, tebucon-azole [107534-96-3] + TX, tetraconazole [112281-77-3] + TX, triadimefon [43121-43-3] + TX, triadimenol [55219-65-3] + TX, triflumizole [99387-89-0] + TX, triticonazole [131983-72-7] + TX, ancymidol [12771-68-5] + TX, fenarimol [60168-88-9] + TX, nuarimol [63284-71-9] + TX, bupirimate [41483-43-6] + TX, dimethirimol [5221-53-4] + TX, ethirimol [23947-60-6] + TX, dodemorph [1593-77-7] + TX, fenpropidine [67306-00-7] + TX, fenpropimorph [67564-91-4] + TX, spiroxamine [118134-30-8] + TX, tridemorph [81412-43-3] + TX, cyprodinil [121552-61-2] + TX, mepanipyrim [110235-47-7] + TX, pyrimethanil [53112-28-0] + TX, fenpiclonil [74738-17-3] + TX, fludioxonil [131341-86-1] + TX, benalaxyl [71626-11-4] + TX, furalaxyl [57646-30-7] + TX, metalaxyl [57837-19-1] + TX, R-metalaxyl [70630-17-0] + TX, ofurace [58810-48-3] + TX, oxadixyl [77732-09-3] + TX, benomyl [17804-35-2] + TX, carbendazim [10605-21-7] + TX, debacarb [62732-91-6] + TX, fuberidazole [3878-19-1] + TX, thiabendazole [148-79-8] + TX, chlozolinate [84332-86-5] + TX, dichlozoline [24201-58-9] + TX, iprodione [36734-19-7] + TX, myclozoline [54864-61-8] + TX, procymidone [32809-16-8] + TX, vinclozoline [50471-44-8] + TX, boscalid [188425-85-6] + TX, carboxin [5234-68-4] + TX, fenfuram [24691-80-3] + TX, flutolanil [66332-96-5] + TX, mepronil [55814-41-0] + TX, oxycarboxin [5259-88-1] + TX, penthiopyrad [183675-82-3] + TX, thifluzamide [130000-40-7] + TX, guazatine [108173-90-6] + TX, dodine [2439-10-3] [112-65-2] (free base) + TX, iminoctadine [13516-27-3] + TX, azoxystrobin [131860-33-8] + TX, dimoxystrobin [149961-52-4] + TX, enestroburin {Proc. BCPC, Int. Congr., Glasgow, 2003, 1, 93} + TX, fluoxastrobin [361377-29-9] + TX, kresoxim-methyl [143390-89-0] + TX, metominostrobin [133408-50-1] + TX, trifloxystrobin [141517-21-7] + TX, orysastrobin [248593-16-0] + TX, picoxystrobin [117428-22-5] + TX, pyraclostrobin [175013-18-0] + TX, ferbam [14484-64-1] + TX, mancozeb [8018-01-7] + TX, maneb [12427-38-2] + TX, metiram [9006-42-2] + TX, propineb [12071-83-9] + TX, thiram [137-26-8] + TX, zineb [12122-67-7] + TX, ziram [137-30-4] + TX, captafol [2425-06-1] + TX, captan [133-06-2] + TX, dichlofluanid [1085-98-9] + TX, fluoroimide [41205-21-4] + TX, folpet [133-07-3 ] + TX, tolylfluanid [731-27-1] + TX, bordeaux mixture [8011-63-0] + TX, copperhydroxid [20427-59-2] + TX, copperoxychlorid [1332-40-7] + TX, coppersulfat [7758-98-7] + TX, copperoxid [1317-39-1] + TX, mancopper [53988-93-5] + TX, oxine-copper [10380-28-6] + TX, dinocap [131-72-6] + TX, nitrothal-isopropyl [10552-74-6] + TX, edifenphos [17109-49-8] + TX, iprobenphos [26087-47-8] + TX, isoprothiolane [50512-35-1] + TX, phosdiphen [36519-00-3] + TX, pyrazophos [13457-18-6] + TX, tolclofos-methyl [57018-04-9] + TX, acibenzolar-S-methyl [135158-54-2] + TX, anilazine [101-05-3] + TX, benthiavalicarb [413615-35-7] + TX, blasticidin-S [2079-00-7] + TX, chinomethionat [2439-01-2] + TX, chloroneb [2675-77-6] + TX, chlorothalonil *[1897-45-6]* + TX, cyflufenamid *[180409-60-3]* + TX, cymoxanil *[57966*-*95*-*7]* + TX, dichlone *[117-80-6]* + TX, diclocymet [139920-32-4] + TX, diclomezine *[62865*-*36*-*5]* + TX, dicloran *[99*-*30*-*9]* + TX, diethofencarb *[87130-20-9]* + TX, dimethomorph *[110488-70-5]* + TX, SYP-LI90 (Flumorph) *[211867*-*47-9*] + TX, dithianon *[3347-22-6]* + TX, ethaboxam *[162650-77-3]* + TX, etridiazole *[2593-15-9]* + TX, famoxadone *[131807-57-3]* + *TX,* fenamidone *[161326-34-7]* + TX, fenoxanil *[115852-48-7]* + TX, fentin *[668-34-8]* + TX, ferimzone *[89269-64-7]* + TX, fluazinam *[79622-59-6]* + TX, fluopicolide [239110-15-7] + TX, flusulfamide [106917-52-6] + TX, fenhexamid [126833-17-8] + TX, fosetyl-aluminium [39148-24-8] + TX, hymexazol [10004-44-1] + TX, iprovalicarb [140923-17-7] + TX, IKF-916 (Cyazofamid) *[120116-88-3]* + TX, kasugamycin [6980-18-3] + TX, methasulfocarb *[66952-49-6]* + TX, metrafenone *[220899-03-6]* + TX, pencycuron *[66063-05-6]* + TX, phthalide [27355-22-2] + TX, polyoxins [11113-80-7] + TX, probenazole [27605-76-1] + TX, propamocarb [25606-41-1] + TX, proquinazid [189278-12-4] + TX, pyroquilon [57369-32-1] + TX, quinoxyfen [124495-18-7] + TX, quintozene [82-68-8] + TX, sulfur [7704-34-9] + TX, tiadinil [223580-51-6] + TX, triazoxide [72459-58-6] + TX, tricyclazole [41814-78-2] + TX, triforine [26644-46-2] + TX, validamycin [37248-47-8] + TX, zoxamide (RH7281) [156052-68-5] + TX, mandipropamid [374726-62-2] + TX, isopyrazam [881685-58-1] + TX, sedaxane [874967-67-6] + TX, 3-difluoromethyl-1-methyl-1H-pyrazole-4-carboxylic acid (9-dichloromethylene-1,2,3,4-tetrahydro-1,4-methano-naphthalen-5-yl)-amide (dislosed in WO 2007/048556) + TX, 3-difluoromethyl-1-methyl-1H-pyrazole-4-carboxylic acid (3',4',5'-trifluoro-biphenyl-2-yl)-amide (disclosed in WO 2006/087343) + TX, [(3*S*,4*R*,4a*R*,6*S*,6a*S*,12*R*, 12a*S*, 12b*S*)-3-[(cyclopropylcarbonyl)oxy]- 1 ,3,4,4a,5,6,6a, 12,12a, 12b-decahydro-6,12-dihydroxy-4,6a,12b-trimethyl-11-oxo-9-(3-pyridinyl)-2*H*,11*H*naphtho[2,1-*b*]pyrano[3,4-e]pyran-4-yl]methyl-cyclopropanecarboxylate [915972-17-7] + TX and 1,3,5-trimethyl-N-(2-methyl-1-oxopropyl)-N-[3-(2-methylpropyl)-4-[2,2,2-trifluoro-1-methoxy-1-(trifluoromethyl)ethyl]phenyl]-1H-pyrazole-4-carboxamide [926914-55-8] + TX; lancotrione [1486617-21-3] + TX, florpyrauxifen [943832-81-3]] + TX, ipfentrifluconazole[1417782-08-1]] + TX, mefentrifluconazole [1417782-03-6]] + TX, quinofumelin [861647-84-9]] + TX, chloroprallethrin [399572-87-3]] + TX, cyhalodiamide [1262605-53-7]] + TX, fluazaindolizine [1254304-22-7]] + TX, fluxametamide [928783-29-3]] + TX, epsilon-metofluthrin [240494-71-7]] + TX, epsilon-momfluorothrin [1065124-65-3]] + TX, pydiflumetofen [1228284-64-7]] + TX, kappa-bifenthrin [439680-76-9]] + TX, broflanilide [1207727-04-5]] + TX, dicloromezotiaz [1263629-39-5]] + TX, dipymetitrone [16114-35-5]] + TX, pyraziflumid [942515-63-1] and kappa-tefluthrin [391634-71-2]] + TX; and
microbials including: *Acinetobacter Iwoffii* + TX, *Acremonium alternatum* + TX + TX, *Acremonium cephalosporium* + TX + TX, *Acremonium diospyri* + TX, *Acremonium obclavatum* + TX, *Adoxophyes orana granulovirus* (AdoxGV) (Capex®) + TX, *Agrobacterium radiobacter* strain K84 (Galltrol-A®) + TX, *Alternaria alternate* + TX, *Alternaria cassia* + TX, *Alternaria destruens* (Smolder®) + TX, *Ampelomyces quisqualis* (AQ10®) + TX, *Aspergillus flavus* AF36 (AF36®) + TX, *Aspergillus flavus* NRRL 21882 (Aflaguard®) + TX, *Aspergillus* spp. + TX, *Aureobasidium pullulans* + TX, *Azospirillum* + TX, (MicroAZ® + TX, TAZO B®) + TX, *Azotobacter* + TX, *Azotobacter chroocuccum* (Azotomeal®) + TX, *Azotobacter* cysts (Bionatural Blooming Blossoms®) + TX, *Bacillus amyloliquefaciens* + TX, *Bacillus cereus* + TX, *Bacillus chitinosporus* strain CM-1 + TX, *Bacillus chitinosporus* strain AQ746 + TX, *Bacillus licheniformis* strain HB-2 (Biostart™ Rhizoboost®) + TX, *Bacillus licheniformis* strain 3086 (EcoGuard® + TX, Green Releaf®) + TX, *Bacillus circulans* + TX, *Bacillus firmus* (BioSafe® + TX, BioNem-WP® + TX, VOTiVO®) + TX, *Bacillus firmus* strain I-1582 + TX, *Bacillus macerans* + TX, *Bacillus marismortui* + TX, *Bacillus megaterium* + TX, *Bacillus mycoides* strain AQ726 + TX, *Bacillus papillae* (Milky Spore Powder®) + TX, *Bacillus pumilus* spp. + TX, *Bacillus pumilus* strain GB34 (Yield Shield®) + TX, *Bacillus pumilus* strain AQ717 + TX, *Bacillus pumilus* strain QST 2808 (Sonata® + TX, Ballad Plus®) + TX, *Bacillus spahericus* (VectoLex®) + TX, *Bacillus* spp. + TX, *Bacillus* spp. strain AQ175 + TX, *Bacillus* spp. strain AQ177 + TX, *Bacillus* spp. strain AQ178 + TX, *Bacillus subtilis* strain QST 713 (CEASE® + TX, Serenade® + TX, Rhapsody®) + TX, *Bacillus subtilis* strain QST 714 (JAZZ®) + TX, *Bacillus subtilis* strain AQ153 + TX, *Bacillus subtilis* strain AQ743 + TX, *Bacillus subtilis* strain QST3002 + TX, *Bacillus subtilis* strain QST3004 + TX, *Bacillus subtilis var. amyloliquefaciens* strain FZB24 (Taegro® + TX, Rhizopro®) + TX, *Bacillus thuringiensis* Cry 2Ae + TX, *Bacillus thuringiensis* Cry1Ab + TX, *Bacillus thuringiensis aizawai* GC 91 (Agree®) + TX, *Bacillus thuringiensis israelensis* (BMP123® + TX, Aquabac® + TX, VectoBac®) + TX, *Bacillus thuringiensis kurstaki* (Javelin® + TX, Deliver® + TX, CryMax® + TX, Bonide® + TX, Scutella WP® + TX, Turilav WP® + TX, Astuto® + TX, Dipel WP® + TX, Biobit® + TX, Foray®) + TX, *Bacillus thuringiensis kurstaki* BMP 123 (Baritone®) + TX, *Bacillus thuringiensis kurstaki* HD-1 (Bioprotec-CAF / 3P®) + TX, *Bacillus thuringiensis* strain BD#32 + TX, *Bacillus thuringiensis* strain AQ52 + TX, *Bacillus thuringiensis var. aizawai* (XenTari® + TX, DiPel®) + TX, bacteria spp. (GROWMEND® + TX, GROWSWEET® + TX, Shootup®) + TX, bacteriophage of *Clavipacter michiganensis* (AgriPhage®) + TX, Bakflor® + TX, *Beauveria bassiana* (Beaugenic® + TX, Brocaril WP®) + TX, *Beauveria bassiana* GHA (Mycotrol ES® + TX, Mycotrol O® + TX, BotaniGuard®) + TX, *Beauveria brongniartii* (Engerlingspilz® + TX, Schweizer Beauveria® + TX, Melocont®) + TX, *Beauveria* spp. + TX, *Botrytis cineria* + TX, *Bradyrhizobium japonicum* (TerraMax®) + TX, *Brevibacillus brevis* + TX, *Bacillus thuringiensis tenebrionis* (Novodor®) + TX, BtBooster + TX, *Burkholderia cepacia* (Deny® + TX, Intercept® + TX, Blue Circle®) + TX, *Burkholderia gladii* + TX, *Burkholderia gladioli* + TX, *Burkholderia* spp. + TX, Canadian thistle fungus (CBH Canadian Bioherbicide®) + TX, *Candida butyri* + TX, *Candida famata* + TX, *Candida fructus* + TX, *Candida glabrata* + TX, *Candida guilliermondii* + TX, *Candida melibiosica* + TX, *Candida oleophila* strain O + TX, *Candida parapsilosis* + TX, *Candida pelliculosa* + TX, *Candida pulcherrima* + TX, *Candida reukaufii* + TX, *Candida saitoana* (Bio-Coat® + TX, Biocure®) + TX, *Candida sake* + TX, *Candida* spp. + TX, *Candida tenius* + TX, *Cedecea dravisae* + TX, *Cellulomonas flavigena* + TX, *Chaetomium cochliodes* (Nova-Cide®) + TX, *Chaetomium globosum* (Nova-Cide®) + TX, *Chromobacterium subtsugae* strain PRAA4-1T (Grandevo®) + TX, *Cladosporium cladosporioides* + TX, *Cladosporium oxysporum* + TX, *Cladosporium chlorocephalum* + TX, *Cladosporium* spp. + TX, *Cladosporium tenuissimum* + TX, *Clonostachys rosea* (EndoFine®) + TX, *Colletotrichum acutatum* + TX, *Coniothyrium minitans* (Cotans WG®) + TX, *Coniothyrium* spp. + TX, *Cryptococcus albidus* (YIELDPLUS®) + TX, *Cryptococcus humicola* + TX, *Cryptococcus infirmo-miniatus* + TX, *Cryptococcus laurentii* + TX, *Cryptophlebia leucotreta granulovirus* (Cryptex®) + TX, *Cupriavidus campinensis* + TX, *Cydia pomonella granulovirus* (CYD-X®) + TX, *Cydia pomonella granulovirus* (Madex® + TX, Madex Plus® + TX, Madex Max/ Carpovirusine®) + TX, *Cylindrobasidium laeve* (Stumpout®) + TX, *Cylindrocladium* + TX, *Debaryomyces hansenii* + TX, *Drechslera hawaiinensis* + TX, *Enterobacter cloacae* + TX, *Enterobacteriaceae* + TX, *Entomophtora virulenta* (Vektor®) + TX, *Epicoccum nigrum* + TX, *Epicoccum purpurascens* + TX, *Epicoccum* spp. + TX, *Filobasidium floriforme* + TX, *Fusarium acuminatum* + TX, *Fusarium chlamydosporum* + TX, *Fusarium oxysporum* (Fusaclean® / Biofox C®) + TX, *Fusarium proliferatum* + TX, *Fusarium* spp. + TX, *Galactomyces geotrichum* + TX, *Gliocladium catenulatum* (Primastop® + TX, Prestop®) + TX, *Gliocladium roseum* + TX, *Gliocladium* spp. (SoilGard®) + TX, *Gliocladium virens* (Soilgard®) + TX, *Granulovirus* (Granupom®) + TX, *Halobacillus halophilus* + TX, *Halobacillus litoralis* + TX, *Halobacillus trueperi* + TX, *Halomonas* spp. + TX, *Halomonas subglaciescola* + TX, *Halovibrio variabilis* + TX, *Hanseniaspora uvarum* + TX, *Helicoverpa armigera nucleopolyhedrovirus* (Helicovex®) + TX, *Helicoverpa* zea *nuclear polyhedrosis virus* (Gemstar®) + TX, Isoflavone - formononetin (Myconate®) + TX, *Kloeckera apiculata* + TX, *Kloeckera* spp. + TX, *Lagenidium giganteum* (Laginex®) + TX, *Lecanicillium longisporum* (Vertiblast®) + TX, *Lecanicillium muscarium* (Vertikil®) + TX, *Lymantria Dispar nucleopolyhedrosis virus* (Disparvirus®) + TX, *Marinococcus halophilus* + TX, *Meira geulakonigii* + TX, *Metarhizium anisopliae* (Met52®) + TX, *Metarhizium anisopliae* (Destruxin WP®) + TX, *Metschnikowia fruticola* (Shemer®) + TX, *Metschnikowia pulcherrima* + TX, *Microdochium dimerum* (Antibot®) + TX, *Micromonospora coerulea* + TX, *Microsphaeropsis ochracea* + TX, *Muscodor albus* 620 (Muscudor®) + TX, *Muscodor roseus* strain A3-5 + TX, *Mycorrhizae* spp. (AMykor® + TX, Root Maximizer®) + TX, *Myrothecium verrucaria* strain AARC-0255 (DiTera®) + TX, BROS PLUS® + TX, *Ophiostoma piliferum* strain D97 (Sylvanex®) + TX, *Paecilomyces farinosus* + TX, *Paecilomyces fumosoroseus* (PFR-97® + TX, PreFeRal®) + TX, *Paecilomyces linacinus* (Biostat WP®) + TX, *Paecilomyces lilacinus* strain 251 (MeloCon WG®) + TX, *Paenibacillus polymyxa* + TX, *Pantoea agglomerans* (BlightBan C9-1®) + TX, *Pantoea* spp. + TX, *Pasteuria* spp. (Econem®) + TX, *Pasteuria nishizawae* + TX, *Penicillium aurantiogriseum* + TX, *Penicillium billai* (Jumpstart® + TX, TagTeam®) + TX, *Penicillium brevicompactum* + TX, *Penicillium frequentans* + TX, *Penicillium griseofulvum* + TX, *Penicillium purpurogenum* + TX, *Penicillium* spp. + TX, *Penicillium viridicatum* + TX, *Phlebiopsis gigantean* (Rotstop®) + TX, phosphate solubilizing bacteria (Phosphomeal®) + TX, *Phytophthora cryptogea* + TX, *Phytophthora palmivora* (Devine®) + TX, *Pichia anomala* + TX, *Pichia guilermondii* + TX, *Pichia membranaefaciens* + TX, *Pichia onychis* + TX, *Pichia stipites* + TX, *Pseudomonas aeruginosa* + TX, *Pseudomonas aureofasciens* (Spot-Less Biofungicide®) + TX, *Pseudomonas cepacia* + TX, *Pseudomonas chlororaphis* (AtEze®) + TX, *Pseudomonas corrugate* + TX, *Pseudomonas fluorescens* strain A506 (BlightBan A506®) + TX, *Pseudomonas putida* + TX, *Pseudomonas reactans* + TX, *Pseudomonas* spp. + TX, *Pseudomonas syringae* (Bio-Save®) + TX, *Pseudomonas viridiflava* + TX, *Pseudomons fluorescens* (Zequanox®) + TX, *Pseudozyma flocculosa* strain PF-A22 UL (Sporodex L®) + TX, *Puccinia canaliculata* + TX, *Puccinia thlaspeos* (Wood Warrior®) + TX, *Pythium paroecandrum* + TX, *Pythium oligandrum* (Polygandron® + TX, Polyversum®) + TX, *Pythium periplocum* + TX, *Rhanella aquatilis* + TX, *Rhanella* spp. + TX, *Rhizobia* (Dormal® + TX, Vault®) + TX, *Rhizoctonia* + TX, *Rhodococcus globerulus* strain AQ719 + TX, *Rhodosporidium diobovatum* + TX, *Rhodosporidium toruloides* + TX, *Rhodotorula* spp. + TX, *Rhodotorula glutinis* + TX, *Rhodotorula graminis* + TX, *Rhodotorula mucilagnosa* + TX, *Rhodotorula rubra* + TX, *Saccharomyces cerevisiae* + TX, *Salinococcus roseus* + TX, *Sclerotinia minor* + TX, *Sclerotinia minor* (SARRlTOR®) + TX, *Scytalidium* spp. + TX, *Scytalidium uredinicola* + TX, *Spodoptera exigua nuclear polyhedrosis virus* (Spod-X® + TX, Spexit®) + TX, *Serratia marcescens* + TX, *Serratia plymuthica* + TX, *Serratia* spp. + TX, *Sordaria fimicola* + TX, *Spodoptera littoralis nucleopolyhedrovirus* (Littovir®) + TX, *Sporobolomyces roseus* + TX, *Stenotrophomonas maltophilia* + TX, *Streptomyces ahygroscopicus* + TX, *Streptomyces albaduncus* + TX, *Streptomyces exfoliates* + TX, *Streptomyces galbus* + TX, *Streptomyces griseoplanus* + TX, *Streptomyces griseoviridis* (Mycostop®) + TX, *Streptomyces lydicus* (Actinovate®) + TX, *Streptomyces lydicus* WYEC-108 (ActinoGrow®) + TX, *Streptomyces violaceus* + TX, *Tilletiopsis minor* + TX, *Tilletiopsis* spp. + TX, *Trichoderma asperellum* (T34 Biocontrol®) + TX, *Trichoderma gamsii* (Tenet®) + TX, *Trichoderma atroviride* (Plantmate®) + TX, *Trichoderma hamatum* TH 382 + TX, *Trichoderma harzianum rifai* (Mycostar®) + TX, *Trichoderma harzianum* T-22 (Trianum-P® + TX, PlantShield HC® + TX, RootShield® + TX, Trianum-G®) + TX, *Trichoderma harzianum* T-39 (Trichodex®) + TX, *Trichoderma inhamatum* + TX, *Trichoderma koningii* + TX, *Trichoderma* spp. LC 52 (Sentinel®) + TX, *Trichoderma lignorum* + TX, *Trichoderma longibrachiatum* + TX, *Trichoderma polysporum* (Binab T®) + TX, *Trichoderma taxi* + TX, *Trichoderma virens* + TX, *Trichoderma virens* (formerly Gliocladium virens GL-21) (SoilGuard®) + TX, *Trichoderma viride* + TX, *Trichoderma viride* strain ICC 080 (Remedier®) + TX, *Trichosporon pullulans* + TX, *Trichosporon* spp. + TX, *Trichothecium* spp. + TX, *Trichothecium roseum* + TX, *Typhula phacorrhiza* strain 94670 + TX, *Typhula phacorrhiza* strain 94671 + TX, *Ulocladium atrum* + TX, *Ulocladium oudemansii* (Botry-Zen®) + TX, *Ustilago maydis* + TX, various bacteria and supplementary micronutrients (Natural II®) + TX, various fungi (Millennium Microbes®) + TX, *Verticillium chlamydosporium* + TX, *Verticillium lecanii* (Mycotal® + TX, Vertalec®) + TX, Vip3Aa20 (VIPtera®) + TX, *Virgibaclillus marismortui* + TX, *Xanthomonas campestris pv. Poae* (Camperico®) + TX, *Xenorhabdus bovienii* + TX, *Xenorhabdus nematophilus;* and
Plant extracts including: pine oil (Retenol®) + TX, azadirachtin (Plasma Neem Oil® + TX, AzaGuard® + TX, MeemAzal® + TX, Molt-X® + TX, Botanical IGR (Neemazad® + TX, Neemix®) + TX, canola oil (Lilly Miller Vegol®) + TX, *Chenopodium ambrosioides near ambrosioides* (Requiem®) + TX, *Chrysanthemum* extract (Crisant®) + TX, extract of neem oil (Trilogy®) + TX, essentials oils of *Labiatae* (Botania®) + TX, extracts of clove rosemary peppermint and thyme oil (Garden insect killer®) + TX, Glycinebetaine (Greenstim®) + TX, garlic + TX, lemongrass oil (GreenMatch®) + TX, neem oil + TX, *Nepeta cataria* (Catnip oil) + TX, *Nepeta catarina* + TX, nicotine + TX, oregano oil (MossBuster®) + TX, *Pedaliaceae* oil (Nematon®) + TX, pyrethrum + TX, *Quillaja saponaria* (NemaQ®) + TX, *Reynoutria sachalinensis* (Regalia® + TX, Sakalia®) + TX, rotenone (Eco Roten®) + TX, *Rutaceae* plant extract (Soleo®) + TX, soybean oil (Ortho ecosense®) + TX, tea tree oil (Timorex Gold®) + TX, thymus oil + TX, AGNIQUE® MMF + TX, BugOil® + TX, mixture of rosemary sesame pepermint thyme and cinnamon extracts (EF 300®) + TX, mixture of clove rosemary and peppermint extract (EF 400®) + TX, mixture of clove pepermint garlic oil and mint (Soil Shot®) + TX, kaolin (Screen®) + TX, storage glucam of brown algae (Laminarin®); and
pheromones including: blackheaded fireworm pheromone (3M Sprayable Blackheaded Fireworm Pheromone®) + TX, Codling Moth Pheromone (Paramount dispenser-(CM)/ Isomate C-Plus®) + TX, Grape Berry Moth Pheromone (3M MEC-GBM Sprayable Pheromone®) + TX, Leafroller pheromone (3M MEC - LR Sprayable Pheromone®) + TX, Muscamone (Snip7 Fly Bait® + TX, Starbar Premium Fly Bait®) + TX, Oriental Fruit Moth Pheromone (3M oriental fruit moth sprayable pheromone®) + TX, Peachtree Borer Pheromone (Isomate-P®) + TX, Tomato Pinworm Pheromone (3M Sprayable pheromone®) + TX, Entostat powder (extract from palm tree) (Exosex CM®) + TX, (E + TX,Z + TX,Z)-3 + TX,8 + TX,11 Tetradecatrienyl acetate + TX, (Z + TX,Z + TX,E)-7 + TX,11 + TX,13-Hexadecatrienal + TX, (E + TX,Z)-7 + TX,9-Dodecadien-1-yl acetate + TX, 2-Methyl-1-butanol + TX, Calcium acetate + TX, Scenturion® + TX, Biolure® + TX, Check-Mate® + TX, Lavandulyl senecioate; and
Macrobials including: *Aphelinus abdominalis* + TX, *Aphidius ervi* (Aphelinus-System®) + TX, *Acerophagus papaya* + TX, *Adalia bipunctata* (Adalia-System®) + TX, *Adalia bipunctata* (Adaline®) + TX, *Adalia bipunctata* (Aphidalia®) + TX, *Ageniaspis citricola* + TX, *Ageniaspis fuscicollis* + TX, *Amblyseius andersoni* (Anderline® + TX, Andersoni-System®) + TX, *Amblyseius californicus* (Amblyline® + TX, Spical®) + TX, *Amblyseius cucumeris* (Thripex® + TX, Bugline cucumeris®) + TX, *Amblyseius fallacis* (Fallacis®) + TX, *Amblyseius swirskii* (Bugline swirskii® + TX, Swirskii-Mite®) + TX, *Amblyseius womersleyi* (WomerMite®) + TX, *Amitus hesperidum* + TX, *Anagrus atomus* + TX, *Anagyrus fusciventris* + TX, *Anagyrus kamali* + TX, *Anagyrus loecki* + TX, *Anagyrus pseudococci* (Citripar®) + TX, *Anicetus benefices* + TX, *Anisopteromalus calandrae* + TX, *Anthocoris nemoralis* (Anthocoris-System®) + TX, *Aphelinus abdominalis* (Apheline® + TX, Aphiline®) + TX, *Aphelinus asychis* + TX, *Aphidius colemani* (Aphipar®) + TX, *Aphidius ervi* (Ervipar®) + TX, *Aphidius gifuensis* + TX, *Aphidius matricariae* (Aphipar-M®) + TX, *Aphidoletes aphidimyza* (Aphidend®) + TX, *Aphidoletes aphidimyza* (Aphidoline®) + TX, *Aphytis lingnanensis* + TX, *Aphytis melinus* + TX, *Aprostocetus hagenowii* + TX, *Atheta coriaria* (Staphyline®) + TX, *Bombus* spp. + TX, *Bombus terrestris* (Natupol Beehive®) + TX, *Bombus terrestris* (Beeline® + TX, Tripol®) + TX, *Cephalonomia stephanoderis* + TX, *Chilocorus nigritus* + TX, *Chrysoperla carnea* (Chrysoline®) + TX, *Chrysoperla carnea* (Chrysopa®) + TX, *Chrysoperla rufilabris* + TX, *Cirrospilus ingenuus* + TX, *Cirrospilus quadristriatus* + TX, *Citrostichus phyllocnistoides* + TX, *Closterocerus chamaeleon* + TX, *Closterocerus* spp. + TX, *Coccidoxenoides perminutus* (Planopar®) + TX, *Coccophagus cowperi* + TX, *Coccophagus lycimnia* + TX, *Cotesia flavipes* + TX, *Cotesia plutellae* + TX, *Cryptolaemus montrouzieri* (Cryptobug® + TX, Cryptoline®) + TX, *Cybocephalus nipponicus* + TX, *Dacnusa sibirica* + TX, *Dacnusa sibirica* (Minusa®) + TX, *Diglyphus isaea* (Diminex®) + TX, *Delphastus catalinae* (Delphastus®) + TX, *Delphastus pusillus* + TX, *Diachasmimorpha krausii* + TX, *Diachasmimorpha longicaudata* + TX, *Diaparsis jucunda* + TX, *Diaphorencyrtus aligarhensis* + TX, *Diglyphus isaea* + TX, *Diglyphus isaea* (Miglyphus® + TX, Digline®) + TX, *Dacnusa sibirica* (DacDigline® + TX, Minex®) + TX, *Diversinervus* spp. + TX, *Encarsia citrina* + TX, *Encarsia formosa* (Encarsia max® + TX, Encarline® + TX, En-Strip®) + TX, *Eretmocerus eremicus* (Enermix®) + TX, *Encarsia guadeloupae* + TX, *Encarsia haitiensis* + TX, *Episyrphus balteatus* (Syrphidend®) + TX, *Eretmoceris siphonini* + TX, *Eretmocerus californicus* + TX, *Eretmocerus eremicus* (Ercal® + TX, Eretline e®) + TX, *Eretmocerus eremicus* (Bemimix®) + TX, *Eretmocerus hayati* + TX, *Eretmocerus mundus* (Bemipar® + TX, Eretline m®) + TX, *Eretmocerus siphonini* + TX, *Exochomus quadripustulatus* + TX, *Feltiella acarisuga* (Spidend®) + TX, *Feltiella acarisuga* (Feltiline®) + TX, *Fopius arisanus* + TX, *Fopius ceratitivorus* + TX, Formononetin (Wirless Beehome®) + TX, *Franklinothrips vespiformis* (Vespop®) + TX, *Galendromus occidentalis* + TX, *Goniozus legneri* + TX, *Habrobracon hebetor* + TX, *Harmonia axyridis* (HarmoBeetle®) + TX, *Heterorhabditis* spp. (Lawn Patrol®) + TX, *Heterorhabditis bacteriophora* (NemaShield HB® + TX, Nemaseek® + TX, Terranem-Nam® + TX, Terranem® + TX, Larvanem® + TX, B-Green® + TX, NemAttack® + TX, Nematop®) + TX, *Heterorhabditis megidis* (Nemasys H® + TX, BioNem H® + TX, Exhibitline hm® + TX, Larvanem-M®) + TX, *Hippodamia convergens* + TX, *Hypoaspis aculeifer* (Aculeifer-System® + TX, Entomite-A®) + TX, *Hypoaspis miles* (Hypoline m® + TX, Entomite-M®) + TX, *Lbalia leucospoides* + TX, *Lecanoideus floccissimus* + TX, *Lemophagus errabundus* + TX, *Leptomastidea abnormis* + TX, *Leptomastix dactylopii* (Leptopar®) + TX, *Leptomastix epona* + TX, *Lindorus lophanthae* + TX, *Lipolexis oregmae* + TX, *Lucilia caesar* (Natufly®) + TX, *Lysiphlebus testaceipes* + TX, *Macrolophus caliginosus* (Mirical-N® + TX, Macroline c® + TX, Mirical®) + TX, *Mesoseiulus longipes* + TX, *Metaphycus flavus* + TX, *Metaphycus lounsburyi* + TX, *Micromus angulatus* (Milacewing®) + TX, *Microterys flavus* + TX, *Muscidifurax raptorellus* and *Spalangia cameroni* (Biopar®) + TX, *Neodryinus typhlocybae* + TX, *Neoseiulus californicus* + TX, *Neoseiulus cucumeris* (THRYPEX®) + TX, *Neoseiulus fallacis* + TX, *Nesideocoris tenuis* (NesidioBug® + TX, Nesibug®) + TX, *Ophyra aenescens* (Biofly®) + TX, *Orius insidiosus* (Thripor-I® + TX, Oriline i®) + TX, *Orius laevigatus* (Thripor-L® + TX, Oriline I®) + TX, *Orius majusculus* (Oriline m®) + TX, *Orius strigicollis* (Thripor-S@) + TX, *Pauesia juniperorum* + TX, *Pediobius foveolatus* + TX, *Phasmarhabditis hermaphrodita* (Nemaslug®) + TX, *Phymastichus coffea* + TX, *Phytoseiulus macropilus* + TX, *Phytoseiulus persimilis* (Spidex® + TX, Phytoline p®) + TX, *Podisus maculiventris* (Podisus®) + TX, *Pseudacteon curvatus* + TX, *Pseudacteon obtusus* + TX, *Pseudacteon tricuspis* + TX, *Pseudaphycus maculipennis* + TX, *Pseudleptomastix mexicana* + TX, *Psyllaephagus pilosus* + TX, *Psyttalia concolor* (complex) + TX, *Quadrastichus* spp. + TX, *Rhyzobius lophanthae* + TX, *Rodolia cardinalis* + TX, *Rumina decollate* + TX, *Semielacher petiolatus* + TX, *Sitobion avenae* (Ervibank®) + TX, *Steinernema carpocapsae* (Nematac C® + TX, Millenium® + TX, BioNem C® + TX, NemAttack® + TX, Nemastar® + TX, Capsanem®) + TX, *Steinernema feltiae* (NemaShield® + TX, Nemasys F® + TX, BioNem F® + TX, Steinernema-System® + TX, NemAttack® + TX, Nemaplus® + TX, Exhibitline sf® + TX, Scia-rid® + TX, Entonem®) + TX, *Steinernema kraussei* (Nemasys L® + TX, BioNem L® + TX, Exhibitline srb®) + TX, *Steinernema riobrave* (BioVector® + TX, BioVektor®) + TX, *Steinernema scapterisci* (Nematac S®) + TX, *Steinernema* spp. + TX, *Steinernematid* spp. (Guardian Nematodes®) + TX, *Stethorus punctillum* (Stethorus®) + TX, *Tamarixia radiate* + TX, *Tetrastichus setifer* + TX, *Thripobius semiluteus* + TX, *Torymus sinensis* + TX, *Trichogramma brassicae* (Tricholine b®) + TX, *Trichogramma brassicae* (Tricho-Strip®) + TX, *Trichogramma evanescens* + TX, *Trichogramma minutum* + TX, *Trichogramma ostriniae* + TX, *Trichogramma platneri* + TX, *Trichogramma pretiosum* + TX, *Xanthopimpla stemmator;* and
other biologicals including: abscisic acid + TX, bioSea® + TX, *Chondrostereum purpureum* (Chontrol Paste®) + TX, *Colletotrichum gloeosporioides* (Collego®) + TX, Copper Octanoate (Cueva®) + TX, Delta traps (Trapline d®) + TX, Erwinia amylovora (Harpin) (ProAct® + TX, Ni-HIBIT Gold CST®) + TX, Ferri-phosphate (Ferramol®) + TX, Funnel traps (Trapline y®) + TX, Gallex® + TX, Grower's Secret® + TX, Homo-brassonolide + TX, Iron Phosphate (Lilly Miller Worry Free Ferramol Slug & Snail Bait®) + TX, MCP hail trap (Trapline f®) + TX, *Microctonus hyperodae* + TX, *Mycoleptodiscus terrestris* (Des-X®) + TX, BioGain® + TX, Aminomite® + TX, Zenox® + TX, Pheromone trap (Thripline ams®) + TX, potassium bicarbonate (MilStop®) + TX, potassium salts of fatty acids (Sanova®) + TX, potassium silicate solution (Sil-Matrix®) + TX, potassium iodide + potassiumthiocyanate (Enzicur®) + TX, SuffOil-X® + TX, Spider venom + TX, *Nosema locustae* (Semaspore Organic Grasshopper Control®) + TX, Sticky traps (Trapline YF® + TX, Rebell Amarillo®) + TX and Traps (Takitrapline y + b®) + TX.

The references in brackets behind the active ingredients, e.g. *[3878-19-1]* refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright© 1995-2004]; for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The active ingredient mixture of the compounds of formula I selected from Tables 1 to 27 and Table P with active ingredients described above comprises a compound selected from Tables 1 to 27 and Table P and an active ingredient as described above preferably in a mixing ratio of from 100:1 to 1:6000, especially from 50:1 to 1:50, more especially in a ratio of from 20:1 to 1:20, even more especially from 10:1 to 1:10, very especially from 5:1 and 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1:750, or 2:750, or 4:750. Those mixing ratios are by weight.

The mixtures as described above can be used in a method for controlling pests, which comprises applying a composition comprising a mixture as described above to the pests or their environment, with the exception of a method for treatment of the human or animal body by surgery or therapy and diagnostic methods practised on the human or animal body.

The mixtures comprising a compound of formula I selected from Tables 1 to 27 and Table P and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds selected from Table P and the active ingredients as described above is not essential for working the present invention.

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds I for the preparation of these compositions are also a subject of the invention.

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is generally 1 to 2000 g of active ingredient per hectare, in particular 10 to 1000 g/ha, preferably 10 to 600 g/ha.

A preferred method of application in the field of crop protection is application to the foliage of the plants (foliar application), it being possible to select frequency and rate of application to match the danger of infestation with the pest in question. Alternatively, the active ingredient can reach the plants via the root system (systemic action), by drenching the locus of the plants with a liquid composition or by incorporating the active ingredient in solid form into the locus of the plants, for example into the soil, for example in the form of granules (soil application). In the case of paddy rice crops, such granules can be metered into the flooded paddy-field.

The compounds of the invention and compositions thereof are also be suitable for the protection of plant propagation material, for example seeds, such as fruit, tubers or kernels, or nursery plants, against pests of the abovementioned type. The propagation material can be treated with the compound prior to planting, for example seed can be treated prior to sowing. Alternatively, the compound can be applied to seed kernels (coating), either by soaking the kernels in a liquid composition or by applying a layer of a solid composition. It is also possible to apply the compositions when the propagation material is planted to the site of application, for example into the seed furrow during drilling. These treatment methods for plant propagation material and the plant propagation material thus treated are further subjects of the invention. Typical treatment rates would depend on the plant and pest/fungi to be controlled and are generally between 1 to 200 grams per 100 kg of seeds, preferably between 5 to 150 grams per 100 kg of seeds, such as between 10 to 100 grams per 100 kg of seeds.

The term seed embraces seeds and plant propagules of all kinds including but not limited to true seeds, seed pieces, suckers, corns, bulbs, fruit, tubers, grains, rhizomes, cuttings, cut shoots and the like and means in a preferred embodiment true seeds.
The present invention also comprises seeds coated or treated with or containing a compound of formula (I-4). The term "coated or treated with and/or containing" generally signifies that the active ingredient is for the most part on the surface of the seed at the time of application, although a greater or lesser part of the ingredient may penetrate into the seed material, depending on the method of application. When the said seed product is (re)planted, it may absorb the active ingredient. In an embodiment, the present invention makes available a plant propagation material adhered thereto with a compound of formula (I-4). Further, it is hereby made available, a composition comprising a plant propagation material treated with a compound of formula (I).

Seed treatment comprises all suitable seed treatment techniques known in the art, such as seed dressing, seed coating, seed dusting, seed soaking and seed pelleting. The seed treatment application of the compound formula (I) can be carried out by any known methods, such as spraying or by dusting the seeds before sowing or during the sowing/planting of the seeds.

### Biological Examples:

Example B1: *Diabrotica balteata* (Banded cucumber beetle):
   larvicide, feeding/contact activity, preventative
   Artificial diet was dispensed into a 96-well microtiter plate and treated with aqueous test solutions prepared from 10'000 ppm DMSO stock solutions. Eggs were suspended above the treated diet and first instar larvae hatched onto the diet. Mortality was assessed 5 days after treatment.
   Compounds P4, P5, P6 and P7 gave at least 70% control of *Diabrotica balteata* at an application rate of 500ppm.
Example B2: *Heliothis virescens* (Tobacco budworm):
   ovo-larvicide, feeding/contact activity, curative
   Cotton leaf discs were placed on agar in a 96-well microtiter plate and infested with eggs. Aqueous test solutions prepared from 10'000 ppm DMSO stock solutions were then applied. Mortality was assessed 3 days after treatment.
   Compound P1, P5 and P9 gave at least 75% control of *Heliothis virescens* at an application rate of 1000 ppm.
Example B3: *Plutella xylostella* (Diamond-back moth):
   larvicide, feeding/contact activity, preventative
   Artificial diet was dispensed into a 96-well microtiter plate and treated with aqueous test solutions prepared from 10'000 ppm DMSO stock solutions. Eggs were suspended above the treated diet and first instar larvae hatched onto the diet. Mortality was assessed 9 days after treatment.
   Compound P7, P8, P12, P13, P14, P15, P16, P17, P18, P19, P20, P21, P22, P23, P24 and P25 gave at least 70% control of *Plutella xylostella* at an application rate of 500ppm.
Example B4: Activity against *Spodoptera littoralis* (Egyptian cotton leaf worm)
   Cotton leaf discs were placed on agar in 24-well microtiter plates and sprayed with aqueous test solutions prepared from 10'000 ppm DMSO stock solutions. After drying the leaf discs were infested with five L1 larvae. The samples were assessed for mortality, anti-feedant effect, and growth inhibition in comparison to untreated samples 3 days after infestation. Control of Spodoptera littoralis by a test sample is given when at least one of the categories mortality, anti-feedant effect, and growth inhibition is higher than the untreated sample.
   The following compounds resulted in at least 80% control at an application rate of 200 ppm: P3, P12, P13, P15, P17, P20, P22 and P23.
Example B5: Activity against *Spodoptera littoralis* (Egyptian cotton leaf worm)
   Test compounds were applied by pipette from 10'000 ppm DMSO stock solutions into 24-well plates and mixed with agar. Lettuce seeds were placed onto the agar and the multi well plate was closed by another plate which contained also agar. After 7 days the compound was absorbed by the roots and the lettuce grew into the lid plate. The lettuce leaves were then cut off into the lid plate. Spodoptera eggs were pipetted through a plastic stencil onto a humid gel blotting paper and the lid plate was closed with it. The samples were assessed for mortality, anti-feedant effect and growth inhibition in comparison to untreated samples 6 days after infestation.
   The following compounds gave an effect of at least 80% in at least one of the three categories (mortality, anti-feeding, or growth inhibition) at a test rate of 12.5 ppm: P15, P20 and P23.
Example B6: Activity against *Diabrotica balteata* (Corn root worm)
   Maize sprouts placed onto an agar layer in 24-well microtiter plates were treated with aqueous test solutions prepared from 10'000 ppm DMSO stock solutions by spraying. After drying, the plates were infested with L2 larvae (6 to 10 per well). The samples were assessed for mortality and growth inhibition in comparison to untreated samples 4 days after infestation.
   The following compounds gave an effect of at least 80% in at least one of the two categories (mortality or growth inhibition) at an application rate of 200 ppm: P5, P12, P13, P14, P15, P17, P19, P20, P21, P22, P23, P24 and P25.
Example B7: Activity against *Euschistus heros* (Neotropical Brown Stink Bug)
   Soybean leaves on agar in 24-well microtiter plates were sprayed with aqueous test solutions prepared from 10'000 ppm DMSO stock solutions. After drying the leaves were infested with N2 nymphs. The samples were assessed for mortality and growth inhibition in comparison to untreated samples 5 days after infestation.
   The following compounds gave an effect of at least 80% in at least one of the two categories (mortality or growth inhibition) at an application rate of 200 ppm: P14, P15, P17 and P25.
Example B8: Activity against *Myzus persicae* (Green peach aphid)
   Sunflower leaf discs were placed onto agar in a 24-well microtiter plate and sprayed with aqueous test solutions prepared from 10'000 ppm DMSO stock solutions. After drying, the leaf discs were infested with an aphid population of mixed ages. The samples were assessed for mortality 6 days after infestation.
   The following compounds resulted in at least 80% mortality at an application rate of 200 ppm: P14, P15, P19 and P20.
Example B9: Activity against *Myzus persicae* (Green peach aphid)
   Roots of pea seedlings infested with an aphid population of mixed ages were placed directly into aqueous test solutions prepared from 10'000 DMSO stock solutions. The samples were assessed for mortality 6 days after placing seedlings into test solutions.
   The following compounds resulted in at least 80% mortality at a test rate of 24 ppm: P20 and P25.
Example B10: Activity against *Myzus persicae* (Green peach aphid)
   Test compounds prepared from 10'000 ppm DMSO stock solutions were applied by pipette into 24-well microtiter plates and mixed with sucrose solution. The plates were closed with a stretched Parafilm. A plastic stencil with 24 holes was placed onto the plate and infested pea seedlings were placed directly on the Parafilm. The infested plate was closed with a gel blotting paper and another plastic stencil and then turned upside down. The samples were assessed for mortality 5 days after infestation.
   The following compounds resulted in at least 80% mortality at a test rate of 12 ppm: P19.
Example B11: Activity against *Plutella xylostella* (Diamond back moth)
   24-well microtiter plates with artificial diet were treated with aqueous test solutions prepared from 10'000 ppm DMSO stock solutions by pipetting. After drying, the plates were infested with L2 larvae (10 to 15 per well). The samples were assessed for mortality and growth inhibition in comparison to untreated samples 5 days after infestation.
   The following compounds gave an effect of at least 80% in at least one of the two categories (mortality or growth inhibition) at an application rate of 200 ppm: P12, P13, P14, P15, P16, P17, P18, P20, P22 and P25.
Example B12: Activity against *Tetranychus urticae* (Two-spotted spider mite)
   Bean leaf discs on agar in 24-well microtiter plates were sprayed with aqueous test solutions prepared from 10'000 ppm DMSO stock solutions. After drying the leaf discs were infested with a mite population of mixed ages. The samples were assessed for mortality on mixed population (mobile stages) 8 days after infestation.
   The following compounds resulted in at least 80% mortality at an application rate of 200 ppm: P12.

## Claims

1. A compound of formula (I-4) 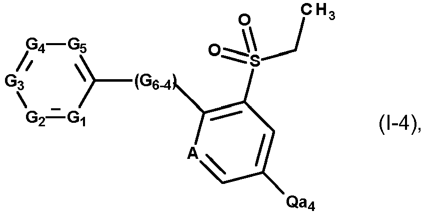 wherein
A is N or CH;
the radical 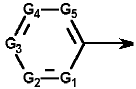 is selected from the group consisting of formula G7, G8 and G9: 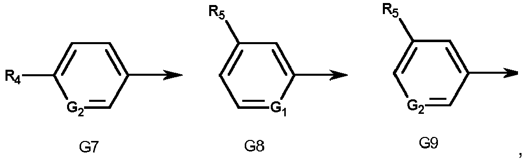
wherein R₄ and R₅ are independently selected from C₁-C₄haloalkyl; and
wherein G₁ and G₂ are independently selected from N and CH;
G₆₋₄ is of formula U-0 : 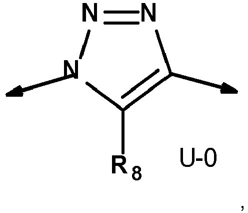
wherein the arrow to the left of each group U denotes the point of attachment to the 6-membered ring containing G₁ and wherein the arrow to the right of group U-0 denotes the point of attachment to the radical containing A; and
wherein R₈ is selected from hydrogen and C₁-C₄alkyl;
Qa₄ is selected from the group consisting of hydrogen, halogen, C₁-C₆haloalkyl and the group J-0 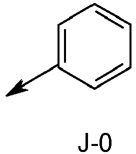 wherein J-0 can be mono-substituted with Rx, wherein Rx is halogen; and agrochemically acceptable salts, stereoismers, enantiomers, tautomers and N-oxides of the compounds of formula (I-4).

2. A compound of formula (I-4) as claimed in claim 1 where R₄ and R₅ are trifluoromethyl.

3. A pesticidal composition, which comprises at least one compound of formula (I-4) according to claim 1 or 2 or, where appropriate, a tautomer thereof, in each case in free form or in agrochemically utilizable salt form, as active ingredient and at least one auxiliary.

4. A method for controlling pests, which comprises applying a composition according to claim 3 to the pests or their environment with the exception of a method for treatment of the human or animal body by surgery or therapy and diagnostic methods practised on the human or animal body.

5. A method for the protection of plant propagation material from the attack by pests, which comprises treating the propagation material or the site, where the propagation material is planted, with a composition according to claim 3.

## Patentansprüche

1. Verbindung der Formel (1-4) 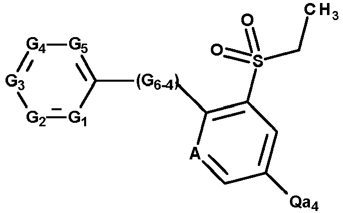 (I-4),
wobei
A für N oder CH steht;
der Rest 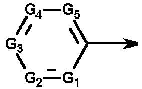 aus der aus der aus Formel G7, G8 und G9 bestehenden Gruppe ausgewählt ist: 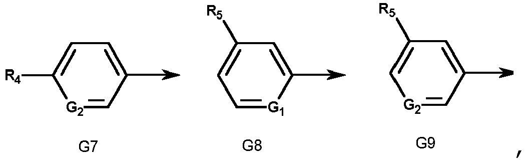
wobei R₄ und R₅ unabhängig voneinander aus C₁-C₄-Halogenalkyl ausgewählt sind und
wobei G₁ und G₂ unabhängig voneinander aus N und CH ausgewählt sind,
G₆₋₄ die Formel U-0 aufweist: 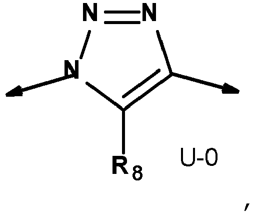
wobei der Pfeil links von der Gruppe U jeweils den Anbindungspunkt an den G₁ enthaltenden 6-gliedrigen Ring kennzeichnet und wobei der Pfeil rechts von der Gruppe U-0 den Anbindungspunkt an den A enthaltenden Rest kennzeichnet und
wobei R₈ aus Wasserstoff und C₁-C₄-Alkyl ausgewählt ist,
Qa₄ aus der aus Wasserstoff, Halogen, C₁-C₆-Halogenalkyl und der Gruppe J-0 bestehenden Gruppe ausgewählt ist, 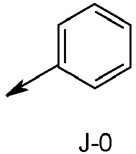 wobei J-0 durch Rx monosubstituiert sein kann, wobei Rx für Halogen steht, und agrochemisch unbedenkliche Salze, Stereoisomere, Enantiomere, Tautomere und N-Oxide der Verbindungen der Formel (I-4) .

2. Verbindung der Formel (1-4) nach Anspruch 1, wobei R₄ und R₅ für Trifluormethyl stehen.

3. Pestizide Zusammensetzung, die mindestens eine Verbindung der Formel (1-4) nach Anspruch 1 oder 2 oder gegebenenfalls ein Tautomer davon, jeweils in freier Form oder in Form eines agrochemisch brauchbaren Salzes, als Wirkstoff und mindestens einen Hilfsstoff umfasst.

4. Verfahren zur Bekämpfung von Schädlingen, bei dem man eine Zusammensetzung nach Anspruch 3 auf die Schädlinge oder ihre Umgebung aufbringt, mit der Ausnahme von einem Verfahren zur Behandlung des menschlichen oder tierischen Körpers durch Chirurgie oder Therapie und Diagnoseverfahren, die am menschlichen oder tierischen Körper durchgeführt werden.

5. Verfahren zum Schutz von Pflanzenfortpflanzungsmaterial gegen Befall durch Schädlinge, bei dem man das Fortpflanzungsmaterial oder die Stelle, an der das Fortpflanzungsmaterial angepflanzt ist, mit einer Zusammensetzung nach Anspruch 3 behandelt.

## Revendications

1. Composé de formule (1-4) 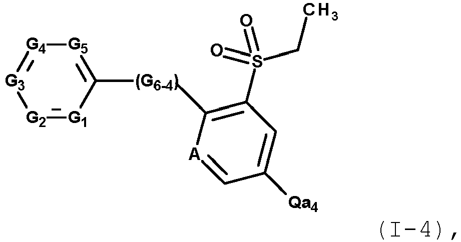
A étant N ou CH ;
le radical 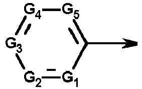 étant choisi dans le groupe constitué par la formule G7, G8 et G9 : 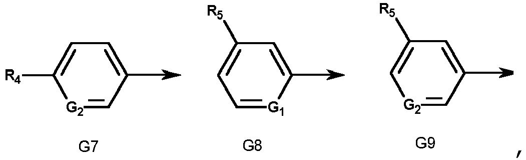
R₄ et R₅ étant chacun choisis indépendamment parmi C₁-C₄halogéno alkyle ; et
G₁ et G₂ étant indépendamment choisis parmi N et CH ;
G₆₋₄ étant de formule U-0 : 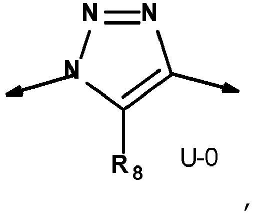
la flèche sur la gauche de chaque groupe U désignant le point de fixation du cycle à 6 chaînons contenant G₁ et la flèche sur la droite du groupe U-0 désignant le point de fixation au radical contenant A ; et
R₈ étant choisi parmi hydrogène et C₁-C₄alkyle ;
Qa₄ étant choisi dans le groupe constitué par hydrogène, halogène, C₁-C₆halogénoalkyle et le groupe J-0 : 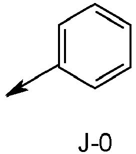 J-0 pouvant être monosubstitué par Rx, Rx étant halogène ; et sels, stéréoisomères, énantiomères, formes tautomères et N-oxydes des composés de formule (I-4), acceptables sur le plan agrochimique.

2. Composé de formule (1-4) selon la revendication 1 dans lequel R₄ et R₅ sont trifluorométhyle.

3. Composition pesticide, qui comprend au moins un composé de formule (1-4) selon la revendication 1 ou 2 ou, le cas échéant, une forme tautomère correspondante, dans chaque cas sous forme libre ou sous forme de sel utilisable sur le plan agrochimique, en tant qu'ingrédient actif, et au moins un auxiliaire.

4. Procédé pour la lutte contre des parasites, qui comprend l'application d'une composition selon la revendication 3 aux parasites ou à leur environnement, à l'exception d'un procédé pour le traitement du corps humain ou animal par chirurgie ou thérapie et de procédés de diagnostic pratiqués sur le corps humain ou animal.

5. Procédé pour la protection de matériel de propagation végétale contre l'attaque par des parasites, qui comprend le traitement du matériel de propagation ou du site, où le matériel de propagation est planté, par une composition selon la revendication 3.
